# EUROPÄISCHE PATENTSCHRIFT

(11) **EP 1 015 437 B1**
(45) Veröffentlichungstag und Bekanntmachung des Hinweises auf die Patenterteilung: **03.12.2003**
(21) Anmeldenummer: 98952562.1
(22) Anmeldetag: 08.09.1998
(51) Int. Cl.: C07D 265/36, C07D 265/34, C07D 498/04, C07D 279/16, C07D 279/14, C07D 413/12, C07D 413/04, C07D 413/06, A61K 31/535, A61K 31/54

(54) **BENZOXAZIN- UND BENZOTHIAZIN-DERIVATE UND DEREN VERWENDUNG IN ARZNEIMITTELN**
BENZOXAZINE AND BENZOTHIAZINE DERIVATIVES AND THEIR USE IN PHARMACEUTICALS
DERIVES DE BENZOXAZINE ET DE BENZOTHIAZINE ET LEUR UTILISATION DANS DES MEDICAMENTS

(30) Priorität: 08.09.1997 DE 19740386; 05.06.1998 DE 19826232
(43) Veröffentlichungstag der Anmeldung: 05.07.2000
(73) Patentinhaber: SCHERING AKTIENGESELLSCHAFT, 13342 Berlin (DE)
(72) Erfinder: HÖLSCHER, Peter, D-10559 Berlin (DE); REHWINKEL, Hartmut, D-12051 Berlin (DE); JAROCH, Stefan, D-10625 Berlin (DE); SUELZLE, Detlev, D-10589 Berlin (DE)
(86) Internationale Anmeldenummer: DE9802690
(87) Internationale Veröffentlichungsnummer: WO99012915

(56) Entgegenhaltungen:
- WO-A-96/14844

## Beschreibung

Die Erfindung betrifft Benzoxazin- und Benzothiazin-Derivate, das Verfahren zu deren Herstellung und deren Verwendung in Arzneimitteln.

In menschlichen Zellen existieren 3 spezifische Formen von Stickstoffmonoxid-Synthasen, die Arginin in Stickstoffmonoxid (NO) und Citrullin überführen. So wurden zwei konstitutive NO-Synthasen (NOS) identifiziert, die als Ca⁺⁺/Calmodulin abhängige Enzyme im Gehirn (ncNOS oder NOS 1 ) bzw. im Endothel (ecNOS oder NOS 3) vorhanden sind. Die dritte Isoform ist die induzierbare NOS (iNOS oder NOS 2), die ein Ca⁺⁺ unabhängiges Enzym ist und induziert wird nach Aktivierung unterschiedlicher Zellen durch Endotoxin.

NOS-Inhibitoren und insbesondere spezifische Inhibitoren der NOS 1, NOS 2 oder NOS 3 sind daher zur Therapie unterschiedlicher Erkrankungen geeignet, die durch pathologische Konzentrationen von NO in Zellen hervorgerufen oder verschlimmert werden (Clin. Neuropharmac. 18, 1995 Seite 482).

Als NOS-Inhibitoren sind unterschiedliche Verbindungen bekannt. Beispielsweise werden cyclische Amidinderivate in WO 96/14844 beschrieben. Aus keiner Publikation ist jedoch bekannt, daß Benzoxazine oder Benzothiazine Stickstoffmonoxid-Synthasen inhibieren.

Die Erfindung betrifft die Verbindungen der Formel I, deren Tautomere, Stereoisomere, geometrische Isomere und Salze 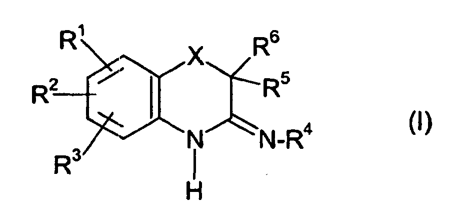 worin
X O oder S,
R¹ NO₂, Cyano, CF₃, -OCF₃, -SO₂NR⁷R⁸, -CONR⁷R⁸, -NR⁹-C(=NR¹⁰)-R^{11,}-NH-CS-NR⁷R⁸, -NH-CO-NR⁷R⁸, NR¹²R¹³, -CO-R¹⁴, C₆₋₁₀-Aryl, das gegebenenfalls substituiert ist mit Halogen, Cyano C₁₋₄-Alkyl, -S-R⁹, -O-R⁹, -NR⁷R⁸ oder CONR⁷R⁸,
   5- oder 6-gliedriges Heteroaryl mit 1 bis 4 Heteroatomen wie Sauerstoff, Stickstoff oder Schwefel, das gegebenenfalls mit -OR⁹, -SR⁹, Halogen, C₁₋₄-Alkyl, NR⁷R⁸ oder CONR⁷R⁸ substituiert ist,
   C₁₋₆-Alkyl, das substituiert ist mit Halogen, -OR⁹, -SR⁹, -NR⁷R⁸, -NR^{7'}R^{8'}, =NR⁷,=NOC₁₋₆-Alkyl, =N-NHAryl, Phenyl, C₃₋₇-Cycloalkyl oder 5- oder 6-gliedrigem Heteroaryl, mit 1 - 3 N-, O- oder S-Atomen,
   C₂₋₆-Alkenyl, das mit Halogen, CONH₂, C≡N oder Phenyl substituiert ist,
   C₂₋₆-Alkinyl, das mit Halogen, CONH₂, C≡N oder Phenyl substituiert ist,
   C₃₋₇-Cycloalkyl,
R² Wasserstoff bedeutet oder
   R¹ und R² gemeinsam mit zwei benachbarten Kohlenstoffatomen einen 5-, 6-, 7-oder 8-gliedrigen Ring bilden, der monocyclisch oder bicyclisch, gesättigt oder ungesättigt und bei dem 1 oder 2 CH₂-Gruppen durch Sauerstoff oder Carbonyl oder dessen Derivat, ausgewählt aus der Gruppe =N-OH, =N-OC₁₋₆-Alkyl, =N-NH₂ und =N-NH-Phenyl, ersetzt sein können und der mit -NR⁷R⁸, -NR^{7'}R^{8'} oder C₁₋₄-Alkyl substituiert sein kann.
R³ Wasserstoff, Halogen, -S-R⁹ oder -O-R⁹ bedeutet oder unabhängig voneinander eine der Bedeutungen von R¹ ist,
R⁴ Wasserstoff oder Acyl,
R⁵ Wasserstoff,
R⁶ C₃₋₇-Cycloalkyl, C₆₋₁₀-Aryl, C₁₋₆-Alkyl-, C₂₋₆-Alkenyl- oder C₂₋₆-Alkynylreste, die jeweils substituiert sein können mit Halogen, OH, O-C₁₋₆-Alkyl, SH, S-C₁₋₆-Alkyl, NR¹⁵R¹⁶, 5- oder 6-gliedriges Heteroaryl mit 1 - 3 N-, O- oder S-Atomen, Phenyl oder C₃₋₇-Cycloalkyl,
R⁷ und R⁸ Wasserstoff, C₁₋₆-Alkyl, gegebenenfalls mit Halogen oder C₁₋₄-Alkyl substituiertes Phenyl, gegebenenfalls mit Halogen oder C₁₋₄-Alkyl substituiertes Benzyl oder C₃₋₇-Cycloalkyl,
   R^{7'} Wasserstoff, gegebenenfalls mit OH, Phenyl, Cyano, COO₁₋₄-Alkyl oder Carbonyl substituiertes C₁₋₆-Alkyl,
R^{8'} C₁₋₆-Alkyl, das substituiert ist mit C₃₋₇-Cycloalkyl, Indanyl, C₆₋₁₀-Aryl oder 5- oder 6-gliedrigen Heteroaryl mit 1 - 3 Stickstoff-, Sauerstoff- oder Schwefelatomen, wobei der Aryl- und Heteroarylrest mit Halogen, C₁₋₄-Alkyl, C₁₋₄-Alkoxy, CF₃, NO₂, NH₂, N(C₁₋₄-Alkyl)₂, SO₂CH₃, -O-CH₂-O, SO₂NH₂, OH oder COO-C₁₋₄-Alkyl substituiert sein können oder Indanyl oder 1,2,3,4-Tetrahydronaphthyl oder
R^{7'} und R^{8'} gemeinsam mit dem Stickstoffatom einen 5 - 7-gliedrigen gesättigten Heterocyclus bilden, der ein weiteres Sauerstoff-, Stickstoff- oder Schwefelatom enthalten und mit C₁₋₄-Alkyl, Phenyl, Benzyl oder Benzoyl substituiert sein kann oder einen ungesättigten 5-gliedrigen Heterocyclus bilden, der 1 - 3 N-Atome enthalten und substituiert sein kann mit Phenyl, C₁₋₄-Alkyl, Halogen oder CH₂-OH,
R⁹, R¹⁰ und R¹⁵, R¹⁶ Wasserstoff oder C₁₋₆-Alkyl,
R¹¹ C₁₋₆-Alkyl, -NH₂, -NH-CH₃,-NH-CN, gegebenenfalls mit Halogen, C₁₋₄-Alkyl oder CF₃ substituiertes C₆₋₁₀-Aryl oder gegebenenfalls mit Halogen, C₁₋₄-Alkyl oder CF₃ substituiertes 5- oder 6-gliedriges Heteroaryl mit 1 bis 4 Stickstoff-, Schwefeloder Sauerstoffatomen,
R¹², R¹³ gemeinsam mit dem Stickstoffatom einen gesättigten 5-, 6- oder 7-gliedrigen Ring bilden, der ein weiteres Stickstoff-, Sauerstoff- oder Schwefelatom enthalten und substituiert sein kann mit C₁₋₄-Alkyl, Phenyl, Benzyl oder Benzoyl und
R¹⁴ Wasserstoff, Phenyl, gegebenenfalls mit CO₂H, CO₂C₁₋₆-Alkyl, Hydroxy, C₁₋₄-Alkoxy, Halogen, NR⁷R⁸, NR¹²R¹³,CONR⁷R⁸ oder Phenyl substituiertes C₁₋₆-Alkyl oder gegebenenfalls mit Phenyl, Cyano, CONR⁷R⁸ oder CO₂C₁₋₄-Alkyl substituiertes C₂₋₆-Alkenyl
bedeuten.

Die Verbindungen der Formel können als Tautomere, Stereoisomere oder geometrische Isomere vorliegen. Die Erfindung umfaßt auch alle möglichen Isomeren wie E- und Z-Isomere, S- und R-Enantiomere, Diastereomere, Razemate und Gemische derselben einschließlich der tautomeren Verbindungen der Formel la und Ib

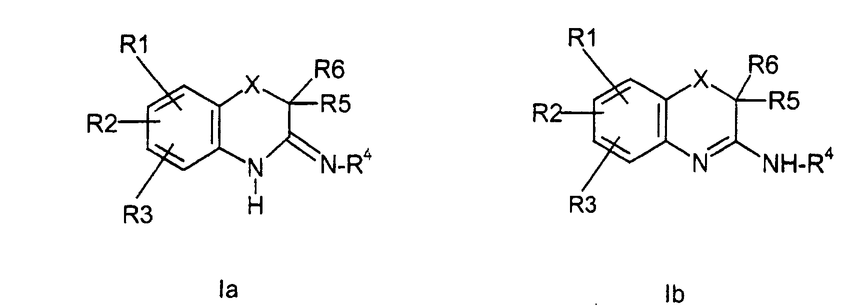

Die physiologisch verträglichen Salze können mit anorganischen und organischen Säuren gebildet werden wie beispielsweise Oxalsäure, Milchsäure, Zitronensäure, Fumarsäure, Essigsäure, Maleinsäure, Weinsäure, Phosphorsäure, HCl, HBr, Schwefelsäure, p-Toluolsulfonsäure, Methansulfonsäure u.a.

Zur Salzbildung von Säuregruppen sind auch die anorganischen oder organischen Basen geeignet, die zur Bildung physiologisch verträglicher Salze bekannt sind wie beispielsweise Alkalihydroxide, wie Natrium- und Kaliumhydroxid, Erdalkalihydroxide wie Calciumhydroxid, Ammoniak, Amine wie Ethanolamin, Diethanolamin, Triethanolamin, N-Methylglucamin, Tris-(hydroxymethyl)-methylamin usw.

Alkyl bedeutet jeweils eine geradkettige oder verzweigte Alkylgruppe wie z.B. Methyl, Ethyl, Propyl, Isopropyl, n-Butyl, sek. Butyl, tert. Butyl, n-Pentyl, sek. Pentyl, tert. Pentyl, Neopentyl, n-Hexyl., sek. Hexyl, Heptyl, Octyl, insbesondere C₁₋₄-Alkylgruppen.

Alkenyl- und Alkynyl-Substituenten sind jeweils geradkettig oder verzweigt. Beispielsweise seien die folgenden Reste genannt: Vinyl, 2-Propenyl, 1-Propenyl, 2-Butenyl, 1-Butenyl, 1-Butenyl, 2-Butenyl, 1-Methyl-1-propenyl, 2-Methyl-2-propenyl, 3-Methyl-2-propenyl, Ethinyl, 1-Propinyl, 2-Propinyl, 1-Butinyl, 2-Butinyl.

Unter Cycloalkyl ist jeweils Cyclopropyl, Cyclobutyl, Cyclopentyl, Cyclohexyl oder Cycloheptyl zu verstehen.

Halogen bedeutet jeweils Fluor, Chlor, Brom oder Jod.

Unter Aryl ist jeweils Naphthyl oder Phenyl zu verstehen, das ein- oder mehrfach substituiert sein kann. Ebenso können die Phenyl- und Benzylreste R⁷, R⁸ und R^{8'} in beliebiger Position ein- oder mehrfach gleich oder verschieden substituiert sein.

Der Hetarylrest kann jeweils einen ankondensierten Benzolring enthalten und ein- bis dreifach gleich oder verschieden substituiert sein und über das Heteroatom oder ein Kohlenstoffatom gebunden sein. Beispielsweise sind die folgenden 5- und 6-Ringheteroaromaten jeweils geeignet:

Imidazol, Indol, Isooxazol, Isothiazol, Furan, Oxadiazol, Oxazol, Pyrazin, Pyridazin, Pyrimidin, Pyridin, Pyrazol, Pyrrol, Tetrazol, Thiazol, Triazol, Thiophen, Thiadiazol, Benzimidazol, Benzofuran, Benzoxazol, Isochinolin, Chinolin. Bevorzugt seien Pyridin, Pyrrol, Thiophen, Thiazol und Imidazol genannt.

Als bevorzugte Ausführungsform für R¹¹ in der Bedeutung Heteroaryl ist Thienyl zu betrachten.

Als gesättigte Heterocyclen NR¹²R¹³ und NR^{7'}R^{8'} seien beispielsweise Piperidin, Pyrrolidin, Morpholin, Thiomorpholin, Hexahydroazepin und Piperazin genannt. Der Heterocyclus kann 1 - 3-fach substituiert sein mit C₁₋₄-Alkyl oder einem gegebenenfalls mit Halogen substituierten Phenyl-, Benzyl- oder Benzoylrest. Beispielsweise seien genannt: N-Methyl-piperazin, 2,6-Dimethylmorpholin, Phenylpiperazin oder 4-(4-Fluorbenzoyl)-piperidin.

Bilden NR^{7'}R^{8'} gemeinsam mit dem Stickstoffatom einen ungesättigten Heterocyclus, so seien beispielsweise Imidazol, Pyrrol, Pyrazol, Triazol Benzimidazol und Indazol genannt, die ein- bis zweifach mit Phenyl, C₁₋₄-Alkyl, Halogen insbesondere Chlor oder CH₂-OH substituiert sein können.

Der Alkylrest R^{8'} kann 1 oder zweifach gleich verschieden substituiert sein. Bedeutet der Substituent des Alkylrestes R^{8'} einen Heteroarylrest, so sind die für R¹¹ genannten Heteroarylreste geeignet, die jedoch nicht über ein N-Atom verknüpft sein können.

Bedeutet R^{8'} Indanyl oder 1,2,3,4-Tetrahydronaphthyl, so kann dieser Rest jeweils in 1-oder 2-Position verknüpft sein.

Der Substituent R⁶ bedeutet bevorzugt Alkyl, das substituiert sein kann und inbesondere C₁₋₆-Alkyl.

Bilden R¹ und R² gemeinsam mit zwei benachbarten Kohlenstoffatomen eine 5, 6-, 7-oder 8-gliedrigen Ring, so kann dieser in Position 5, 6 oder 6, 7 oder 7, 8 des Benzoxazins bzw. Benzothiazins stehen und hat die Formel 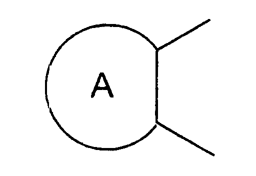 worin

A einen gesättigten oder ungesättigten C₃₋₈-Alkylenrest bedeutet, der 1 bis 4-fach gleich oder verschieden mit -NR⁷R⁸, -NR^{7'}R^{8'} oder C₁₋₄-Alkyl substituiert sein kann und bei dem 1 oder 2 CH₂-Gruppen durch Sauerstoff oder Carbonyl oder dessen Derivat ersetzt sein können und wobei der Alkylenrest einen ankondensierten Benzolrest enthalten kann wie beispielsweise Indan oder als Bicyclus vorliegen kann wie beispielsweise Bicycloheptan.

Als Strukturen von A seien beispielsweise genannt:

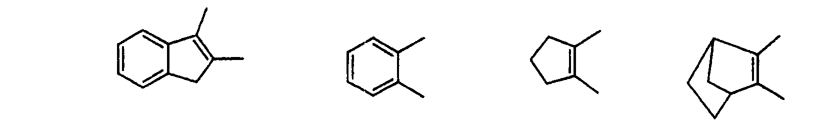

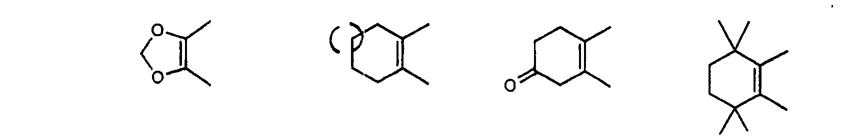

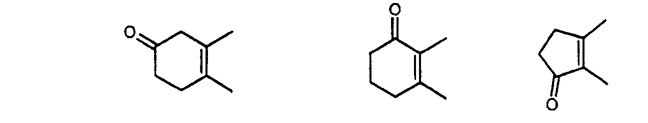

Als Carbonylderivate sind beispielsweise =NOH, =N-OC₁₋₆-Alkyl, =NH-NH₂, =N-NH-Phenyl geeignet. Vorzugsweise sind zwei benachbarte Kohlenstoffatome des Aromaten mit C₁₋₆-Alkylen zu einem 3 - 8-gliedrigen ungesättigtem Ring verknüpft, der in beliebiger Position substituiert sein kann.

Der Acylrest R⁴ leitet sich von insbesondere geradkettigen oder verzweigten C₁₋₆-aliphatischen Carbonsäuren ab wie beispielsweise Ameisensäure, Essigsäure, Propionsäure, Buttersäure, Trimethylessigsäure oder Capronsäure oder von bekannten Benzolsulfonsäuren, die mit Halogen oder C₁₋₄-Alkyl substituiert sein können, sowie C₁₋₄-Alkansulfonsäuren geeignet wie beispielsweise Methansulfonsäure, p-Toluolsulfonsäure.

Bevorzugte Ausführungsformen von X sind S und O und von R³ Wasserstoff.

Vorzugsweise bedeutet R⁴ Wasserstoff.

Die Erfindung betrifft auch die Verwendung der erfindungsgemäßen Verbindungen zur Herstellung eines Arzneimittels zur Behandlung von Erkrankungen, die durch die Wirkung von Stickstoffmonoxid in pathologischen Konzentrationen hervorgerufen werden. Dazu zählen neurodegenerative Erkrankungen, inflammatorische Erkrankungen, Autoimmunerkrankungen, Herz-Kreislauf-Erkrankungen.

Beispielsweise seien genannt: Cerebrale Ischaemie, Hypoxie und andere neurodegenerative Erkrankungen, die mit Entzündungen in Verbindung gebracht werden wie Multiple Sklerose, Amyotrophe Lateralsklerose und vergleichbare sklerotische Erkrankungen, Morbus Parkinson, Huntington's Disease, Korksakoffs Disease, Epilepsie, Erbrechen, Stress, Schlafstörungen, Schizophrenie, Depression, Migräne, Hypoglykämie, Demenz wie z.B. Alzheimersche Krankheit, HIV-Demenz und Presenile Demenz.

Ferner eignen sie sich zur Behandlung von Krankheiten des Herz-Kreislauf Systems und zur Behandlung autoimmuner und/oder inflammatorischer Erkrankungen wie Hypotension, ARDS (adult respiratory distress syndrome), Sepsis oder Septischer Schock, Rheumatoider Arthritis, Osteoarthritis, von insulinabhängiger Diabetes Mellitus (IDDM), entzündlicher Erkrankung des Beckens /Darms (bowel disease), von Meningitis, Glomerulonephritis, akute und chronische Lebererkrankungen, Erkrankungen durch Abstoßung (beispielsweise allogene Herz-,Nieren- oder Lebertransplantationen) oder entzündlichen Hautkrankheiten wie Psoriasis und andere. Auf Grund ihres Wirkprofils eignen sich die erfindungsgemäßen Verbindungen sehr gut zur Inhibition der neuronalen NOS.

Zur Verwendung der erfindungsgemäßen Verbindungen als Arzneimittel werden diese in die Form eines pharmazeutischen Präparats gebracht, das neben dem Wirkstoff für die enterale oder parenterale Applikation geeignete Träger-, Hilfs- und/oder Zusatzstoffe enthält. Die Applikation kann oral oder sublingual als Feststoff in Form von Kapseln oder Tabletten oder als Flüssigkeit in Form von Lösungen, Suspensionen, Elixieren, Aerosolen oder Emulsionen oder rektal in Form von Suppositorien oder in Form von gegebenenfalls auch subcutan intramuskulär oder intravenös anwendbaren Injektionslösungen oder topisch oder intrathekal erfolgen. Als Hilfsstoffe für die gewünschte Arzneimittelformulierung sind die dem Fachmann bekannten inerten organischen und anorganischen Trägermaterialien geeignet wie z.B. Wasser, Gelantine, Gummmi arabicum, Milchzucker, Stärke, Magnesiumstearat, Talk, pflanzliche Öle, Polyalkylenglykole usw. Gegebenenfalls können darüber hinaus Konservierungs-, Stabilisierungs-, Netzmittel, Emulgatoren oder Salze zur Veränderung des osmotischen Druckes oder Puffer enthalten sein.

Für die parenterale Anwendung sind insbesondere Injektionslösungen oder Suspensionen, insbesondere wäßrige Lösungen der aktiven Verbindungen in polyhydroxyethoxyliertem Rizinusöl, geeignet.

Als Trägersysteme können auch grenzflächenaktive Hilfsstoffe wie Salze der Gallensäuren oder tierische oder pflanzliche Phospholipide, aber auch Mischungen davon sowie Liposome oder deren Bestandteile verwendet werden.

Für die orale Anwendung sind insbesondere Tabletten, Dragees oder Kapseln mit Talkum und/oder Kohlenwasserstoffträger oder -binder, wie zum Beispiel Lactose, Mais- oder Kartoffelstärke, geeignet. Die Anwendung kann auch in flüssiger Form erfolgen, wie zum Beispiel als Saft, dem gegebenenfalls ein Süßstoff beigefügt ist.

Die Dosierung der Wirkstoffe kann je nach Verabfolgungsweg, Alter und Gewicht des Patienten, Art und Schwere der zu behandelnden Erkrankung und ähnlichen Faktoren variieren. Die tägliche Dosis beträgt 1-2000 mg, vorzugsweise 20-500 mg, wobei die Dosis als einmal zu verabreichende Einzeldosis oder unterteilt in 2 oder mehreren Tagesdosen gegeben werden kann.

Die NOS-inhibitorische Wirksamkeit der Verbindungen der Formel I und deren physiologisch verträglicher Salze kann nach den Methoden von Bredt und Snyder in Proc. Natl. Acad. Sci. USA (1989) 86, 9030-9033 bestimmt werden.

Die Herstellung der erfindungsgemäßen Verbindungen erfolgt dadurch, daß man eine Verbindung der Formel II oder deren Salz 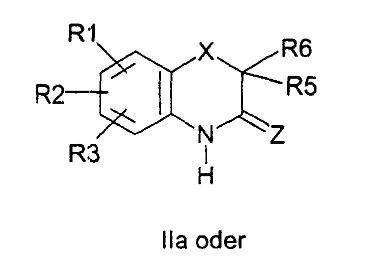 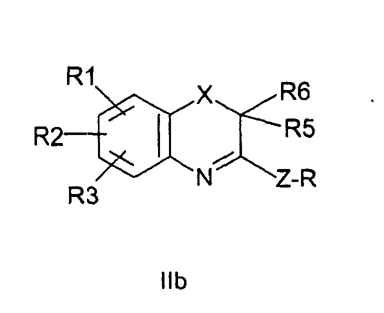 worin
R¹, R², R³, R⁵, R⁶ und X die obige Bedeutung haben, Z Sauerstoff oder Schwefel ist und R C₁₋₆-Alkyl bedeutet, mit Ammoniak oder primären oder sekundären Aminen umsetzt, wobei vorhandene primäre und sekundäre Aminogruppen gegebenenfalls intermediär geschützt sind und gewünschtenfalls anschließend acyliert, die Isomeren trennt oder die Salze bildet.

Die Umsetzung mit Ammoniak gelingt unter Druck in Autoklaven bei Ammoniaküberschuß bei tiefen Temperaturen (- 78 °C) oder durch Rühren in mit Ammoniak gesättigten Methanol bei Raumtemperatur. Bevorzugt werden Thiolactame umgesetzt. Wird mit Aminen umgesetzt, so stellt man aus dem Lactam oder Thiolactam zunächst den Iminether oder Iminothioether als Zwischenverbindung dar (z.B. mit Methyliodid oder Methylsulfat) und setzt diesen mit oder ohne Isolierung der Zwischenverbindung mit den entsprechenden Aminen oder deren Salzen um.

Als Aminoschutzgruppen sind beispielsweise Carbamate wie tert. Butoxycarbonyl, Benzyloxycarbonyl oder Acetyl geeignet.

An den Vorstufen werden gewünschtenfalls Sulfide oxidiert, Ester verseift, Säuren verestert, Hydroxygruppen verethert oder acyliert, Amine acyliert, alkyliert, diazotiert, halogeniert, NO₂ eingeführt oder reduziert, mit Isocyanaten oder Isothiocyanaten umgesetzt, die Isomeren getrennt oder die Salze gebildet.

Die Verseifung einer Estergruppe kann basisch oder sauer erfolgen, indem man bei Raumtemperatur oder erhöhter Temperatur bis zur Siedetemperatur des Reaktionsgemisches in Gegenwart von Alkalihydroxiden in Ethanol oder anderen Alkoholen oder mittels Säuren wie z.B. Salzsäure hydrolysiert und ggf. Salze der Aminobenzoxazine oder -thiazine weiterverarbeitet.

Die Veresterung der Carbonsäure geschieht in an sich bekannter Weise mit Diazomethan oder dem entsprechenden Alkohol in Säure oder in Gegenwart eines aktivierten Säurederivats. Als aktivierte Säurederivate kommen zum Beispiel Säurechlorid, -imidazolid oder -anhydrid in Frage.

Die Reduktion einer Estergruppe zum Alkohol erfolgt in an sich bekannter Weise mit DiBAH in geeignetem Lösungsmittel bei tiefen Temperaturen. Die reduktive Aminierung von Benzaldehyd mit Amin unter Zugabe eines Borhydrides gibt benzylische Amine.

Zusätzlich kann durch elektrophile aromatische Substitution eine Nitrogruppe oder Halogen, insbesondere Brom, eingeführt werden. Dabei entstehendeGemische können in üblicher Weise, auch mittels HPLC, getrennt werden. Wenn ein Nitril vorliegt, kann dieses nach bekannten Verfahren verseift werden oder in das entsprechende Amin, Tetrazol oder Amidoxim überführt werden oder es wird durch Angriff von substituierten Anilinen oder Aminen zu einem substituierten Amidin.

Die Friedel-Crafts Acylierung wird bei Lactamen vom Typ Ila erfolgreich angewandt, und anschliessend kann selektiv das Lactam in das Thiolactam überführt werden.

Die Reduktion der Nitrogruppe oder ggf. der Cyanogruppe zur Aminogruppe erfolgt katalytisch in polaren Lösungsmitteln bei Raumtemperatur oder erhöhter Temperatur unter Wasserstoffdruck. Als Katalysatoren sind Metalle wie Raney-Nickel oder Edelmetallkatalysatoren wie Palladium oder Platin gegebenenfalls in Gegenwart von Bariumsulfat oder auf Trägern geeignet. Statt Wasserstoff kann auch Ammoniumformiat oder Ameisensäure in bekannter Weise benutzt werden. Reduktionsmittel wie Zinn-II-chlorid oder Titan-III-chlorid können ebenso verwendet werden wie komplexe Metallhydride eventuell in Gegenwart von Schwermetallsalzen. Es kann vorteilhaft sein vor der Reduktion die Estergruppe einzuführen. Für Nitrogruppen bewährt hat sich die Reduktion mit Zink oder Eisen in Essigsäure.

Wird eine einfache oder mehrfache Alkylierung einer Aminogruppe oder einer CHaciden Kohlenstoffposition gewünscht, so kann nach üblichen Methoden beispielsweise mit Alkylhalogeniden alkyliert werden.Gegebenenfalls ist Schutz der Lactamgruppe als Anion durch ein 2. Equivalent Base oder durch eine passende Schutzgruppe erforderlich.

Die Acylierung der Aminogruppe erfolgt in üblicher Weise beispielsweise mit einem Säurehalogenid oder Säureanhydrid gegebenenfalls in Gegenwart einer Base.

Die Einführung der Halogene Chlor, Brom oder Jod über die Aminogruppe kann beispielsweise auch nach Sandmeyer erfolgen, indem man die mit Nitriten intermediär gebildete Diazoniumsalze mit Cu(I)chlorid oder Cu(I)bromid in Gegenwart der entsprechenden Säure wie Salzsäure oder Bromwasserstoffsäure umsetzt oder mit Kaliumjodid umsetzt.

Benzylalkohole lassen sich wie üblich mit Methansulfonylchlorid in die entsprechenden Benzylhalogenide überführen.

Die Einführung einer NO₂-Gruppe gelingt durch eine Reihe von bekannten Nitrierungsmethoden. Beispielsweise kann mit Nitraten oder mit Nitroniumtetrafluoroborat in inerten Lösungsmitteln wie halogenierten Kohlenwasserstoffen oder in Sulfolan oder Eisessig nitriert werden. Möglich ist auch die Einführung z.B. durch Nitriersäure in Wasser oder konz. Schwefelsäure als Lösungsmittel bei Temperaturen zwischen - 10 °C und 30 °C.

Die Isomerengemische können nach üblichen Methoden wie beispielsweise Kristallisation, Chromatographie oder Salzbildung in die Enantiomeren bzw. E/Z-Isomeren aufgetrennt werden. Die Enantiomeren können auch durch Chromatographie an chiralen Phasen sowie durch stereoselektive Synthese erhalten werden.

Die Herstellung der Salze erfolgt in üblicher Weise, indem man eine Lösung der Verbindung der Formel I mit der äquivalenten Menge oder einem Überschuß einer Säure, die gegebenenfalls in Lösung ist, versetzt und den Niederschlag abtrennt oder in üblicher Weise die Lösung aufarbeitet.

Soweit die Herstellung der Ausgangsverbindungen nicht beschrieben wird, sind diese bekannt und käuflich oder analog zu bekannten Verbindungen oder nach hier beschriebenen Verfahren herstellbar.

Nucleophile Substitution von Benzylhalogeniden mit sekundären Aminen liefert die korrespondierenden Benzylamine.

Thiolactame der Formel IIa (Z = S) erhält man beispielsweise aus Lactamen mit Phosphorpentasulfid (P₄S₁₀) oder Lawessons Reagenz (2,4-Bis(4-methoxphenyl)-1,3,2,4-dithiaphosphetan-2,4-disulfid) in geeigneten Lösungsmitteln und Verbindungen der Formell IIb können beispielsweise durch Umsetzung mit Meerwein-Reagenz (Trimethyloxoniumtetrafluoroborat) erhalten werden.

Die Herstellung der Verbindungen der Forml IIa kann beispielsweise dadurch erfolgen, daß man eine Verbindung der Formel III 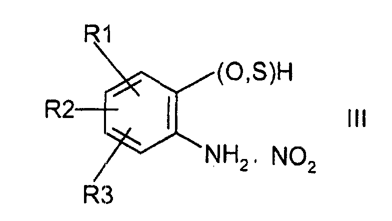 worin R¹ bis R³ die obige Bedeutung haben mit einer Verbindung der Formel IV 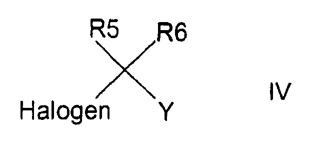 worin R⁵ und R⁶ die obige Bedeutung haben und Y eine reaktive Carboxylgruppe ist wie Säurehalogenid, Nitril, Carbonsäureester umsetzt und gegebenenfalls reduktiv cyclisiert oder dadurch, daß man eine Verbindung der Formel V 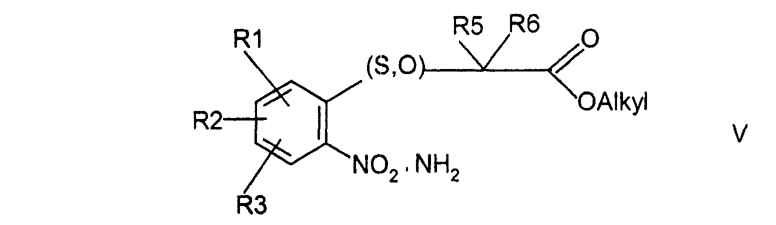 reduktiv cyclisiert.

Aromatische Thiole vom Typ III erhält man unter anderem wie in Chem. Pharm. Bull. 1991, 39, 2888 und der dort genannten Literatur beschrieben durch Umlagerung der entsprechenden Dimethylaminothiocarbamate.

Die Einführung der Substituenten R¹ bis R³ kann auf der Stufe der Verbindungen der Formel III oder II erfolgen.

Zur Herstellung von Verbindungen der Formel II mit R¹ in der Bedeutung eines mit NR^{7'}R^{8'} substituierten Alkylrestes oder falls R¹ und R² gemeinsam einen mit NR^{7'}R^{8'} substituierten Ring bedeuten, kann der Aldehyd oder das Keton des ensprechenden 1,4-Benzoxazin-3-thions bzw. 1,4-Benzothiazin-3-thions reduktiv aminiert werden. Wird die Einführung eines Heteroarylrestes NR^{7'}R^{8'} gewünscht, so kann das entsprechende Halogenderivat nucleophil substituiert werden. Ist eine primäre oder sekundäre Aminogruppe vorhanden, so kann es vorteilhaft sein, diese intermediär zu schützen, beispielsweise durch Einführung einer tert. Butoxycarbonylgruppe, die nach der Amidin-Bildung in üblicherweise abgespalten wird.

Neue Verbindungen wurden durch eine oder mehrere der folgenden Methoden charakterisiert: Schmelzpunkt, Massenspektroskopie, Infrarotspektroskopie, Nuklearmagnetische Resonanzspektroskopie (NMR). NMR Spektren wurden mit einem Bruker 300 MHz Gerät gemessen, die (deuterierten) Lösemittel werden jeweils angegeben und wie folgt abgekürzt: CDCl₃ (Chloroform), DMSO (Dimethylsulfoxid). Verschiebungen sind in delta und ppm angegeben. Es bedeuten: br (breites Signal), m (Multiplett, mehrere Signale), s (Singulett), d (Dublett), dd (Doppeldublett usw.), tr (Triplett), q (Quartett), H (Wasserstoffprotonen), J (Kopplungskonstante). Ferner bedeuten: THF (Tetrahydrofuran), DMF (N,N-Dimethylformamid), MeOH (Methanol), EE (Ethylacetat) ml (Milliliter), RT (Raumtemperatur). Alle Lösemittel sind p.A.Qualität, wenn nicht anders vermerkt. Alle Reaktionen werden unter Schutzgas ausgeführt, es sei denn es handelt sich um wässrige Lösungen. Schmelzpunkte werden in Grad Celsius angegeben und sind nicht korrigiert.

Die Erfindung betrifft auch die Verbindungen der Formel IIa, worin R³, R⁵, R⁶, X und Z die vorne genannte Bedeutung besitzen und R¹ und R² gemeinsam mit zwei benachbarten Kohlenstoffatomen einen 5-, 6-, 7- oder 8-gliedrigen Ring bilden, worin A einen gesättigten oder ungesättigten C₃₋₈-Alkylrenrest bedeutet, bei dem 1 oder 2 CH₂-Gruppen durch Carbonyl oder dessen Derivat ersetzt sein können. Sie sind wertvolle Zwischenverbindungen zur Herstellung von pharmakologisch wirksamen Verbindungen. Die Umwandlung der Zwischenprodukte in die Wirksubstanzen erfolgt nach den oben beschriebenen Verfahren.

Nachfolgend wird die Darstellung einiger Vorstufen, Zwischenprodukte und Produkte exemplarisch beschrieben.

### Ausgangsverbindungen (1)

### A) 6-Chlor-7-nitro-2-methyl-1,4-benzoxazin-3-on

7,52 g 2-Amino-4-chlor-5-nitrophenol werden zu einer Mischung von 1,6 g Natriumhydrid in 60 ml DMF gegeben und mit 1,1 Equivalenten D,L-2-Brompropionsäureethylester in 20 ml THF bei 5 °C versetzt. Es wird 4 Stunden gerührt. Die Mischung wird auf Wasser gegossen, mit Essigester extrahiert, die organische Phase mit Magnesiumsulfat getrocknet und eingeengt. Es resultieren 1,7 g Rohprodukt, das aus Isopropylether umkristallisiert wird. Die Ausbeute beträgt 72%.
[1H]-NMR (CDCl₃): 10,85 br 1 H, 7,55 s 1 H, 7,03 s 1 H, 4,60 q 1 H, 1,51 d 3H.
Nach dem hier und in B) genannten Verfahren lassen sich enantiomerenreine Verbindungen herstellen, die in weitere Umsetzungen eingesetzt werden können.
Auf die gleiche Weise wird hergestellt:
8-Chlor-6-nitro-2-methyl-1,4-benzoxazin-3-on
Die Ausbeute beträgt 51 %.
[1H]-NMR (DMSO): 11,1 br 1H, 7,98 d 1H, 7,72 d 1H, 5,05 q 1H, 1,53 d 3H.

### B) 6-Phenyl-2-methyl-1,4-benzoxazin-3-on

4,5 g 3-Amino-4-hydroxybiphenyl werden in 34 ml DMF mit 3,5 ml D,L-2-Brompropionsäureethylester (1.1 Equivalente ) sowie mit 4,48 g Kaliumcarbonat versetzt und bei 70°C 5 Stunden gerührt. Die Mischung wird auf Wasser gegossen, mit Essigester extrahiert, die organische Phase wird gewaschen, mit Magnesiumsulfat getrocknet und eingeengt. Es resultieren 1,7 g Rohprodukt, das aus Isopropanol umkristallisiert wird.
[1H]-NMR (DMSO): 10,65 br 1H, 7,6-7,3 m 8H, 4,72 q 1H, 1,48 d 3H.
Auf die gleiche Weise werden hergestellt:
6-Benzyl-2-methyl-1,4-benzoxazin-3-on
5-Nitro-2-methyl-1,4-benzoxazin-3-on
6-Nitro-2-methyl-1,4-benzoxazin-3-on
7-Nitro-2-methyl-1,4-benzoxazin-3-on
6-Nitro-2-ethyl-1,4-benzoxazin-3-on
7-Nitro-2-ethyl-1,4-benzoxazin-3-on
6-Cyano-2-methyl-1,4-benzoxazin-3-on
6-Trifluormethyl-2-methyl-1,4-benzoxazin-3-on
9-H-Fluoreno-[2,3-b]-2-methyl-1,4-oxazin-3-on
aus 3-Amino-2-hydroxyfluoren.
1H-Napht [2,1-b]-3-methyl-1,4-oxazin-2-on
1H-Napht [2,1-b]-3-ethyl-1,4-oxazin-2-on
aus 1-Aminonaphth-2-ol.
4H-Napht [2,3-b]-2-methyl-1,4-oxazin-3-on
4H-Napht [2,3-b]-2-ethyl-1,4-oxazin-3-on
4H-Napht [2,3-b]-2H-1,4-oxazin-3-on
4H-Napht [2,3-b]-2-propyl-1,4-oxazin-3-on
aus 3-Aminonaphth-2-ol.
4H-Napht [1,2-b]-2-methyl-1,4-oxazin-3-on
4H-Napht [1,2-b]-2-ethyl-1,4-oxazin-3-on
aus 2-Amino-1-naphthol, das durch Reduktion von 2-Nitro-1-naphthol erhalten wird.

### C) 6-(Imidazol-1-yl)-7-nitro-2-methyl-1,4-benzoxazin-3-on

485 mg 6-Chlor-7-nitro-2-methyl-1,4-benzoxazin-3-on werden mit 136 mg Imidazol und 332 mg Kaliumcarbonat in 15 ml DMF unter Zusatz von Kupferpulver 6 Stunden bei 180°C gerührt. Man verdünnt mit Essigester, extrahiert mit Sole und trocknet die organische Phase. Das Rohprodukt wird mittels Säulenchromatografie mit Essigester unter Zusatz von Ethanol gereinigt. Es resultieren 88 mg.
[1H]-NMR (DMSO): 11,2 br 1H, 7,83 s br 2H, 7,38 s br 1H, 7,1 s 1H, 6,98 s 1H, 4,93 q 1H, 1,55 d 3H.

### D) 5,6,7,8-Tetrahydro-3-nitro-2-naphthol und 5,6,7,8-Tetrahydro-1-nitro-2-naphthol

14,8 g 5,6,7,8-Tetrahydro-2-naphthol werden mit 4,35 ml rauchender Salpetersäure in 100 ml Eisessig nitriert. Die Innentemperatur sollte dabei 10°C nicht überschreiten. Nach 1 Stunde gießt man die Mischung auf Eiswasser, extrahiert mit Dichlormethan und anschließend mit Essigester, die vereinten organischen Phasen werden mit Wasser gewaschen, getrocknet und eingeengt. Säulenchromatografie mit Hexan / Essigester liefert zwei Regioisomere in je 25% Ausbeute sowie 5,6,7,8-Tetrahydro-1,3-dinitro-2-naphthol. Die in Chem. Pharm. Bull. 1991, 39, 2896 genannte Methode liefert ähnliche Ergebnisse.
Auf die gleiche Weise werden hergestellt:
6-Nitro-5-indanol sowie 4-Nitro-5-indanol aus 5-Indanol in zusammen 57% Ausbeute. Die Verbindungen werden gemeinsam wie in Beispiel B) beschrieben alkyliert, wie in Beispiel G) beschrieben cyclisiert und erst die Produkte dieser Reaktion getrennt.
5-Hydroxy-6-nitro-benzo-1,3-dioxol
wird wie in J. Org. Chem. 1959, 24, 327 beschrieben hergestellt.
In Eur. J. Med. Chem. Chim. Therap. 1974, 9, S. 26 wird die Darstellung von 6-Hydroxy-1,2,3,4-tetrahydro-1,4-methanonaphthalin beschrieben, das wie oben angegeben nitriert wird. Man trennt die Mischung der Nitroverbindungen chromatographisch auf und erhält 5,6,7,8-Tetrahydro-5,8-methano-3-nitronaphth-2-ol.
Aus 5,5,8,8-Tetramethyl-5,6,7,8-tetrahydro-naphth-2-ol erhält man 5,5,8,8-Tetramethyl-5,6,7,8-tetrahydro-3-nitro-naphth-2-ol.

### E) 6-Nitro-5-methoxy-indan-1-on

0,81 g 5-Methoxy-indan-1-on werden mit 1,33 g Kupfer-II-nitrat in 7,5 ml Acetanhydrid bei Raumtemperatur nitriert. Nach 3 Stunden gießt man auf Eiswasser, extrahiert mehrfach mit Essigester, wäscht die organische Phase mit Hydrogencarbonatlösung, dann mit Sole, trocknet und engt ein. Säulenchromatografie mit Hexan / Essigester liefert zwei Regioisomere, man erhält 6-Nitro-5-methoxy-indan-1-on in 38% Ausbeute.
[1H]-NMR (CDCl₃):8,19 s 1H, 7,11 s 1H, 4,05 s 3H, 3,21 dd 2H, 2,75 dd 2H.
Auf die gleiche Weise werden hergestellt:
5-Nitro-6-methoxy-indan-1-on
7-Nitro-6-methoxy-2-tetralon
6-Nitro-7-methoxy-2-tetralon
7-Nitro-6-methoxy-1-tetralon
Die Methoxyverbindungen werden in gewohnter Weise mit HBr oder Lithiumchlorid in DMF zu den substituierten Hydroxyaromaten gespalten.

### F) 2-(5,6,7,8-Tetrahydro-3-nitro-2-naphthoxy)-propionsäureethylester

Man alkyliert 5,6,7,8-Tetrahydro-3-nitro-2-naphthol nach der in Beispiel A) genannten allgemeinen Vorschrift mit D,L-2-Brompropionsäureethylester. Nach Säulenchromatografie mit Hexan / Essigester erhält man die Nitroverbindung in 43% Ausbeute.
[1H]-NMR (CDCl₃): 7,60 s 1H, 6,65 s 1H, 4,77 q 1H, 4,2 q 2H, 2,74 und 1,8 je m 4H, 1,59 d 3H, 1,25 tr 3H.
Auf die gleiche Weise werden hergeatellt:
2-(5,6,7,8-Tetrahydro-1-nitro-2-naphthoxy)-propionsäureethylester
2-(5,6,7,8-Tetrahydro-3-nitro-2-naphthoxy)-buttersäureethylester
2-(5,6,7,8-Tetrahydro-1-nitro-2-naphthoxy)-buttersäureethylester
2-(5,6,7,8-Tetrahydro-3-nitro-2-naphthoxy)-essigsäureethylester
2-(5,6,7,8-Tetrahydro-1-nitro-2-naphthoxy)-essigsäureethylester
2-(2-Nitro-4,5-methylendioxy-phenoxy)-propionsäureethylester
2-(6-Nitro-5-indanoxy)-propionsäureethylester und 2-(4-Nitro-5-indanoxy)-propionsäureethylester
2-(6-Nitro-5-indanoxy)-buttersäureethylester und 2-(4-Nitro-5-indanoxy)-buttersäureethylester
2-(6-oxo-5,6,7,8-tetrahydro-1-nitro-2-naphthoxy)-propionsäureethylester
2-(7-oxo-5,6,7,8-Tetrahydro-1-nitro-2-naphthoxy)-propionsäureethylester
2-(8-oxo-5,6,7,8-Tetrahydro-1-nitro-2-naphthoxy)-propionsäureethylester
2-(5,6,7,8-Tetrahydro-5,8-methano-3-nitro-2-naphthoxy)-propionsäureethylester
2-(5,5,8,8-Tetramethyl-5,6,7,8-tetrahydro-3-nitro-2-naphthoxy)-propionsäureethylester
2-(1-Keto-6-Nitro-5-indanoxy)-propionsäureethylester

### G) 2-Methyl-6,7,8,9-tetrahydronapht [2,3-b]-1,4-oxazin-3(4H)-on

In einer Mischung von 10 ml Eisessig mit 1,25 ml Wasser werden 2-(5,6,7,8-Tetrahydro-3-nitro-2-naphthoxy)-propionsäureethylester gelöst und portionsweise mit 960 mg Eisenpulver versetzt. Es wird erhitzt, abgekühlt und auf Wasser gegossen. Die Kristalle werden abgesaugt und mit Wasser gewaschen, erneut mit Essigester gelöst und mit Sodalösung gewaschen, die organische Phase mit Sole gewaschen, mit Magnesiumsulfat getrocknet und eingeengt. Es resultieren 740 mg, Ausbeute 81%.
[1H]-NMR (CDCl₃): 9,61 br 1H, 6,69 s 1H, 6,51 s 1H, 4,61 q 1H, 2.68 und 1,75 je m 4H, 1,56 d 3H.
Auf die gleiche Weise werden hergestellt:
2-Ethyl-6,7,8,9-tetrahydro-napht [2,3-b]-1,4-oxazin-3(4H)-on
3-Methyl-6,7,8,9-tetrahydro-napht [2,1-b]-1,4-oxazin-2(1H)-on
3-Ethyl-6,7,8,9-tetrahydro-napht [2,1-b]-1,4-oxazin-2(1H)-on
6,7-Cyclopenteno-2-methyl-1,4-benzoxazin-3-on
6,7-Cyclopenteno-2-ethyl-1,4-benzoxazin-3-on
5,6-Cyclopenteno-2-methyl-1,4-benzoxazin-3-on
5,6-Cyclopenteno-2-ethyl-1,4-benzoxazin-3-on
6,7-(Methylendioxy)-2-methyl-1,4-benzoxazin-3-on
6,7-(Methylendioxy)-2-ethyl-1,4-benzoxazin-3-on
6-Cyclohexyl-2-methyl-1,4-benzoxazin-3-on
7-(1-Morpholinyl)-2-methyl-1,4-benzoxazin-3-on
6-Keto-2-methyl-6,7,8,9-tetrahydro-naphth-[2,3-b]-1,4-oxazin-3-(4H)-on
7-Keto-2-methyl-6,7,8,9-tetrahydro-naphth-[2,3-b]-1,4-oxazin-3-(4H)-on
8-Keto-2-methyl-6,7,8,9-tetrahydro-naphth-[2,3-b]-1,4-oxazin-3-(4H)-on
2-Methyl-6,7,8,9-tetrahydro-6,9-methano-naphth-[2,3-b]-1,4-oxazin-3-(4H)-on
6-Keto-6,7-cyclopenteno-2-methyl-1,4-benzoxazin-3-on
6,6,9,9-Tetramethyl-6,7,8,9-tetrahydro-naphth-[2,3-b]-1,4-oxazin-3-(4H)-on

### H) 5,6,7,8-Tetrahydro-3-amino-naphthalin-2-thiol

5,6,7,8-Tetrahydro-[2-(N,N-dimethylaminothiocarbamoyl)oxy]-3-nitronaphthalin wird nach der Vorschrift von Chem. Pharm. Bull. 1991, 39, 2888 und der dort genannten Literatur synthetisiert aus 5,6,7,8-Tetrahydro-3-nitronaphth-2-ol und N,N-Dimethylaminothiocarbamoylchlorid. [1H]-NMR (CDCl₃): 7,87 s 1H, 6,92 s 1H, 3,48 und 3,39 je s 3H, 2,85 und 1,85 je m 4H.
Nachfolgende Umlagerung bei 180°C liefert 5,6,7,8-Tetrahydro-[2-(N,N-dimethylaminocarbamoyl)thio]-3-nitronaphthalin. Die Ausbeute beträgt 80%. Daraus erhält man nach Reduktion mit Lithiumaluminiumhydrid 5,6,7,8-Tetrahydro-3-aminonaphthalin-2-thiol, das als Rohprodukt eingesetzt wird.

Auf die gleiche Weise werden hergestellt:
6-Aminoindan-5-thiol
Nach der Vorschrift B) synthetisiert man aus diesen analog:
2-Methyl-6,7,8,9-tetrahydro-naphto [2,3-b]-1,4-thiazin-3(4H)-on
2-Ethyl-6,7,8,9-tetrahydro-naphto [2,3-b]-1,4-thiazin-3(4H)-on
6,7-Cyclopenteno-2-methyl-1,4-benzothiazin-3-on
6,7-Cyclopenteno-2-ethyl-1,4-benzothiazin-3-on
Aus den korrespondierenden käuflichen Aminothiophenolen erhält man mit dieser Methode ferner:
6-Trifluormethyl-2-methyl-1,4-benzothiazin-3-on
6-Trifluormethyl-2-ethyl-1,4-benzothiazin-3-on
6-Trifluormethyl-2-propyl-1,4-benzothiazin-3-on

### I) 2-Nitro-4,5-(pentamethylen)phenol

Eine Lösung von 3.2 g (20 mmol) 4,5-(Pentamethylen)phenol (V. Prelog, L. Ruzicka, O. Metzler, *Helv. Chim. Acta* 1947, *30*, 1883) in 40 mL Eisessig wird mit 1.3 g (20 mmol) 100 proz. Salpetersäure versetzt und 1.5 h bei Raumtemp. gerührt. Das Reaktionsgemisch wird mit Wasser verdünnt und mit Ether extrahiert. Die vereinigten Etherextrakte werden mit Wasser gewaschen, getrocknet (Na₂SO₄) und i. Vak. eingeengt. Säulenchromatographie an Kieselgel mit Hexan-Ether liefert 1.83 g Produkt (48% d.Th.).
Schmp. 88 °C.
Analog werden hergestellt:
4,5-(Hexamethylen)-2-nitrophenol
(41% d.Th.). ¹H-NMR (CDCl₃): 1.37 (m, 4H), 1.60 - 1.78 (m, 4H), 2.75 (m, 4H), 6.90 (s, 1H), 7.84 (s, 1H), 10.47 (s, 1H).

### J1) 2-[2-Nitro-4,5-(pentamethylen)phenoxy]propionsäure-ethylester

Zu einer Lösung von 1.71 g (8.25 mmol) 2-Nitro-4,5-(pentamethylen)phenol und 2.16 g (8.25 mmol) Triphenylphosphin in 55 mL THF wird eine Lösung von 1.3 mL (8.25 mmol) Azodicarbonsäure-diethylester und 1.46 mL (12.4 mmol) Milchsäure-ethylester in 15 mL THF gegeben. Nach dreistündigem Rühren bei Raumtemp. werden weitere 1.08 g (4.13 mmol) Triphenylphosphin, 0.65 mL (4.13 mmol) Azodicarbonsäure-diethylester und 0.73 mL (6.19 mmol) Milchsäure-ethylester dazugegeben. Nach 2 h wird das Reaktionsgemisch mit Essigester (200 mL) verdünnt, mit Wasser (2 x 40 mL) gewaschen, getrocknet (Na₂SO₄) und i. Vak. eingeengt. Säulenchromatographie an Kieselgel mit Hexan-Ether liefert 2.27 g Produkt (90% d. Th.).
¹H-NMR (CDCl₃): 1.25 (t, 3H), 1.63 (m, 4H), 1.67 (d, 3H), 1.83 (m, 2H), 2.77 (m, 4H), 4.22 (q, 2H), 4.80 (q, 1H), 6.72 (s, 1H), 7.63 (s, 1H).
Analog werden erhalten:
2-[4,5-(Hexamethylen)-2-nitrophenoxy]propionsäure-ethylester
   (86% d. Th.). 1H-NMR (CDCl₃): 1.23 (t, 3H), 1.35 (m, 4H), 1.60 -1.72 (m, 4H), 1.68 (d, 3H), 2.73 (m, 4H), 4.21 (qd, 2H), 4.82 (q, 1H), 6.71 (s, 1H), 7.64 (s, 1H).
Nach dem hier genannten Verfahren lassen sich enantiomerenreine Verbindungen wie (2S)-2-[2-Nitro-4,5-(pentamethylen)phenoxy]propionsäure-ethylester oder (2R)-2-[2-Nitro-4,5-(pentamethylen)phenoxy]propionsäure-ethylester mit ee bis 98% herstellen, die in weitere Umsetzungen eingesetzt werden können.

### J2) 2-Methyl-6,7-pentamethylen-1,4-dihydrobenzoxazin-3-on

Eine Suspension von 2.20 g (7.2 mmol) 2-[2-Nitro-4,5-(pentamethylen)phenoxy]propionsäure-ethylester, 5.94 g (90.9 mmol) Zink und 1.8 g Ammoniumchlorid in 400 mL THF-Ethanol-Wasser (1:3:2) wird 18 h bei Raumtemp. gerührt. Der Ansatz wird filtriert und das Filtrat i. Vak. eingeengt. Säulenchromatographie an Kieselgel mit Hexan-Ether liefert 1.53 g Produkt (93% d. Th.).
Schmp. 215°C.
Analog:
6,7-Hexamethylen-2-methyl-1,4-dihydrobenzoxazin-3-on
(73% d. Th.). Schmp. 194-195 °C.J3) 6-(Propionyl)-2-methyl-1,4-benzoxazin-3-(4H)-on

13,3 g Aluminium-III-chlorid werden tropfenweise mit 2,2 ml DMF versetzt. Man gibt 1,63 g 2-Methyl-1,4-benzoxazin-3-(4H)-on portionsweise dazu und dann vorsichtig 1 Equivalent Propionylchlorid. Die Mischung wird bei 70°C nachgerührt. Nach 3 Stunden wird auf viel Eiswasser gegossen, angesäuert, mit Wasser die Kristalle gewaschen, in Essigester aufgenommen und diese Phase erneut mit Wasser gewaschen. Nach Trocknen und Einengen erhält man 89% Ausbeute.
[1H]-NMR (DMSO): 10,8 br 1H, 7,65 dd, 7,54 d, 7,10 d je 1H, 4,82 q 1H,3,0 q 2H, 1,51 d 3H, 1,12 tr 3H.
Auf die gleiche Weise werden hergestellt:
6-(Styrylcarboxy)-2-methyl-1,4-benzoxazin-3-(4H)-on
6-(3-Carboxy-propionyl)-2-methyl-1,4-benzoxazin-3-(4H)-on
6-(4-Ethoxycarbonyl-butyryl)-2-methyl-1,4-benzoxazin-3-(4H)-on
6-(2-Chloracetyl)-2-methyl-1,4-benzoxazin-3-(4H)-on

### K) 6-(1-Hydroxyprop-1-yl)-2-methyl-1,4-benzoxazin-3-(4H)-on

6-(Propionyl)-2-methyl-1,4-benzoxazin-3-(4H)-on (1,1 g) wird mit einem halben Equivalent Natriumborhydrid in 30 ml Ethanol/THF 1:1 drei Stunden bei Raumtemperatur gerührt. Die Mischung wird auf Wasser gegossen, mit Essigester extrahiert, die organische Phase mit Magnesiumsulfat getrocknet und eingeengt. Es resultieren 95% Ausbeute.
Für die Umsetzung zum Thion und Amidin wird in üblicher Weise das Carbinol als Silylether geschützt, der beim Endprodukt abgespalten wird.

### L) 6-Brom-2-methyl-1,4-benzoxazin-3-(4H)-on

In 56 ml Eisessig werden 6 g 2-Methyl-1,4-benzoxazin-3-(4H)-on bei 0°C vorgekühlt und tropfenweise mit 1,9 ml Brom in 19 ml Eisessig versetzt. Man läßt auf Raumtemperatur kommen und rührt weitere 8 Stunden. Die Mischung wird auf Eiswasser gegossen, die Kristalle abgesaugt und mit Wasser gewaschen. Es resultieren 9,3 g Rohprodukt, das aus Ethanol / Wasser umkristallisiert wird. Man erhält als Nebenprodukt 7-Brom-2-methyl-1,4-benzoxazin-3-(4H)-on. Alternativ kann auch 4-Brom-2-aminophenol nach den beschriebenen Verfahren umgesetzt werden.

### M) 6-(2-Thienyl)-2-methyl-1,4-benzoxazin-3-(4H)-on

315 mg 6-Brom-2-methyl-1,4-benzoxazin-3-(4H)-on werden in 4 ml DME mit 0,2 g 2-Thiophenboronsäure sowie 150 mg Tetrakis-triphenylphosphin-palladium (0) und 340 mg Natriumhydrogencarbonat (gelöst in 4 ml Wasser) versetzt. Man rührt einige Zeit bei 100°C. Das Produkt wird mit Essigester verdünnt, 3 mal mit Wasser extrahiert, mit Magnesiumsulfat getrocknet. Man engt ein. Das Rohprodukt wird durch Säulenchromatografie mit Hexan / Essigester Gemisch gereinigt und liefert reines 33% Produkt.
Auf die gleiche Weise werden hergestellt:
6-(3-Thienyl)-2-methyl-1,4-benzoxazin-3-(4H)-on
6-(3-Pyridyl)-2-methyl-1,4-benzoxazin-3-(4H)-on
aus 3-Pyridyldiethylboran
6-(Styryl)-2-methyl-1,4-benzoxazin-3-(4H)-on
aus trans-Styrolboronsäure

### N) 3-[2-Methyl-3-keto-1,4-benzoxazin-6-yl]-acrylamid

726 mg 6-Brom-2-methyl-1,4-benzoxazin-3-(4H)-on werden in 1,5 ml Triethylamin mit 0,267 g Acrylamid sowie 8 mg Palladium-II-acetat und 46 mg Tri-orthotoluyl-phosphin einige Stunden bei 100°C gerührt. Das Produkt wird in Methanol aufgenommen und durch Säulenchromatografie mit Ethanol/ Essigester Gemisch gereinigt. Ausbeute 36%.
[1H]-NMR (DMSO):10,7 br 1H, 7,4 bis 7 br NH, 7,32 d 1H, 7,15 m 2H, 7,1 d 1H, 6,95 d 1H, 6,5 d J=16Hz 1H,4,7 q 1H, 1,47 d7.
Auf die gleiche Weise werden hergestellt:
3-[2-Methyl-3-keto-1,4-benzoxazin-7-yl]-acrylamid
3-[2-Methyl-3-keto-1,4-benzoxazin-6-yl]-acrylnitril

### P)

Aus 6-(Propionyl)-2-methyl-1,4-benzoxazin-3-(4H)-on erhält man mit O-Methylhydroxylamin-Hydrochlorid
2-Methyl-6-([1-methoxyimino]-prop-1-yl)-1,4-benzoxazin-3-on.

### Q)

Aus 6-(Propionyl)-2-methyl-1,4-benzoxazin-3-(4H)-on synthetisiert man mit Toluolsulfonylchlorid in Pyridin das Tosylat des Carbinols, setzt mit Natriumazid in DMSO zum Azid um und reduziert mit Zinn-II-chlorid zu 2-Methyl-6-(1-aminoprop-1-yl)
1,4-benzoxazin-3-on.

### R)Aus 6-(Propionyl)-2-methyl-1,4-benzoxazin-3-(4H)-on läßt sich mit Methylmagnesiumbromid in guten Ausbeuten 2-Methyl-6-(1-methyl-1-hydroxy-prop-1-yl)-1,4-benzoxazin-3-on darstellen, das mit tert-Butyl-dimethylsilylchlorid verethert wird.

### S) 6-Phenyl-2-methyl-1,4-benzoxazin-3-thion

1,62g 6-Phenyl-2-methyl-1,4-benzoxazin-3-on werden mit 0,5 g Phosphorpentasulfid in 6 ml Pyridin p.a. erhitzt. Das Produkt wird mit Wasser verdünnt, 3 mal mit Essigester extrahiert, die organische Phase mit Sole gewaschen und mit Magnesiumsulfat getrocknet. Man engt ein. Das Rohprodukt entspricht100% Ausbeute. Säulenchromatografie mit Hexan / Essigester Gemisch liefert reines Produkt.
Auf die gleiche Weise werden hergestellt:
6-Chlor-7-nitro-2-methyl-1,4-benzoxazin-3-thion
8-Chlor-6-nitro-2-methyl-1,4-benzoxazin-3-thion
6-Benzyl-2-methyl-1,4-benzoxazin-3-thion
5-Nitro-2-methyl-1,4-benzoxazin-3-thion
6-Nitro-2-methyl-1,4-benzoxazin-3-thion
7-Nitro-2-methyl-1,4-benzoxazin-3-thion
6-Nitro-2-ethyl-1,4-benzoxazin-3-thion
6-Brom-2-methyl-1,4-benzoxazin-3-(4H)-thion
7-Nitro-2-ethyl-1,4-benzoxazin-3-thion
6-Cyano-2-methyl-1,4-benzoxazin-3-thion
6-Trifluormethyl-2-methyl-1,4-benzoxazin-3-thion
9-H-Fluoreno-[2,3-b]-2-methyl-1,4-oxazin-3-thion
1H-Napht [2,1-b]-3-methyl-1,4-benzoxazin-2-thion
1H-Napht [2,1-b]-3-ethyl-1,4-benzoxazin-2-thion
4H-Napht [2,3-b]-2-methyl-1,4-benzoxazin-3-thion
4H-Napht [2,3-b]-2-ethyl-1,4-benzoxazin-3-thion
4H-Napht [2,3-b]-1,4-benzoxazin-3-thion
4H-Napht [2,3-b]-2-propyl-1,4-oxazin-3-thion
4H-Napht [1,2-b]-2-methyl-1,4-oxazin-3-thion
4H-Napht [1,2-b]-2-ethyl-1,4-oxazin-3-thion
6-(Imidazol-1-yl)-7-nitro-2-methyl-1,4-benzoxazin-3-thion
6,7-Cyclopenteno-2-methyl-1,4-benzoxazin-3-thion
6,7-Cyclopenteno-2-ethyl-1,4-benzoxazin-3-thion
5,6-Cyclopenteno-2-methyl-1,4-benzoxazin-3-thion
5,6-Cyclopenteno-2-ethyl-1,4-benzoxazin-3-thion
6,7-(Methylendioxy)-2-methyl-1,4-benzoxazin-3-thion
6,7-(Methylendioxy)-2-ethyl-1,4-benzoxazin-3-thion
6-Cyclohexyl-2-methyl-1,4-benzoxazin-3-thion
7-(1-Morpholinyl)-2-methyl-1,4-benzoxazin-3-thion
2-Ethyl-6,7,8,9-tetrahydro-napht [2,3-b]-1,4-oxazin-3(4H)-thion
2-Methyl-6,7,8,9-tetrahydro-napht [2,3-b]-1,4-oxazin-3(4H)-thion
3-Methyl-6,7,8,9-tetrahydronapht [2,1-b]-1,4-oxazin-2(1H)-thion
3-Ethyl-6,7,8,9-tetrahydronapht [2,1-b]-1,4-oxazin-2(1H)-thion

2-Methyl-6,7,8,9-Tetrahydro-naphto[2,3-b]-1,4-thiazin-3(4H)-thion
2-Ethyl-6,7,8,9-Tetrahydro-naphto [2,3-b]-1,4-thiazin-3(4H)-thion
6,7-Cyclopenteno-2-methyl-1,4-benzothiazin-3-thion
6,7-Cyclopenteno-2-ethyl-1,4-benzothiazin-3-thion
6-Trifluormethyl-2-methyl-1,4-benzothiazin-3-thion
6-Trifluormethyl-2-ethyl-1,4-benzothiazin-3-thion
6-Trifluormethyl-2-propyl-1,4-benzothiazin-3-thion

6-Keto-2-methyl-6,7,8,9-tetrahydro-naphth-1,4-oxazin-3-(4H)-thion
7-Keto-2-methyl-6,7,8,9-tetrahydro-naphth-1,4-oxazin-3-(4H)-thion
8-Keto-2-methyl-6,7,8,9-tetrahydro-naphth-1,4-oxazin-3-(4H)-thion
2-Methyl-6,7,8,9-tetrahydro-6,9-methano-naphth-1,4-oxazin-3-(4H)-thion
6-Keto-6,7-cyclopenteno-2-methyl-1,4-benzoxazin-3-thion
6,6,9,9-Tetramethyl-6,7,8,9-tetrahydro-naphth-[2,3-b]-1,4-oxazin-3-(4H)-thion

3-[2-Methyl-3-thio-1,4-benzoxazin-6-yl]-acrylamid
3-[2-Methyl-3-thio-1,4-benzoxazin-6-yl]-acrylnitril

6-(2-Thienyl)-2-methyl-1,4-benzoxazin-3-(4H)-thion
6-(3-Thienyl)-2-methyl-1,4-benzoxazin-3-(4H)-thion
6-(3-Pyridyl)-2-methyl-1,4-benzoxazin-3-(4H)-thion
6-(Styryl)-2-methyl-1,4-benzoxazin-3-(4H)-thion
6-(Propionyl)-2-methyl-1,4-benzoxazin-3-(4H)-thion
6-(Styrylcarboxy)-2-methyl-1,4-benzoxazin-3-(4H)-thion
6-(3-Carboxy-propionyl)-2-methyl-1,4-benzoxazin-3-(4H)-thion
6-(4-Ethoxycarbonyl-butyryl)-2-methyl-1,4-benzoxazin-3-(4H)-thion
6-(2-Chloracetyl)-2-methyl-1,4-benzoxazin-3-(4H)-thion

### S1) 2-Methyl-6,7-pentamethylen-1,4-dihydrobenzoxazin-3-thion

Eine Lösung von 0.41 g (1.8 mmol) 2-Methyl-6,7-pentamethylen-1,4-dihydrobenzoxazin-3-on in 40 mL DME wird mit 0.78 g (1.9 mmol) Lawessons Reagenz versetzt. Nach 5 h Rühren bei Raumtemp. wird der Ansatz i. Vak. eingeengt. Säulenchromatographie an Kieselgel mit Hexan-Ether liefert 0.27 g Produkt (63% d. Th.). ¹H-NMR (CDCl₃): 1.52 - 1.70 (m, 4H), 1.61 (d, 3H), 1.80 (m, 2H), 2.71 (m, 4H), 4.98 (q, 1H), 6.61 (s, 1H), 6.72 (s, 1H), 9.83 (br.s, 1H).
Analog entsteht:
6,7-Hexamethylen-2-methyl-1,4-dihydrobenzoxazin-3-thion
(83% d. Th.) ¹H-NMR (CDCl₃): 1.37 (m, 4H), 1.55 - 1.73 (m, 4H), 1.63 (d, 3H), 2.69 (t, 4H), 4.98 (q, 1H), 6.60 (s, 1H), 6.75 (s, 1H), 9.64 (br.s, 1H).

### T) 6-Amino-2-ethyl-1,4-benzoxazin-3-thion

1 g 6-Nitro-2-ethyl-1,4-benzoxazin-3-thion werden mit 7,6 ml Eisessig und 1 ml Wasser gelöst und mit 950 mg Eisenpulver versetzt. Nach einigen Stunden bei Raumtemperatur wird auf Wasser gegossen. Die Kristalle werden abgesaugt und mit Wasser gewaschen, erneut mit Essigester gelöst und mit Sole neutral gewaschen, die organische Phase mit Magnesiumsulfat getrocknet und eingeengt. Es resultieren nach Säulenchromatografie mit Hexan / Essigester 811 mg Ausbeute.
Auf die gleiche Weise werden hergestellt:
5-Amino-2-ethyl-1,4-benzoxazin-3-thion
5-Amino-2-methyl-1,4-benzoxazin-3-thion
6-Amino-2-methyl-1,4-benzoxazin-3-thion
7-Amino-2-methyl-1,4-benzoxazin-3-thion

### U) N-[(2-Ethyl-1,4-benzoxazin-3-thion-6-yl]-phenylcarboximidamid Hydroiodid

Man gibt zu 150 mg 6-Amino-2-ethyl-1,4-benzoxazin-3-thion in 3 ml Isopropanol 201 mg S-Methylthio-benzamid Hydroiodid und kocht 4 Stunden Rückfluss. Das Rohprodukt kann eingeengt und für Folgereaktionen eingesetzt werden.
Auf die gleiche Weise werden hergestellt (teilweise als Hydroiodide):
N-[(2-Methyl-1,4-benzoxazin-3-thion-7-yl]- 4-methyl-phenylcarboximidamid
N-[(2-Ethyl-1,4-benzoxazin-3-thion-7-yl]- 4-methyl-phenylcarboximidamid
N-[(2-Methyl-1,4-benzoxazin-3-thion-6-yl]- 4-methyl-phenylcarboximidamid
N-[(2-Ethyl-1,4-benzoxazin-3-thion-6-yl]-4-methyl-phenylcarboximidamid
N-[(2-Methyl-1,4-benzoxazin-3-thion-7-yl]- phenylcarboximidamid
N-[(2-Ethyl-1,4-benzoxazin-3-thion-7-yl]- phenylcarboximidamid
N-[(2-Methyl-1,4-benzoxazin-3-thion-6-yl]-phenylcarboximidamid
N-[(2-Methyl-1,4-benzoxazin-3-thion-7-yl]- 2,4-dichlor-phenylcarboximidamid
N-[(2-Ethyl-1,4-benzoxazin-3-thion-7-yl]- 2,4-dichlor-phenylcarboximidamid
N-[(2-Methyl-1,4-benzoxazin-3-thion-6-yl]-2,4-dichlor-phenylcarboximidamid
N-[(2-Ethyl-1,4-benzoxazin-3-thion-6-yl]-2,4-dichlor-phenylcarboximidamid
N-[(2-Methyl-1,4-benzoxazin-3-thion-7-yl]-(2-thienyl)carboximidamid
N-[(2-Ethyl-1,4-benzoxazin-3-thion-7-yl]- (2-thienyl)carboximidamid
N-[(2-Methyl-1,4-benzoxazin-3-thion-6-yl]-(2-thienyl)carboximidamid
N-[(2-Ethyl-1,4-benzoxazin-3-thion-6-yl]-(2-thienyl)carboximidamid

### V) 2-Ethyl-6-(1-pyrrolo)-1,4-benzoxazin3-thion

Zu 100 mg 6-Amino-2-ethyl-1,4-benzoxazin-3-thion in 1 ml Essigsäure gibt man 1 Equivalent 2,5-Dimethoxytetrahydrofuran und erhitzt bis zum Rückfluss. Die Mischung wird auf Sodalösung gegossen, mit Essigester extrahiert, die organische Phase neutral gewaschen, mit Magnesiumsulfat getrocknet und eingeengt. Es resultieren 77 mg Produkt, das weiter eingesetzt wird.

### W) N-[(2-Ethyl-1,4-benzoxazin-3-thio-6-yl]-phenylharnstoff

Man gibt zu 150 mg 6-Amino-2-ethyl-1,4-benzoxazin-3-thion in 2 ml THF einen geringen Überschuss Phenylisocyanat und rührt bei Eisbadtemperatur 4 Stunden. Das Rohprodukt kann eingeengt und für Folgereaktionen eingesetzt werden.
Auf die gleiche Weise werden unter Verwendung der Isocyanate beziehungsweise Isothiocyanate hergestellt:
N-[6-(2-Ethyl-3-thio-1,4-benzoxazinyl)-N-phenylthioharnstoff
N-[6-(2-Methyl-3-thio-1,4-benzoxazinyl)-N-phenylharnstoff
N-[6-(2-Methyl-3-thio-1,4-benzoxazinyl)-N-phenylthioharnstoff
N-[6-(2-Ethyl-3-thio-1,4-benzoxazinyl)-N-cyclohexylharnstoff
N-[6-(2-Ethyl-3-thio-1,4-benzoxazinyl)-N-2-methyl-4-chlorphenylharnstoff
N-[6-(2-Ethyl-3-thio-1,4-benzoxazinyl)-N-2-chlorphenylharnstoff
N-[7-(2-Ethyl-3-thio-1,4-benzoxazinyl)-N-phenylthioharnstoff
N-[7-(2-Methyl-3-thio-1,4-benzoxazinyl)-N-phenylharnstoff
N-[7-(2-Ethyl-3-thio-1,4-benzoxazinyl)-N-phenylharnstoff
N-[7-(2-Methyl-3-thio-1,4-benzoxazinyl)-N-phenylthioharnstoff
N-[7-(2-Ethyl-3-thio-1,4-benzoxazinyl)-N-cyclohexylharnstoff
N-[7-(2-Ethyl-3-thio-1,4-benzoxazinyl)-N-2-methyl-4-chlorphenylharnstoff
N-(7-(2-Ethyl-3-thio-1,4-benzoxazinyl)-N-2-chlorphenylharnstoff

### Beispiel 1

### 6-Phenyl-2-methyl-3-amino-1,4-benzoxazin

Man rührt 1,7 g 6-Phenyl-2-methyl-1,4-benzoxazin-3-thion in 10 ml ges. Ammoniaklösung in Methanol (kommerziell erhältlich). Nach 2 Tagen bei Raumtemperatur erhält man 100% Rohprodukt nach einengen. Mittels Säulenchromatografie mit Hexan/ Essigester wird das Produkt gereinigt. Es resultieren 59% Ausbeute.
[1H]-NMR (DMSO): 7,58 m 2H, 7,42 dd 2H, 7,3 m 1H, 7,10 m 2H , 6,85 d 1H, 6,7 br NH, 4,71 q 1H, 1,32 d 3H.
Auf die gleiche Weise werden hergestellt:
6-Chlor-7-nitro-2-methyl-3-amino-1,4-benzoxazin
[1H]-NMR (DMSO): 7,53 s 1H, 7,01 s 1H, 4,82 q 1H, 1,33 d 3H.
6-Methylthio-7-nitro-2-methyl-3-amino-1,4-benzoxazin
6-Methoxy-7-nitro-2-methyl-3-amino-1,4-benzoxazin
6-(Imidazol-1-yl)-7-nitro-2-methyl-3-amino-1,4-benzoxazin
8-Chlor-6-nitro-2-methyl-3-amino-1,4-benzoxazin
   [1H]-NMR (DMSO): 7,4 br 2H, 7,55 d 1H, 7,81 d 1H, 5,02 q 1H, 1,38 d 3H.
6-Benzyl-2-methyl-3-amino-1,4-benzoxazin
5-Nitro-2-methyl-3-amino-1,4-benzoxazin
6-Nitro-2-methyl-3-amino-1,4-benzoxazin
7-Nitro-2-methyl-3-amino-1,4-benzoxazin
6-Nitro-2-ethyl-3-amino-1,4-benzoxazin
7-Nitro-2-ethyl-3-amino-1,4-benzoxazin
6-Cyano-2-methyl-3-amino-1,4-benzoxazin
6-Trifluormethyl-2-methyl-3-amino-1,4-benzoxazin
9-H-Fluoreno-[2,3-b]-3-amino-2-methyl-1,4-oxazin
1H-Napht [2,1-b]methyl-3-amino-1,4-oxazin
1H-Napht [2,1-b]ethyl-3-amino-1,4-oxazin
4H-Napht [2,3-b]-2-methyl-3-amino-1,4-oxazin
4H-Napht [2,3-b]-2-ethyl-3-amino-1,4-oxazin
4H-Napht [2,3-b]-2-n-propyl-3-amino-1,4-oxazin
4H-Napht [1,2-b]-2-methyl-3-amino-1,4-oxazin
4H-Napht [1,2-b]-2-ethyl-3-amino-1,4-oxazin
6,7-Cyclopenteno-2-methyl-3-amino-1,4-benzoxazin
   [1H]-NMR (DMSO): 6,71 s 1H, 6,63 s 1H, 6,5 br NH, 4,59 q 1H, 2,75 m 4H, 1,95 pentett 2H, 1,7 m 2H, 1,25 d 3H.
6,7-Cyclopenteno-2-ethyl-3-amino-1,4-benzoxazin
   [1H]-NMR (DMSO): 6,85 s 1H, 6,72 s 1H, 4,34 dd 1H, 2,8 m 4H, 2,05 pentett 2H, 1,7 m 2H, 1,05 tr 3H.
5,6-Cyclopenteno-2-methyl-3-amino-1,4-benzoxazin
5,6-Cyclopenteno-2-ethyl-3-amino-1,4-benzoxazin
6,7-(Methylendioxy)-2-methyl-3-amino-1,4-benzoxazin
   [1H]-NMR (DMSO): 6,5 br NH, 6,5 2 x s 2H, 5,87 s 2H, 4,55 q 1H, 1,25 d 3H.
6,7-(Methylendioxy)-2-ethyl-3-amino-1,4-benzoxazin
6-Cyclohexyl-2-methyl-3-amino-1,4-benzoxazin
   [1H]-NMR (DMSO): 6,6 br NH, 6,7 m 3H, 4,41 q 1H, 2,85 m 1H, 1,8 bis 1,1 m 12H, 0,92 tr 3H.
7-(1-Morpholinyl)-2-methyl-3-amina-1,4-benzoxazin
2-Ethyl-3-amino-6,7,8,9-tetrahydro-napht [2,3-b]-1,4-oxazin
2-Methyl-3-amino-6,7,8,9-tetrahydro-napht [2,3-b]-1,4-oxazin
   [1H]-NMR (CDCl₃): 6,75 s 1H, 6,56 s 1H, 4,59 q 1H, 2,7 und 1,8 je m 4H, 1,50 d 3H.
3-Methyl-2-amino-6,7,8,9-tetrahydronapht [2,1-b]-1,4-oxazin
3-Ethyl-2-amino-6,7,8,9-tetrahydronapht [2,1-b]-1,4-oxazin
6-Keto-3-amino-2-methyl-6,7,8,9-tetrahydro-naphth-1,4-oxazin
7-Keto-3-amino-2-methyl-6,7,8,9-tetrahydro-naphth-1,4-oxazin
8-Keto-3-amino-2-methyl-6,7,8,9-tetrahydro-naphth-1,4-oxazin
2-Methyl-3-amino-6,7,8,9-tetrahydro-6,9-methano-naphth-1,4-oxazin
3-Amino-6-keto-6,7-cyclopenteno-2-methyl-1,4-benzoxazin
6,6,9,9-Tetramethyl-6,7,8,9-tetrahydro-3-amino-2-methyl-[2,3-b]-napht-1,4-oxazin
2-Methyl-3-amino-6,7,8,9-tetrahydro-napht [2,3-b]-1,4-thiazin
2-Ethyl-3-amino-6,7,8,9-tetrahydro-napht [2,3-b]-1,4-thiazin

3-Amino-2-methyl-6,7-pentamethylen-1,4-(2H)benzoxazin
   0.23 g (0.93 mmol) (2R)-2-Methyl-6,7-pentamethylen-1,4-dihydrobenzoxazin-3-thion werden in 20 mL 7N methanolischer Ammoniaklösung gelöst. Nach 5 h wird der Ansatz eingeengt und der Rückstand säulenchromatographisch an Kieselgel mit Essigester als Eluens gereinigt: 0.20 g Produkt (95% d. Th.).
   ¹H-NMR (CDCl₃): 1.48 (d, 3H), 1.53 - 1.71 (m, 4H), 1.81 (m, 2H), 2.71 (m, 4H), 4.57 (q, 1H), 6.62 (s, 1H), 6.78 (s, 1H).
Analog:
3-Amino-6,7-hexamethylen-2-methyl-1,4-(2H)benzoxazin
(57% d. Th.)¹H-NMR (CDCl₃): 1.35 (m, 4H), 1.47 (d, 3H), 1.63 (m, 4H), 2.66 (t, 4H), 4.58 (q, 1H), 6.60 (s, 1H), 6.77 (s, 1H).
6,7-Cyclopenteno-2-methyl-3-amino-1,4-benzothiazin
   [1H]-NMR (DMSO): 6,6 br NH, 7,01 s 1H, 6,79 s 1H, 3,45 q 1H,2,8 m 4H, 2,0 m 2H, 1,13 d 3H.
6,7-Cyclopenteno-2-ethyl-3-amino-1,4-benzothiazin
6-Trifluormethyl-2-methyl-3-amino-1,4-benzothiazin
6-Trifluormethyl-2-ethyl-3-amino-1,4-benzothiazin
6-Trifluormethyl-2-propyl-3-amino-1,4-benzothiazin
N-[(2-Methyl-1,4-benzoxazin-3-amino-7-yl]-(2-thienyl)carboximidamid
   [1H]-NMR (DMSO): 6,6 br NH, 7,72 m, 7,61 d, 7,10 dd, 6,8 d , 6,42 d, 6.32 m je 1H, 4,63 q 1H, 1,31 d 3H.
N-[(2-Ethyl-1,4-benzoxazin-3-amino-7-yl]- (2-thienyl)carboximidamid
N-[(2-Methyl-1,4-benzoxazin-3-amino-6-yl]-(2-thienyl)carboximidamid
N-[(2-Ethyl-1,4-benzoxazin-3-amino-6-yl]-(2-thienyl)carboximidamid
N-[(2-Methyl-1,4-benzoxazin-3-amino-7-yl]- 4-methyl-phenylcarboximidamid
N-[(2-Ethyl-1,4-benzoxazin-3-amino-7-yl]- 4-methyl-phenylcarboximidamid
N-[(2-Methyl-1,4-benzoxazin-3-amino-6-yl]- 4-methyl-phenylcarboximidamid
N-[(2-Ethyl-1,4-benzoxazin-3-amino-6-yl]- 4-methyl-phenylcarboximidamid
N-[(2-Methyl-1,4-benzoxazin-3-amino-7-yl]- phenylcarboximidamid
N-[(2-Ethyl-1,4-benzoxazin-3-amino-7-yl]- phenylcarboximidamid
N-[(2-Methyl-1,4-benzoxazin-3-amino-6-yl]-phenylcarboximidamid
N-[(2-Ethyl-1,4-benzoxazin-3-amino-6-yl]-phenylcarboximidamid
N-[(2-Methyl-1,4-benzoxazin-3-amino-7-yl]- 2,4-dichlor-phenylcarboximidamid
N-[(2-Ethyl-1,4-benzoxazin-3-amino-7-yl]- 2,4-dichlor-phenylcarboximidamid
N-[(2-Methyl-1,4-benzoxazin-3-amino-6-yl]- 2,4-dichlor-phenylcarboximidamid
N-[(2-Ethyl-1,4-benzoxazin-3-amino-6-yl]-2,4-dichlor-phenylcarboximidamid Hydrochlorid
   [1H]-NMR (DMSO): 9,5 breit, 7,97 m 2H, 7,74 dd 1H, 7,40 d 1H, 7,27 d 1H, 7,20 dd 1H, 5,23 q 1H, 1,8 m 2H, 1,05 tr 3H.

N-[6-(2-Ethyl-3-amino-1,4-benzoxazinyl)-N-phenylharnstoff
N-[6-(2-Ethyl-3-amino-1,4-benzoxazinyl)-N-phenylthioharnstoff
   [1H]-NMR (DMSO): 9,6 br NH, 7,5 m 2H, 7,3 m 2H, 7,1 dd 1H, 6,7 bis 6,9 m ca. 5H, 4,45 dd 1H, 1,6 m 2H, 0,93 tr 3H.
N-[6-(2-Methyl-3-amino-1,4-benzoxazinyl)-N-phenylharnstoff
N-[6-(2-Methyl-3-amino-1,4-benzoxazinyl)-N-phenylthioharnstoff
N-[6-(2-Ethyl-3-amino-1,4-benzoxazinyl)-N-cyclohexylharnstoff
N-[6-(2-Ethyl-3-amino-1,4-benzoxazinyl)-N-2-methyl-4-chlorphenylharnstoff
N-[6-(2-Ethyl-3-amino-1,4-benzoxazinyl)-N-2-chlorphenylharnstoff
N-[7-(2-Ethyl-3-amino-1,4-benzoxazinyl)-N-phenylharnstoff
N-[7-(2-Ethyl-3-amino-1,4-benzoxazinyl)-N-phenylthioharnstoff
N-[7-(2-Methyl-3-amino-1,4-benzoxazinyl)-N-phenylharnstoff
N-[7-(2-Methyl-3-amino-1,4-benzoxazinyl)-N-phenylthioharnstoff
N-[7-(2-Ethyl-3-amino-1,4-benzoxazinyl)-N-cyclohexylharnstoff
N-[7-(2-Ethyl-3-amino-1,4-benzoxazinyl)-N-2-methyl-4-chlorphenylharnstoff
N-[7-(2-Ethyl-3-amino-1,4-benzoxazinyl)-N-2-chlorphenylharnstoff

2-Methyl-3-amino-6-(2-thienyl)-1,4-benzoxazin
   [1H]-NMR (CDCl₃): 7,3 bis 7,0 m 6H, 4,55 q 1 H, 1,52 d 3H.
2-Methyl-3-amino-6-(3-thienyl)-1,4-benzoxazin
3-[2-Methyl-3-amino-1,4-benzoxazin-6-yl]-acrylamid
3-[2-Methyl-3-amino-1,4-benzoxazin-6-yl]-acrylnitril
2-Methyl-3-amino-6-(3-pyrido)-1,4-benzoxazin
2-Methyl-3-amino-6-(1-pyrrolo)-1,4-benzoxazin

6-(Propionyl)-3-amino-2-methyl-1,4-benzoxazin
   [1H]-NMR (DMSO): 6,8 br NH, 7,5 d 1H, 7,46 d1H, 6,88 d 1H, 4,77 q 1H, 2,95 tr 2H, 1,33 d 3H, 1,11 tr 3H.
6-(Styrylcarboxy)-3-amino-2-methyl-1,4-benzoxazin
6-(3-Carboxy-propionyl)-3-amino-2-methyl-1,4-benzoxazin
6-(4-Ethoxycarbonyl-butyryl)--3-amino-2-methyl-1,4-benzoxazin
6-(2-Chloracetyl)-3-amino-2-methyl-1,4-benzoxazin
2-Methyl-3-amino-6-(1-hydroxyprop-1-yl)-1,4-benzoxazin
   [1H]-NMR (CDCl₃): 7,0 d, 6,91 dd, 6,82 d je 1H, 4,60 q 1H,4,52 tr 1H, 1,8 m 2H, 1,49 d 3H, 0,9 tr 3H.
2-Methyl-3-amino-6-([1-methoxyimino]-prop-1-yl)-1,4-benzoxazin
2-Methyl-3-amino-6-(1-aminoprop-1-yl)-1,4-benzoxazin
2-Methyl-3-amino-6-(1-methyl-1-hydroxy-prop-1-yl)-1,4-benzoxazin

### Ausgangsverbindungen (2)

### 4-Hydroxy-3-nitrobenzaldehyd-ethylenacetal

25 g 4-Hydroxy-3-nitrobenzaldehyd werden in Toluol mit 1,1 Equivalenten Ethylenglycol und 370 mg para-Toluolsulfonsäure versetzt. Es wird 4 Stunden am Wasserabscheider gekocht. Die Mischung wird auf Natriumhydrogencarbonatlösung gegossen, mit Essigester extrahiert, die organische Phase mit Sole gewaschen, mit Magnesiumsulfat getrocknet und eingeengt. Es resultieren 23,6 g Rohprodukt, das für weitere Umsetzungen geeignet ist.

### 2-(4-(1,3-Dioxolan-2-yl)-2-nitrophenoxy) propionsäureethylester

23 g 4-Hydroxy-3-nitrobenzaldehyd-ethylenacetal löst man in 150 ml DMF und gibt 20,6 g Kaliumcarbonat sowie 1,1 Equivalente D,L-2-Brompropionsäureethylester in 20 ml THF bei 5 °C dazu. Es wird 14 Stunden gerührt. Die Mischung wird auf Wasser gegossen, mit Essigester extrahiert, die organische Phase mit Magnesiumsulfat getrocknet und eingeengt. Es resultieren 42 g Rohprodukt, das umkristallisiert wird. Die Ausbeute ist quantitativ.
Analog stellt man her:
2-(4-(1,3-Dioxolan-2-yl)-2-nitrophenoxy) buttersäureethylester
2-(4-(1,3-Dioxolan-2-yl)-2-nitrophenoxy) pentansäureethylester

### 6-Formyl-2-methyl-2H-1,4-benzoxazin-3-on

Zu 34 g 2-(4-(1,3-Dioxolan-2-yl)-2-nitrophenoxy) propionsäureethylester in 360 ml Eisessig mit 80 ml Wasser wird unter Eisbadkühlung portionsweise 28,8 g gepulvertes Eisen gegeben. Die Mischung wird warm. Nach 2 Stunden wird auf 1 I Wasser gegossen, extrahiert mit Ethylacetat und die organische Phase mit Sole gewaschen. Man trocknet mit Magnesiumsulfat und engt ein. Es resultieren 20,3 g bräunlicher Feststoff.
[1H]-NMR (CDCl3): 9,9 1H Aldehyd, 9,36 br 1H, 7,57 dd 1H, 7,42 d 1H, 7,11 d 1H, 4,80 q 1H, 1,64 d 3H.
Nach dem gleichen Verfahren werden hergestellt:
6-Formyl-2-ethyl-1,4-benzoxazin-3-on
6-Formyl-2-propyl-1,4-benzoxazin-3-on

### 2-Methyl-3-oxo-6-(2-nitro-ethenyl)-3,4-dihydro-2H-1,4-benzoxazin

10 g (52,31 mmol) [ 2-Methyl-3-oxo-3,4-dihydro-2H-1,4-benzoxazin-6-yl]-carbaldehyd werden in 37 ml Eisessig mit 10 ml Nitromethan und 3,71 g Ammoniumacetat zwei Stunden am Rückfluß gekocht. Das Reaktionsgemisch wird auf Eiswasser gegeben und das ausgefallene Produkt abgesaugt, mit Wasser neutralgewaschen und nach dem Trocknen ohne weitere Reinigung in die nächste Stufe eingesetzt (Ausbeute 93%).

### 2-Methyl-3-oxo-6-(2-amino-ethyl)-3,4-dihydro-2H-1,4-benzoxazin

2 g (8,5 mmol) 2-Methyl-3-oxo-6-(2-nitro-ethenyl)-3,4-dihydro-2H-1,4-benzoxazin werden mit 80 ml Essigsäure und 1,6 ml konzentrierter Schwefelsäure versetzt und nach Zugabe von 200 mg Platindioxid im Autoklaven reduziert. Der Katalysator wird anschließend abgesaugt, und der Ansatz bis zur Trockene einrotiert. Der verbleibende Rückstand wird an Kieselgel (Laufmittel Isopropanol/ Ammoniak) chromatographiert. Es werden 923,5 mg (52,4%) der gewünschten Verbindung erhalten.

### 6-((Thien-2-yl)-methylaminomethyl)-2-methyl-1,4-benzoxazin-3-on

In 4 ml einer Mischung von Methanol und THF werden 193 mg 6-Formyl-2-methyl-1,4-benzoxazin-3-on gelöst und mit 113 mg 2-(Aminomethyl)-thiophen versetzt. Man rührt 30 Minuten bei RT und gibt dann 0,6 Equivalente Kaliumborhydrid hinzu. Nach drei Stunden bei RT wird auf Wasser gegossen, extrahiert mit Ethylacetat und die organische Phase mit Sole gewaschen. Man trocknet mit Magnesiumsulfat und engt ein. Man erhält 248 mg (86%) Rohprodukt, das mit einer Schutzgruppe versehen wird. [1H]-NMR (CDCl3): 9,4 br 1H, 6,8 bis 7,3 6H, 4,64 q 1H, 4,0 s 2H, 3,73 s 2H, 1,59 d 3H.
Auf die gleiche Weise werden hergestellt:
6-((Thien-3-yl)-methylaminomethyl)-2-methyl-1,4-benzoxazin-3-on
6-(Benzylaminomethyl)-2-methyl-1,4-benzoxazin-3-on
6-((4-Methoxybenzyl)aminomethyl)-2-methyl-1,4-benzoxazin-3-on
6-((3-Chlorbenzyl)aminomethyl)-2-methyl-1,4-benzoxazin-3-on
6-((2-Chlor-6-fluorbenzyl)aminomethyl)-2-methyl-1,4-benzoxazin-3-on
6-((4-Chlorbenzyl)aminomethyl)-2-methyl-1,4-benzoxazin-3-on
6-((3,4-Dichlorbenzyl)aminomethyl)-2-methyl-1,4-benzoxazin-3-on
6-((2-Chlorbenzyl)aminomethyl)-2-methyl-1,4-benzoxazin-3-on
6-((2,4-Dichlorbenzyl)aminomethyl)-2-methyl-1,4-benzoxazin-3-on
6-((2,3-Dimethylbenzyl)aminomethyl)-2-methyl-1,4-benzoxazin-3-on
6-((3-Fluorbenzyl)aminomethyl)-2-methyl-1,4-benzoxazin-3-on
6-((Indan-1-yl)aminomethyl)-2-methyl-1,4-benzoxazin-3-on
6-((Indan-2-yl)aminomethyl)-2-methyl-1,4-benzoxazin-3-on
6-((Cyclohexylmethyl)aminomethyl)-2-methyl-1,4-benzoxazin-3-on
6-((1,2,3,4-Tetrahydronaphth-1-yl)aminomethyl)-2-methyl-1,4-benzoxazin-3-on
6-((Diphenylmethyl)aminomethyl)-2-methyl-1,4-benzoxazin-3-on
6-((3-Methoxybenzyl)aminomethyl)-2-methyl-1,4-benzoxazin-3-on
6-((3-Nitrobenzyl)aminomethyl)-2-methyl-1,4-benzoxazin-3-on
6-((4-Nitrobenzyl)aminomethyl)-2-methyl-1,4-benzoxazin-3-on
6-((4-Sulfamoylbenzyl)aminomethyl)-2-methyl-1,4-benzoxazin-3-on
6-((4-Methylsulfonylbenzyl)aminomethyl)-2-methyl-1,4-benzoxazin-3-on
6-((4-Fluorbenzyl)aminomethyl)-2-methyl-1,4-benzoxazin-3-on
6-((4-Dimethylaminobenzyl)aminomethyl)-2-methyl-1,4-benzoxazin-3-on
6-((3,4-Methylendioxybenzyl)aminomethyl)-2-methyl-1,4-benzoxazin-3-on
6-((2-Fluorbenzyl)aminomethyl)-2-methyl-1,4-benzoxazin-3-on
6-((4-Methylbenzyl)aminomethyl)-2-methyl-1,4-benzoxazin-3-on
6-((4-Pyridyl)-methylaminomethyl)-2-methyl-1,4-benzoxazin-3-on
6-((3-Pyridyl)-methylaminomethyl)-2-methyl-1,4-benzoxazin-3-on
6-((2-Furyl)-methylaminomethyl)-2-methyl-1,4-benzoxazin-3-on
6-((Naphth-1-yl)-methylaminomethyl)-2-methyl-1,4-benzoxazin-3-on
6-((3-Trifluormethylbenzyl)aminomethyl)-2-methyl-1,4-benzoxazin-3-on
6-((Thien-2-yl)-methylaminomethyl)-2-ethyl-1,4-benzoxazin-3-on
6-(Benzylaminomethyl)-2-ethyl-1,4-benzoxazin-3-on
6-((Thien-2-yl)-methylaminomethyl)-2-propyl-1,4-benzoxazin-3-on
6-(Benzylaminomethyl)-2-propyl-1,4-benzoxazin-3-on

### Entsprechend werden erhalten:

Aus 6-(Propionyl)-2-methyl-1,4-benzoxazin-3-(4H)-on:
6-(1-(Benzylamino)-prop-1-yl)-2-methyl-1,4-benzoxazin-3-on
6-(1-((Thien-2-yl)-methylamino)-prop-1-yl)-2-methyl-1,4-benzoxazin-3-on
Aus 6-(2-Ketoprop-1-yl)-2-methyl-1,4-benzoxazin-3-(4H)-on:
6-(2-(Benzylamino)-prop-1-yl)-2-methyl-1,4-benzoxazin-3-on
Man erhält aus 6-Oxo-6,7-cyclopenteno-2-methyl-1,4-benzoxazin-3-on (Deutsches Aktenzeichen 197 40 386.7)
6-(Benzylamino)-6,7-cyclopenteno-2-methyl-1,4-benzoxazin-3-on
6-(3-Chlorbenzylamino)-6,7-cyclopenteno-2-methyl-1,4-benzoxazin-3-on
und erhält analog aus dem Keton 6-Oxo-6,7-cyclohexeno-2-methyl-1,4-benzoxazin-3-on her:
6-(Benzylamino)-6,7-cyclohexeno-2-methyl-1,4-benzoxazin-3-on
6-(3-Chlorbenzylamino)-6,7-cyclohexeno-2-methyl-1,4-benzoxazin-3-on
analog entsteht aus dem Keton 7-Oxo-6,7-cyclohexeno-2-methyl-1,4-benzoxazin-3-on, welches wie 6-Oxo-6,7-cyclohexeno-2-methyl-1,4-benzoxazin-3-on aus 6-Methoxy-2-tetralon hergestellt wurde, 7-(3-Chlorbenzylamino)-6,7-cyclohexeno-2-methyl-1,4-benzoxazin-3-on

### 2-Methyl-3-oxo-6-[2-(3-chlorbenzylamino)-ethyl]-3,4-dihydro-2H-1,4-benzoxazin

915 mg (4,437 mmol) 2-Methyl-3-oxo-6-(2-amino-ethyl)-3,4-dihydro-2H-1,4-benzoxazin werden in 16 ml eines Metanol / Tetrahydrofurangemisches (4:1) gelöst. Nach Zugabe von 623,7 mg (4,437 mmol) 3- Chlorbenzaldehyd wird der Ansatz zwei Stunden bei Raumtemperatur gerührt. Anschließend werden portionsweise 91,1 mg (2,408 mmol) Natriumborhydrid zugegeben und weitere zwei Stunden bei Raumtemperatur gerührt. Der Ansatz wird auf 50 ml Wasser gegeben , und nach dreimaliger Extraktion mit Essigsäureethylester werden die vereinigten organischen Extrakte mit Sole gewaschen, getrocknet und das Lösungsmittel abrotiert. Der verbleibende Rückstand wird an Kieselgel chromatographiert (Laufmittel Essigsäureethylester/ Methanol). Ausbeute 584,6 mg (39,8%).

### 6-((Thien-2-yl)-methyl-(tert.-butyloxycarbonyl)aminomethyl)-2-methyl-1,4-benzoxazin-3-on

Das Produkt erhält man durch Umsetzung von 6-((Thien-2-yl)-methylaminomethyl)-2-methyl-1,4-benzoxazin-3-on (245 mg) in 5 ml Dichlormethan unter Zugabe von 0,177 ml Triethylamin und 223 mg Di-*tertiär*-butyldicarbonat. Nach 12 Stunden bei RT ist die Reaktion beendet. Man verdünnt mit Dichlormethan, wäscht mit Natriumhydrogencarbonat und danach mit Sole, trocknet die org. Phase und engt ein. Nach Säulenchromatographie mit Hexan / Ethylacetat resultieren 211 mg Produkt. [1H]-NMR (CDCl3): 8 br 1H, 7,24dd1H, 6,95 m 2H, 6,88 br 2H, 6,7 br 1H, 4,62 q 1H, 4,44 s 2H, 4,32 s 2H, 1,55 m 9H plus 3H.
Auf die gleiche Weise werden hergestellt:
6-((Thien-3-yl)-methyl-(*tert*.-butyloxycarbonyl)aminomethyl)-2-methyl-1,4-benzoxazin-3-on
6-(Benzyl-(*tert*.-butyloxycarbonyl)aminomethyl)-2-methyl-1,4-benzoxazin-3-on
6-((4-Methoxybenzyl)(tert.-butyloxycarbonyl) aminomethyl)-2-methyl-1,4-benzoxazin-3-on
6-((3-Chlorbenzyl)(tert.-butyloxycarbonyl) aminomethyl)-2-methyl-1,4-benzoxazin-3-on
6-((2-Chlor-6-fluorbenzyl)(tert.-butyloxycarbonyl) aminomethyl)-2-methyl-1,4-benzoxazin-3-on
6-((4-Chlorbenzyl)(tert.-butyloxycarbonyl) aminomethyl)-2-methyl-1,4-benzoxazin-3-on
6-((3,4-Dichlorbenzyl)(tert.-butyloxycarbonyl) aminomethyl)-2-methyl-1,4-benzoxazin-3-on
6-((2-Chlorbenzyl)(tert.-butyloxycarbonyl) aminomethyl)-2-methyl-1,4-benzoxazin-3-on
6-((2,4-Dichlorbenzyl)(tert.-butyloxycarbonyl) aminomethyl)-2-methyl-1,4-benzoxazin-3-on
6-((2,3-Dimethylbenzyl)(tert.-butyloxycarbonyl) aminomethyl)-2-methyl-1,4-benzoxazin-3-on
6-((3-Fluorbenzyl)(tert.-butyloxycarbonyl) aminomethyl)-2-methyl-1,4-benzoxazin-3-on
6-((Indan-1-yl)(tert.-butyloxycarbonyl) aminomethyl)-2-methyl-1,4-benzoxazin-3-on
6-((Indan-2-yl)(tert.-butyloxycarbonyl) aminomethyl)-2-methyl-1,4-benzoxazin-3-on
6-((Cyclohexylmethyl)(tert.-butyloxycarbonyl)aminomethyl)-2-methyl-1,4-benzoxazin-3-on
6-((1,2,3,4-Tetrahydronaphth-1-yl)(tert.-butyloxycarbonyl) aminomethyl)-2-methyl-1,4-benzoxazin-3-on
6-((Diphenylmethyl)(tert.-butyloxycarbonyl) aminomethyl)-2-methyl-1,4-benzoxazin-3-on
6-((3-Methoxybenzyl)(tert.-butyloxycarbonyl) aminomethyl)-2-methyl-1,4-benzoxazin-3-on
6-((3-Nitrobenzyl)(tert.-butyloxycarbonyl) aminomethyl)-2-methyl-1,4-benzoxazin-3-on
6-((4-Nitrobenzyl)(tert.-butyloxycarbonyl) aminomethyl)-2-methyl-1,4-benzoxazin-3-on
6-((4-Sulfamoylbenzyl)(tert.-butyloxycarbonyl) aminomethyl)-2-methyl-1,4-benzoxazin-3-on
6-((4-Methylsulfonylbenzyl)(tert.-butyloxycarbonyl) aminomethyl)-2-methyl-1,4-benzoxazin-3-on
6-((4-Fluorbenzyl)(tert.-butyloxycarbonyl) aminomethyl)-2-methyl-1,4-benzoxazin-3-on
6-((4-Dimethylaminobenzyl)(tert.-butyloxycarbonyl) aminomethyl)-2-methyl-1,4-benzoxazin-3-on
6-((3,4-Methylendioxybenzyl)(tert.-butyloxycarbonyl) aminomethyl)-2-methyl-1,4-benzoxazin-3-on
6-((2-Fluorbenzyl)(tert.-butyloxycarbonyl) aminomethyl)-2-methyl-1,4-benzoxazin-3-on
6-((4-Methylbenzyl)(tert.-butyloxycarbonyl) aminomethyl)-2-methyl-1,4-benzoxazin-3-on
6-((4-Pyridyl)-methyl(tert.-butyloxycarbonyl) aminomethyl)-2-methyl-1,4-benzoxazin-3-on
6-((3-Pyridyl)-methyl(tert.-butyloxycarbonyl) aminomethyl)-2-methyl-1,4-benzoxazin-3-on
6-((2-Furyl)-methyl(tert.-butyloxycarbonyl) aminomethyl)-2-methyl-1,4-benzoxazin-3-on
6-((Naphth-1-yl)-methyl(tert.-butyloxycarbonyl) aminomethyl)-2-methyl-1,4-benzoxazin-3-on
6-((3-Trifluormethylbenzyl)(tert.-butyloxycarbonyl) aminomethyl)-2-methyl-1,4-benzoxazin-3-on

6-(Benzyl(tert.-butyloxycarbonyl) amino)-6,7-cyclopenteno-2-methyl-1,4-benzoxazin-3-on
6-(Benzyl(tert.-butyloxycarbonyl) amino)-6,7-cyclohexeno-2-methyl-1,4-benzoxazin-3-on

6-((Thien-2-yl)-methyl-(tert.-butyloxycarbonyl)amino)-6,7-cyclopenteno-2-methyl-1,4-benzoxazin-3-on
6-((Thien-2-yl)-methyl-(tert.-butyloxycarbonyl)amino)-6,7-cyclohexeno-2-methyl-1,4-benzoxazin-3-on
6-(3-Chlorbenzyl(tert.-butyloxycarbonyl) amino)-6,7-cyclopenteno-2-methyl-1,4-benzoxazin-3-on
6-(3-Chlorbenzyl(tert.-butyloxycarbonyl) amino)-6,7-cyclohexeno-2-methyl-1,4-benzoxazin-3-on
7-(3-Chlorbenzyl(tert.-butyloxycarbonyl) amino)-6,7-cyclohexeno-2-methyl-1,4-benzoxazin-3-on
6-(2-(Benzyl(tert.-butyloxycarbonyl) amino)-prop-1-yl)-2-methyl-1,4-benzoxazin-3-on
6-(1-(Benzyl(tert.-butyloxycarbonyl) amino)-prop-1-yl)-2-methyl-1,4-benzoxazin-3-on
6-(1-((Thien-2-yl)-methyl(tert.-butyloxycarbonyl) amino)-prop-1-yl)-2-methyl-1,4-benzoxazin-3-on

6-((Thien-2-yl)-methyl-(*tert*.-butyloxycarbonyl)aminomethyl)-2-ethyl-1,4-benzoxazin-3-on
6-((Thien-2-yl)-methyl-(*tert*.-butyloxycarbonyl)aminomethyl)-2-propyl-1,4-benzoxazin-3-on

### 2-Methyl-3-oxo-6-[2-(3-chlorbenzyl-tert. butyloxycarbonylamino)-ethyl]-3,4-dihydro-2H-1,4-benzoxazin

550 mg (1,623 mmol) 2-Methyl-3-oxo-6-[2-(3-chlorbenzylamino)-ethyl]-3,4-dihydro-2H-1,4-benzoxazin werden in 10 ml absolutem Dichlormethan mit 425,2 mg Ditert.-Butyldicarbonat und 243,3 mg Triethylamin versetzt. Nach dreistündigem Rühren bei Raumtemperatur wird der Ansatz mit Dichlormethan verdünnt und anschließend mit gesättigter Natriumhydrogencarbonatlösung und mit Sole gewaschen. Nach dem Trocknen der organischen Phase wird das Lösungsmittel abrotiert und der verbleibende Rückstand an Kieselgel chromatographiert (Laufmittel Essigsäureethylester/ Hexan). Die Ausbeute beträgt 742,6 mg (> 100%).

### 6-((Thien-2-yl)-methyl-(tert.-butyloxycarbonyl) aminomethyl)-2-methyl-1,4-benzoxazin-3-thion

Zu 200 mg 6-((Thien-2-yl)-methyl(*tert*.-butyloxycarbonyl) aminomethyl)-2-methyl-1,4-benzoxazin-3-on in 15 ml Dimethoxyethan gibt man bei RT 243 mg Lawessons Reagenz und rührt 4 Stunden nach. Nach einengen und nach Säulenchromatographie mit Hexan / Ethylacetat resultieren 151 mg Produkt.
[1H]-NMR (CDCl3): 9,4 br 1H, 6,7 bis 7,3 6H, 5,01 q 1H, 4,5 s br 2H, 4,35 s br 2H, 1,5 s 9H, 1,6 d 3H. MS: 404 m/e (M+).
Auf die gleiche Weise werden hergestellt:
6-((Thien-3-yl)-methyl(*tert*.-butyloxycarbonyl) aminomethyl)-2-methyl-1,4-benzoxazin-3-thion
6-(Benzyl(*tert*.-butyloxycarbonyl) aminomethyl)-2-methyl-1,4-benzoxazin-3-thion
6-((4-Methoxybenzyl)(tert.-butyloxycarbonyl) aminomethyl)-2-methyl-1,4-benzoxazin-3-thion
6-((3-Chlorbenzyl)(tert.-butyloxycarbonyl) aminomethyl)-2-methyl-1,4-benzoxazin-3-thion
6-((2-Chlor-6-fluorbenzyl)(tert.-butyloxycarbonyl) aminomethyl)-2-methyl-1,4-benzoxazin-3-thion
6-((4-Chlorbenzyl)(tert.-butyloxycarbonyl) aminomethyl)-2-methyl-1,4-benzoxazin-3-thion
6-((3,4-Dichlorbenzyl)(tert.-butyloxycarbonyl) aminomethyl)-2-methyl-1,4-benzoxazin-3-thion
6-((2-Chlorbenzyl)(tert.-butyloxycarbonyl) aminomethyl)-2-methyl-1,4-benzoxazin-3-thion
6-((2,4-Dichlorbenzyl)(tert.-butyloxycarbonyl) aminomethyl)-2-methyl-1,4-benzoxazin-3-thion
6-((2,3-Dimethylbenzyl)(tert.-butyloxycarbonyl) aminomethyl)-2-methyl-1,4-benzoxazin-3-thion
6-((3-Fluorbenzyl)(tert.-butyloxycarbonyl) aminomethyl)-2-methyl-1,4-benzoxazin-3-thion
6-((Indan-1-yl)(tert.-butyloxycarbonyl) aminomethyl)-2-methyl-1,4-benzoxazin-3-thion
6-((Indan-2-yl)(tert.-butyloxycarbonyl) aminomethyl)-2-methyl-1,4-benzoxazin-3-thion
6-((Cyclohexylmethyl)(tert.-butyloxycarbonyl)aminomethyl)-2-methyl-1,4-benzoxazin-3-thion
6-((1,2,3,4-Tetrahydronaphth-1-yl)(tert.-butyloxycarbonyl) aminomethyl)-2-methyl-1,4-benzoxazin-3-thion
6-((Diphenylmethyl)(tert.-butyloxycarbonyl) aminomethyl)-2-methyl-1,4-benzoxazin-3-thion
6-((3-Methoxybenzyl)(tert.-butyloxycarbonyl) aminomethyl)-2-methyl-1,4-benzoxazin-3-thion
6-((3-Nitrobenzyl)(tert.-butyloxycarbonyl) aminomethyl)-2-methyl-1,4-benzoxazin-3-thion
6-((4-Nitrobenzyl)(tert.-butyloxycarbonyl) aminomethyl)-2-methyl-1,4-benzoxazin-3-thion
6-((4-Sulfamoylbenzyl)(tert.-butyloxycarbonyl) aminomethyl)-2-methyl-1,4-benzoxazin-3-thion
6-((4-Methylsulfonylbenzyl)(tert.-butyloxycarbonyl) aminomethyl)-2-methyl-1,4-benzoxazin-3-thion
6-((4-Fluorbenzyl)(tert.-butyloxycarbonyl) aminomethyl)-2-methyl-1,4-benzoxazin-3-thion
6-((4-Dimethylaminobenzyl)(tert.-butyloxycarbonyl) aminomethyl)-2-methyl-1,4-benzoxazin-3-thion
6-((3,4-Methylendioxybenzyl)(tert.-butyloxycarbonyl) aminomethyl)-2-methyl-1,4-benzoxazin-3-thion
6-((2-Fluorbenzyl)(tert.-butyloxycarbonyl) aminomethyl)-2-methyl-1,4-benzoxazin-3-thion
6-((4-Methylbenzyl)(tert.-butyloxycarbonyl) aminomethyl)-2-methyl-1,4-benzoxazin-3-thion
6-((4-Pyridyl)-methyl(tert.-butyloxycarbonyl) aminomethyl)-2-methyl-1,4-benzoxazin-3-thion
6-((3-Pyridyl)-methyl(tert.-butyloxycarbonyl) aminomethyl)-2-methyl-1,4-benzoxazin-3-thion
6-((2-Furyl)-methyl(tert.-butyloxycarbonyl) aminomethyl)-2-methyl-1,4-benzoxazin-3-thion
6-((Naphth-1-yl)-methyl(tert.-butyloxycarbonyl) aminomethyl)-2-methyl-1,4-benzoxazin-3-thion
6-((3-Trifluormethylbenzyl)(tert.-butyloxycarbonyl) aminomethyl)-2-methyl-1,4-benzoxazin-3-thion
6-(Benzyl(tert.-butyloxycarbonyl)amino)-6,7-cyclopenteno-2-methyl-1,4-benzoxazin-3-thion
6-(Benzyl(tert.-butyloxycarbonyl)amino)-6,7-cyclohexeno-2-methyl-1,4-benzoxazin-3-thion
6-((Thien-2-yl)-methyl-(tert.-butyloxycarbonyl)amino)-6,7-cyclopenteno-2-methyl-1,4-benzoxazin-3-thion
6-((Thien-2-yl)-methyl-(tert.-butyloxycarbonyl)amino)-6,7-cyclohexeno-2-methyl-1,4-benzoxazin-3-thion
6-(3-Chlorbenzyl(tert.-butyloxycarbonyl) amino)-6,7-cyclopenteno-2-methyl-1,4-benzoxazin-3-thion
6-(3-Chlorbenzyl(tert.-butyloxycarbonyl) amino)-6,7-cyclohexeno-2-methyl-1,4-benzoxazin-3-thion
7-(3-Chlorbenzyl(tert.-butyloxycarbonyl) amino)-6,7-cyclohexeno-2-methyl-1,4-benzoxazin-3-thion
6-(2-(Benzyl(tert.-butyloxycarbonyl) amino)-prop-1-yl)-2-methyl-1,4-benzoxazin-3-thion
6-(1-(Benzyl(tert.-butyloxycarbonyl) amino)-prop-1-yl)-2-methyl-1,4-benzoxazin-3-thion
6-(1-((Thien-2-yl)-methyl(tert.-butyloxycarbonyl) amino)-prop-1-yl)-2-methyl-1,4-benzoxazin-3-thion
6-((Thien-2-yl)-methyl-(*tert*.-butyloxycarbonyl)aminomethyl)-2-ethyl-1,4-benzoxazin-3-thion
6-((Thien-2-yl)-methyl-(*tert*.-butyloxycarbonyl)aminomethyl)-2-propyl-1,4-benzoxazin-3-thion

### 2-Methyl-6-[2-(3-chlorbenzyl-tert. butyloxycarbonylamino)-ethyl]-3,4-dihydro-2H-1,4-benzoxazin-3-thion

730,6 mg (1,696 mmol) 2-Methyl-3-oxo-6-[2-(3-chlorbenzyl-tert. butyloxycarbonylamino)-ethyl]-3,4-dihydro-2H-1,4-benzoxazin werden in 8 ml Pyridin mit 201,1 mg (0,607 mmol) Phosphorpentasulfid versetzt und anschließend vier Stunden unter Rückfluß gekocht. Das Lösungsmittel wird abrotiert und der verbleibende Rückstand an Kieselgel chromatographiert (Laufmittel Essigsäureethylester/ Hexan). Die Ausbeute beträgt 439,3 mg (58%).

### Beispiel 2

### 6-((Thien-2-yl)-methyl(tert.-butyloxycarbonyl) amino-methyl)-3-amino-2-methyl-1,4-benzoxazin

Man rührt 148 mg 6-((Thien-2-yl)-methyl(*tert*.-butyloxycarbonyl) amino-methyl)-2-methyl-1,4-benzoxazin-3-thion in 70 ml ges. Ammoniaklösung in Methanol (kommerziell erhältlich). Nach 1 Tag bei Raumtemperatur erhält man das Rohprodukt nach einengen. Säulenchromatografie mit Essigester reinigt das Produkt. Es resultieren 88% Ausbeute.
[1H]-NMR (DMSO): 7,42 dd 1H, 6,99 d 2H, 6,7 m 3H, 4,64 q 1H, 4,45 s br 2H, 4,23 s br 2H, 1,49 s 9H, 1,28 d 3H. MS: 387 m/e (M+).
Auf die gleiche Weise werden hergestellt:

### 6-((Thien-3-yl)-methyl(tert.-butyloxycarbonyl) aminomethyl)-3-amino-2-methyl-1,4-benzoxazin

### 6-(Benzyl-(tert.-butyloxycarbonyl) aminomethyl)-3-amino-2-methyl-1,4-benzoxazin

[1H]-NMR (MeOH): 7,07 bis 7,23 m 5H, 6,75 1H, 6,64 2H, 4,61 q 1H, 4,3 s br 2H, 4,2 s br 2H, 1,41 s 9H, 1,26 d 3H.

### 6-((4-Methoxybenzyl)(tert.-butyloxycarbonyl) aminomethyl)-3-amino-2-methyl-1,4-benzoxazin

[1H]-NMR (CDCl3): 7,2 m 2H, 6,9 bis 6,75 m 4H, 4,62 q 1H, 4,2 bis 4,4 m br 4H, 3,80 s 3H, 1,50 s 9H, 1,49 d 3H. MS: 411 m/e (M+).
6-((3-Chlorbenzyl)(tert.-butyloxycarbonyl) aminomethyl)-3-amino-2-methyl-1,4-benzoxazin
6-((2-Chlor-6-fluorbenzyl)(tert.-butyloxycarbonyl) aminomethyl)-3-amino-2-methyl-1,4-benzoxazin
6-((4-Chlorbenzyl)(tert.-butyloxycarbonyl) aminomethyl)-3-amino-2-methyl-1,4-benzoxazin
6-((4-Methylbenzyl)(tert.-butyloxycarbonyl) aminomethyl)-3-amino-2-methyl-1,4-benzoxazin
6-((4-Pyridyl)-methyl(tert.-butyloxycarbonyl) aminomethyl)-3-amino-2-methyl-1,4-benzoxazin
6-((3-Pyridyl)-methyl(tert.-butyloxycarbonyl) aminomethyl)-3-amino-2-methyl-1,4-benzoxazin
6-((2-Furyl)-methyl(tert.-butyloxycarbonyl) aminomethyl)-3-amino-2-methyl-1,4-benzoxazin
6-((Naphth-1-yl)-methyl(tert.-butyloxycarbonyl) aminomethyl)-3-amino-2-methyl-1,4-benzoxazin
6-((3-Trifluormethylbenzyl)(tert.-butyloxycarbonyl) aminomethyl)-3-amino-2-methyl-1,4-benzoxazin
6-((3,4-Dichlorbenzyl)(tert.-butyloxycarbonyl) aminomethyl)-3-amino-2-methyl-1,4-benzoxazin
6-((2-Chlorbenzyl)(tert.-butyloxycarbonyl) aminomethyl)-3-amino-2-methyl-1,4-benzoxazin
6-((2,4-Dichlorbenzyl)(tert.-butyloxycarbonyl) aminomethyl)-3-amino-2-methyl-1,4-benzoxazin
6-((2,3-Dimethylbenzyl)(tert.-butyloxycarbonyl) aminomethyl)-3-amino-2-methyl-1,4-benzoxazin
6-((3-Fluorbenzyl)(tert.-butyloxycarbonyl) aminomethyl)-3-amino-2-methyl-1,4-benzoxazin
6-((Indan-1-yl)(tert.-butyloxycarbonyl) aminomethyl)-3-amino-2-methyl-1,4-benzoxazin
6-((Indan-2-yl)(tert.-butyloxycarbonyl) aminomethyl)-3-amino-2-methyl-1,4-benzoxazin
6-((Cyclohexylmethyl)(tert.-butyloxycarbonyl)aminomethyl)-3-amino-2-methyl-1,4-benzoxazin
6-((1,2,3,4-Tetrahydronaphth-1-yl)(tert.-butyloxycarbonyl) aminomethyl)-3-amino-2-methyl-1,4-benzoxazin6-((Diphenylmethyl)(tert.-butyloxycarbonyl) aminomethyl)-3-amino-2-methyl-1,4-benzoxazin
6-((3-Methoxybenzyl)(tert.-butyloxycarbonyl) aminomethyl)-3-amino-2-methyl-1,4-benzoxazin
6-((3-Nitrobenzyl)(tert.-butyloxycarbonyl) aminomethyl)-3-amino-2-methyl-1,4-benzoxazin
6-((4-Nitrobenzyl)(tert.-butyloxycarbonyl) aminomethyl)-3-amino-2-methyl-1,4-benzoxazin
6-((4-Sulfamoylbenzyl)(tert.-butyloxycarbonyl) aminomethyl)-3-amino-2-methyl-1,4-benzoxazin
6-((4-Methylsulfonylbenzyl)(tert.-butyloxycarbonyl) aminomethyl)-3-amino-2-methyl-1,4
benzoxazin
6-((4-Fluorbenzyl)(tert.-butyloxycarbonyl) aminomethyl)-3-amino-2-methyl-1,4-benzoxazin
6-((4-Dimethylaminobenzyl)(tert.-butyloxycarbonyl) aminomethyl)-3-amino-2-methyl-1,4-benzoxazin
6-((3,4-Methylendioxybenzyl)(tert.-butyloxycarbonyl) aminomethyl)-3-amino-2-methyl-1,4-benzoxazin
6-((2-Fluorbenzyl)(tert.-butyloxycarbonyl) aminomethyl)-3-amino-2-methyl-1,4-benzoxazin
Aus 6-((4-Nitrobenzyl)(tert.-butyloxycarbonyl) aminomethyl)-2-methyl-1,4-benzoxazin-3-thion nach Reduktion
6-((4-Aminobenzyl)(tert.-butyloxycarbonyl) aminomethyl)-3-amino-2-methyl-1,4-benzoxazin
6-(Benzyl(tert.-butyloxycarbonyl) amino)-6,7-cyclopenteno-3-amino-2-methyl-1,4-benzoxazin
   [1H]-NMR (DMSO): 7,1 bis 7,33 m 4H, 6,4 bis 6,7 m 3H, 4,6 q 1H, 4,45 s br 2H, 4,0 s br 1H, 2,7 m 2H, 2,3 m 1H, 1,8 m 1H, 1,33 s 9H, 1,24 d 3H.
6-(Benzyl(tert.-butyloxycarbonyl) amino)-6,7-cyclohexeno-3-amino-2-methyl-1,4-benzoxazin
   [1H]-NMR (MeOH): 7,05 bis 7,21 m 5H, 6,6 s 1H, 6,42 d 1H, 4,55 q 1H, 4,5 s br 4H, 2,5 m 2H, 1,8 m 2H, 1,6 m 2H 1,27 m 12H.
6-((Thien-2-yl)-methyl-(tert.-butyloxycarbonyl)amino)-6,7-cyclopenteno-3-amino-2-methyl-1,4-benzoxazin
6-((Thien-2-yl)-methyl-(tert.-butyloxycarbonyl)amino)-6,7-cyclohexeno-3-amino-2-methyl-1,4-benzoxazin
6-(3-Chlorbenzyl-(tert.-butyloxycarbonyl)amino)-6,7-cyclopenteno-3-amino-2-methyl-1,4-benzoxazin
6-(3-Chlorbenzyl-(tert.-butyloxycarbonyl) amino)-6,7-cyclohexeno-3-amino-2-methyl-1,4-benzoxazin
7-(3-Chlorbenzyl-(tert.-butyloxycarbonyl)amino)-6,7-cyclohexeno-3-amino-2-methyl-1,4-benzoxazin
6-(2-(Benzyl-(tert.-butyloxycarbonyl)amino)-prop-1-yl)-3-amino-2-methyl-1,4-benzoxazin
6-(1-(Benzyl(tert.-butyloxycarbonyl) amino)-prop-1-yl)-3-amino-2-methyl-1,4-benzoxazin
   [1H]-NMR (DMSO): 6,6 bis 7,2 m 10H, 5,0 m 1H, 4,63q 1H, 4,05 bis 4,3 m br 2H, 1,8 dq br 2H, 1,39 s 9H, 1,25 d 3H, 0,81 tr 3H.
6-(1-((Thien-2-yl)-methyl(tert.-butyloxycarbonyl) amino)-prop-1-yl)-3-amino-2-methyl-1,4-benzoxazin
6-((Thien-2-yl)-methyl-(*tert*.-butyloxycarbonyl)aminomethyl)-3-amino-2-ethyl-1,4-benzoxazin
6-((Thien-2-yl)-methyl-(*tert*.-butyloxycarbonyl)aminomethyl)-3-amino-2-propyl-1,4-benzoxazin
2-Methyl-3-amino-6-[2-(3-chlorbenzyl-tert. butyloxycarbonylamino)-ethyl]-2H-1,4-benzoxazin

430 mg (0,962 mmol) 2-Methyl-6-[2-(3-chlorbenzyl-tert. butyloxycarbonylamino)-ethyl]-3,4-dihydro-2H-1,4-benzoxazin-3-thion werden mit 10 ml einer Lösung von Ammoniak in Methanol (7n) versetzt und über Nacht bei Raumtemperatur gerührt. Das Lösungsmittel wird abrotiert und der Rückstand an Kieselgel chromatographiert (Laufmittel Dichlormethan / Isopropanol). Die Ausbeute beträgt 218,6 mg (52,9%).

### Beispiel 3

### 6-((Thien-2-yl)-methylaminomethyl)-3-amino-2-methyl-1,4-benzoxazin Dihydrochlorid

28 mg von 6-((Thien-2-yl)-methyl(*tert*.-butyloxycarbonyl) amino-methyl)-3-amino-2-methyl-1,4-benzoxazin werden in 2 ml Dioxan mit 0,6 ml 4 n Salzsäure gerührt. Nach 12 Stunden wird mit etwas Ethylacetat verdünnt, die Kristalle abgesaugt, mit wenig Ethylacetat gewaschen und im Vakuum getrocknet. Man erhält 20,4 mg Produkt (79% Ausbeute).
[1H]-NMR (DMSO): 9,8 breit, 7,63 dd 1H, 7,45 d 1H, 7,37 d 1H, 7,32 dd 1H, 7, 11 m 2H, 5,37 q 1H, 4,4 br 2H, 4,13 br 2H, 1,51 d 3H.
Auf die gleiche Weise werden hergestellt:
6-((Thien-3-yl)-methylaminomethyl)-3-amino-2-methyl-1,4-benzoxazin Dihydrochlorid
6-(Benzylaminomethyl)-3-amino-2-methyl-1,4-benzoxazin Dihydrachlorid
6-((4-Methoxybenzyl)aminomethyl)-3-amino-2-methyl-1,4-benzoxazin Dihydrochlorid
   [1H]-NMR (DMSO): 9,65 breit, 7,51 m 2H, 7,49 d 1H, 7,34 dd 1H, 7, 11 d 1H, 7,0 m 2H, 5,35 q 1H, 4,1 br 4H, 3,8 s 3H, 1,51 d 3H.
6-((3-Chlorbenzyl)aminomethyl)-3-amino-2-methyl-1,4-benzoxazin Dihydrochlorid
6-((2-Chlor-6-fluorbenzyl)aminomethyl)-3-amino-2-methyl-1,4-benzoxazin Dihydrochlorid
6-((4-Chlorbenzyl)aminomethyl)-3-amino-2-methyl-1,4-benzoxazin Dihydrochlorid
6-((4-Methylbenzyl)aminomethyl)-3-amino-2-methyl-1,4-benzoxazin Dihydrochlorid
6-((4-Pyridyl)-methylaminomethyl)-3-amino-2-methyl-1,4-benzoxazin Dihydrochlorid
   [1H]-NMR (DMSO): 9,6 breit, 8,83 br 2H, 8,05 br 2H, 7,1 bis 7,55 m 3H, 5,40 q 1H, 4,39 s br 2H, 4,2 s br 2H, 1,51 d 3H.
6-((3-Pyridyl)-methylaminomethyl)-3-amino-2-methyl-1,4-benzoxazin Dihydrochlorid
6-((2-Furyl)-methylaminomethyl)-3-amino-2-methyl-1,4-benzoxazin Dihydrochlorid
6-((Naphth-1-yl)-methylaminomethyl)-3-amino-2-methyl-1,4-benzoxazin Dihydrochlorid
6-((3-Trifluormethylbenzyl)aminomethyl)-3-amino-2-methyl-1,4-benzoxazin Dihydrochlorid
6-((3,4-Dichlorbenzyl)aminomethyl)-3-amino-2-methyl-1,4-benzoxazin Dihydrochlorid
6-((2-Chlorbenzyl)aminomethyl)-3-amino-2-methyl-1,4-benzoxazin Dihydrochlorid
6-((2,4-Dichlorbenzyl)aminomethyl)-3-amino-2-methyl-1,4-benzoxazin Dihydrochlorid
6-((2,3-Dimethylbenzyl)aminomethyl)-3-amino-2-methyl-1,4-benzoxazin Dihydrochlorid
6-((3-Fluorbenzyl)aminomethyl)-3-amino-2-methyl-1,4-benzoxazin Dihydrochlorid
6-((Indan-1-yl) aminomethyl)-3-amino-2-methyl-1,4-benzoxazin Dihydrochlorid
6-((Indan-2-yl) aminomethyl)-3-amino-2-methyl-1,4-benzoxazin Dihydrochlorid
6-((Cyclohexylmethyl)aminomethyl)-3-amino-2-methyl-1,4-benzoxazin Dihydrochlorid
6-((1,2,3,4-Tetrahydronaphth-1-yl) aminomethyl)-3-amino-2-methyl-1,4-benzoxazin Dihydrochlorid
6-((Diphenylmethyl) aminomethyl)-3-amino-2-methyl-1,4-benzoxazin Dihydrochlorid
6-((3-Methoxybenzyl)aminomethyl)-3-amino-2-methyl-1,4-benzoxazin Dihydrochlorid
6-((3-Nitrobenzyl)aminomethyl)-3-amino-2-methyl-1,4-benzoxazin Dihydrochlorid
6-((4-Nitrobenzyl)aminomethyl)-3-amino-2-methyl-1,4-benzoxazin Dihydrochlorid
6-((4-Sulfamoylbenzyl)aminomethyl)-3-amino-2-methyl-1,4-benzoxazin Dihydrochlorid
6-((4-Methylsulfonylbenzyl)aminomethyl)-3-amino-2-methyl-1,4-benzoxazin Dihydrochlorid
6-((4-Fluorbenzyl) aminomethyl)-3-amino-2-methyl-1,4-benzoxazin Dihydrochlorid
6-((4-Dimethylaminobenzyl)aminomethyl)-3-amino-2-methyl-1,4-benzoxazin Trihydrochlorid
6-((3,4-Methylendioxybenzyl)aminomethyl)-3-amino-2-methyl-1,4-benzoxazin Dihydrochlorid
6-((2-Fluorbenzyl)aminomethyl)-3-amino-2-methyl-1,4-benzoxazin Dihydrochlorid
6-((4-Aminobenzyl)aminomethyl)-3-amino-2-methyl-1,4-benzoxazin Trihydrochlorid
6-(Benzylamino)-6,7-cyclopenteno-3-amino-2-methyl-1,4-benzoxazin Dihydrochlorid
   [1H]-NMR (MeOH):7,4 m 6H, 7,00 s 1H, 5,12 q 1H, 4,2 s br 2H, 4,9 1H, 3,2, 3,0, 2,65 und 2,4 je m 1H 1,47 d 3H.
6-(Benzylamino)-6,7-cyclohexeno-3-amino-2-methyl-1,4-benzoxazin Dihydrochlorid
   [1H]-NMR (DMSO): 9,5 breit, 7,67 m 2H, 7,45 m 4H, 6,92s 1H, 5,31 q 1H, 4,44 br 1H, 4,21 br 2H, 2,77 ABq 2H, 1,7 bis 2,3 m 4H, 1,49 d 3H.
6-((Thien-2-yl)-methylamino)-6,7-cyclopenteno-3-amino-2-methyl-1,4-benzoxazin Dihydrochlorid
   [1H]-NMR (DMSO): 9,6 breit, 7,67 m 2H, 7,43 dd 1H, 7,13 dd 1H, 7,97 s 1H, 5,32 q 1H, 4,75 br 1H, 4,4 br 1H, 3,2 m 1H, 2,9 m 1H, 2,32 bis 2,5 m 2H, 1,5 d 3H.
6-((Thien-2-yl)-methylamino)-6,7-cyclohexeno-3-amino-2-methyl-1,4-benzoxazin Dihydrochlorid
   [1H]-NMR (DMSO): 9,5 breit, 7,65 d 1H, 7,45 d 1H, 7,41 s 1H, 7,13 dd 1H, 6,91s 1H, 5,30 q 1H, 4,47 br 2H, 4,43 br 1H, 2,75 m 2H, 2,2 m 2H, 1,8 m 2H, 1,49 d 3H.
6-(3-Chlorbenzylamino)-6,7-cyclopenteno-3-amino-2-methyl-1,4-benzoxazin Dihydrochlorid
6-(3-Chlorbenzylamino)-6,7-cyclohexeno-3-amino-2-methyl-1,4-benzoxazin Dihydrochlorid
6-(2-(Benzylamino)-prop-1-yl)-3-amino-2-methyl-1,4-benzoxazin Dihydrochlorid
7-(3-Chlorbenzylamino)-6,7-cyclohexeno-3-amino-2-methyl-1,4-benzoxazin Dihydrochlorid
6-(1-(Benzylamino)-prop-1-yl)-3-amino-2-methyl-1,4-benzoxazin Dihydrochlorid
6-(1-((Thien-2-yl)-methylamino)-prop-1-yl)-3-amino-2-methyl-1,4-benzoxazin Dihydrochlorid
6-((Thien-2-yl)-methyl-aminomethyl)-3-amino-2-ethyl-1,4-benzoxazin Dihydrochlorid
6-((Thien-2-yl)-methyl-aminomethyl)-3-amino-2-propyl-1,4-benzoxazin Dihydrochlorid

### 2-Methyl-3-amino-6-[2-(3-chlorbenzylamino)-ethyl]-2H-1,4-benzoxazin Dihydrochlorid

203,9 mg (0,474 mmol) 2-Methyl-3-amino-6-[2-(3-chlorbenzyl-tert. butyloxycarbonylamino)-ethyl]- -2H-1,4-benzoxazin werden mit 7ml einer HCl in Dioxan Lösung (4M) versetzt und drei Stunden bei Raumtemperatur gerührt. Das ausgefallene Produkt wird abgesaugt, mit Toluol und mit Dichlormethan gewaschen. Nach dem Trocknen an der Ölpumpe werden 163,3 mg (85,5 %) des gewünschten Produkts als Dihydrochlorid erhalten.

### Beispiel 4

### a) 4-Hydroxy-3-amino-benzoesäuremethylester

25 g (126,8 mmol) 3-Nitro-4-hydroxybenzoesäuremethylester werden in einem Gemisch aus 1120 ml Ethanol und 460 ml THF gelöst. Nach Zugabe von 104,7 g (1,6 Mol) Zinkstaub und 31,7 g (592,5 mmol) Ammoniumchlorid, gelöst in 215 ml Wasser, wird der Ansatz 1¼ Stunde bei Raumtemperatur gerührt. Das Reaktionsgemisch wird über einen Glasfaserfilter abgesaugt und mit reichlich Essigsäureethylester gewaschen. Nach dem Einrotieren des Filtrats bis zur Trockne wird der Rückstand in 1500 ml Essigsäureethylester aufgenommen und die organische Phase zweimal mit je 150 ml Sole gewaschen. Nach dem Trocknen über Natriumsulfat und Abrotieren des Lösungsmittels verbleiben 28,1 g Rohprodukt. Aus einem zweiten Ansatz gleicher Größe werden 26,5 g erhalten.
Beide Rohprodukte werden gemeinsam an Kieselgel gesäult (Hexan/Essigsäureethylester als Elutionsmittel).
Die Ausbeute an 4-Hydroxy-3-amino-benzoesäuremethylester beträgt 31,3g (73,9%)., Schmelzpunkt 141 - 149 °C.

### b) (±)-2-Methyl-3-oxo-6-(methoxycarbonyl)-3,4-dihydro-2H-1,4-benzoxazin

3,71 g (154,6 mmol) Natriumhydrid (in Form einer 60%igen Suspension) werden in 340 ml Dimethylformamid aufgeschlämmt.Bei 0 °C werden zu dieser Suspension 15,5 g (92,7 mmol) 4-Hydroxy-3-amino-benzoesäure-methylester, gelöst in 170 ml Dimethylformamid innerhalb von 25 Minuten zugetropft. Nach einer Stunde Rühren bei Raumtemperatur werden 135 ml Tetrahydrofuran zugegeben und wiederum bei 0°, 16,78 g (92,6 mmol) (±)-2-Brompropionsäureethylester, gelöst in 340 ml Dimethylformamid, innherhalb von 10 Minuten zugetropft. Anschließend wird 15 Stunden bei Raumtemperatur und 2 Stunden bei 40 - 45 °C gerührt. Obwohl noch Ausgangsmaterial vorhanden ist, wird aufgearbeitet. Das Reaktionsgemisch wird vorsichtig mit 25 ml Wasser versetzt, kurz nachgerührt und dann bis zur Trockene eingeengt. Es wurde ein zweiter Ansatz mit identischen Einsatzmengen durchgeführt. Die Rückstände beider Ansätze werden in Essigsäureethylester aufgenommen. Dabei fällt ein Teil des Produktes aus, das abgesaugt wird. Das Filtrat wird nochmals einrotiert und mit wenig Essigester behandelt. Wiederum fällt Produkt aus. Anschließend wird das Filtrat an Kieselgel chromatographiert (Hexan/Essigsäureethylester als Laufmittel).
Die Gesamtausbeute an (±)-2-Methyl-3-oxo-6-(methoxycarbonyl)-3,4-dihydro-2H-1,4-benzoxazin-6-carbonsäuremethylester beträgt 19,33 g (50,8%), Schmelzpunkt 164-167 °C.

### c) (±)-2-Methyl-3-oxo-6-hydroxymethyl-3,4-dihydro-2H-1,4-benzoxazin

10,3 g (46,6 mmol) (±)-2-Methyl-3-oxo-6-(methoxycarbonyl)-3,4-dihydro-2H-1,4-benzoxazin werden in 164 ml Tetrahydrofuran gelöst. Nach Zugabe von 330 ml Toluol wird die Lösung auf - 15 °C gekühlt. Bei dieser Temperatur werden innerhalb von 30 Minuten 144,2 ml einer 20%igen DIBAH-Lösung in Toluol zugetropft. Es erfolgt eine Farbänderung von gelb nach orange. Nach 45' Minuten Rühren bei -15 °C werden bei dieser Temperatur 31 ml Isopropanol und bei 0 °C 68 ml Wasser zugetropft. Nach 2 ½ stündigem kräftigen Rühren bei Raumtemperatur wird der entstandene Niederschlag abgesaugt, mit Tetrahydrofuran gewaschen und das Filtrat einrotiert. Der Rückstand wird an Kieselgel chromatographiert (Laufmittel Methylenchlorid/Ethanol).
Insgesamt werden 5,09 g (56,6%) des gewünschten Alkohols erhalten, Schmelzpunkt 149 - 154 °C.

### d) (±)-2-Methyl-3-oxo-6-chlormethyl-3,4-dihydro-2H-1,4-benzoxazin

4,59 g (23,8 mmol) (±)-2-Methyl-3-oxo-6-hydroxymethyl-3,4-dihydro-2H-1,4-benzoxazin werden nach dem Lösen in 900 ml Methylenchlorid mit 5,53 g (54,6mmol) Triethylamin versetzt. Bei 0 °C werden 4,08 g (35,6 mmol) Methansulfonsäurechlorid zugetropft und der Ansatz anschließend bei Raumtemperatur 5 Stunden gerührt. Es werden weitere 3,8 ml Triethylamin und 1,4 ml Methansulfonsäurechiorid zugegeben und nochmals 20 Stunden bei Raumtemperatur gerührt, Ein Teil des Methylenchlorids (600 ml) wird abrotiert. Nach Verdünnen mit 1 I Diethylether wird die organische Phase zweimal mti je 50 ml Wasser, einmal mit 50 ml gesättigter Natriumhydrogencarbonatlösung und nochmals mit 50 ml Wasser gewaschen. Die organische Phase wird nach dem Trocknen einrotiert und der Rückstand an Kieselgel (Laufmittel Methylenchlorid/Ethanol) chromatographiert.Es werden 2,95 g (58,7%) des gewünschten Produktes neben 1,1 g (16,6%) des entsprechenden Mesylats erhalten, Schmelzpunkt 162 -169 °C.

### e) (±)-2-Methyl-3-oxo-6-[(1-imidazolyl)-methyl]-3,4-dihydro-2H-1,4-benzoxazin

1,25 g (5,9 mmol) (±)-2-Methyl-3-oxo-6-chlormethyl-3,4-dihydro-2H-1,4-benzoxazin werden in 12,5 ml Dimethylsulfoxid gelöst,mit 0,804 g (11,8 mmol) Imidazol versetzt und 8 Stunden bei 70 °C gerührt. Nach dem Rühren über Nacht bei Raumtemperatur wird das Reaktionsgemisch an der Ölpumpe zur Trockene einrotiert und der Rückstand an Kieselgel (Laufmittel Methylenchlorid/Ethanol) chromatographiert. Es werden 940 mg (65,7 %) der gewünschten Imidazolverbindung erhalten, Schmelzpunkt 219 - 222 °C.

### f) (±)-2-Methyl-3-thioxo-6-[(1-imidazolyl)-methyl]-3,4-dihydro-2H-1,4-benzoxazin

390 mg (1,603 mmol) (±)-2-Methyl-3-oxo-6-[(1-imidazolyl)-methyl]-3,4-dihydro-2H-1,4-benzoxazin werden in 42 ml Dimethoxyethan gelöst, mit 1,297 g (3,206 mmol) Lawesson-Reagenz versetzt und 46 Stunden bei Raumtemperatur gerührt. Nach dem Einrotieren des Lösungsmittels wird der Rückstand an Kieselgel (Laufmittel Methylenchlorid/Ethanol) chromatographiert. Die durch die Chromatographie erhaltenen Fraktionen werden durch Schütteln mit gesättigter Natriumhydrogencarbonatlösung von den polaren Verunreinigungen befreit. Es werden 238.5 mg (57%) des gewünschten Produktes isoliert, Schmelzpunkt 206 - 210 °C.

### g) (±)-2-Methyl-3-amino-6-[(1-imidazolyl)-methyl]-3,4-dihydro-2H-1,4-benzoxazin

155 mg (0,597 mmol (±)-2-Methyl-3-thioxo-6-[(1-imidazolyl)-methyl]-3,4-dihydro-2H-1,4-benzoxazin werden mit 10 ml 7N NH₃ in Methanol versetzt und 2,5 Stunden bei Raumtemperatur gerührt. Nach Zugabe von 20 ml Toluol werden anschließend die Lösungsmittel abrotiert und die gewünschte Verbindung quantitativ erhalten, Schmelzpunkt 152 - 156 °C.

## Patentansprüche

1. Verbindungen der Formel I, deren Tautomere, Stereoisomere, geometrische Isomere und Salze 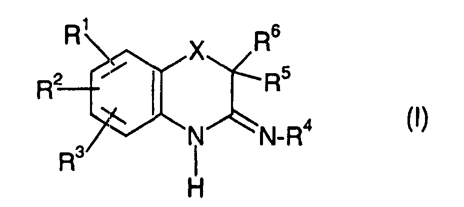 worin
X O oder S,
R¹ NO₂, Cyano, CF₃, -OCF₃, -SO₂NR⁷R⁸, -CONR⁷R⁸, -NR⁹-C(=NR¹⁰)-R¹¹, -NH-CS-NR⁷R⁸, -NH-CO-NR⁷R⁸, NR¹²R¹³, -CO-R¹⁴, C₆₋₁₀-Aryl, das gegebenenfalls substituiert ist mit Halogen, Cyano C₁₋₄-Alkyl, -S-R⁹, -O-R⁹,-NR⁷R⁸ oder CONR⁷R⁸,
5- oder 6-gliedriges Heteroaryl mit 1 bis 4 Heteroatomen wie Sauerstoff, Stickstoff oder Schwefel, das gegebenenfalls mit -OR⁹, -SR⁹, Halogen, C₁₋₄-Alkyl, NR⁷R⁸ oder CONR⁷R⁸ substituiert ist,
C₁₋₆-Alkyl, das substituiert ist mit Halogen, -OR⁹, -SR⁹, -NR⁷R⁸, - NR^{7'}R^{8'}, =NR⁷,=NOC₁₋₆-Alkyl, =N-NHAryl, Phenyl, C₃₋₇-Cycloalkyl oder 5- oder 6-gliedrigem Heteroaryl,
C₂₋₆-Alkenyl, das mit Halogen, CONH₂, C≡N oder Phenyl substituiert ist,
C₂₋₆-Alkinyl, das mit Halogen, CONH₂, C≡N oder Phenyl substituiert ist,
C₃₋₇-Cycloalkyl,
R² Wasserstoff bedeutet oder
R¹ und R² gemeinsam mit zwei benachbarten Kohlenstoffatomen einen 5-, 6-, 7-oder 8-gliedrigen Ring bilden, der monocyclisch oder bicyclisch, gesättigt oder ungesättigt und mit Benzol anelliert sein kann und bei dem 1 oder 2 CH₂-Gruppen durch Sauerstoff oder Carbonyl oder dessen Derivat, ausgewählt aus der Gruppe umfassend =N-OH, =N-OC₁₋₆-Alkyl, =N-NH₂ und =N-NH-Phenyl, ersetzt sein können und der mit -NR⁷R⁸, -NR^{7'}R^{8'} oder C₁₋₄-Alkyl substituiert sein kann.
R³ Wasserstoff, Halogen, -S-R⁹ oder -O-R⁹ bedeutet oder unabhängig voneinander eine der Bedeutungen von R¹ ist,
R⁴ Wasserstoff oder Acyl,
R⁵ Wasserstoff,
R⁶ C₃₋₇-Cycloalkyl, C₆₋₁₀-Aryl, C₁₋₆-Alkyl-, C₂₋₆-Alkenyl- oder C₂₋₆-Alkynylreste, die jeweils substituiert sein können mit Halogen, OH, O-C₁₋₆-Alkyl, SH, S-C₁₋₆-Alkyl, NR¹⁵R¹⁶, 5- oder 6-gliedriges Heteroaryl mit 1 - 3 N-, O- oder S-Atomen, Phenyl oder C₃₋₇-Cycloalkyl,
R⁷ und R⁸ Wasserstoff, C₁₋₆-Alkyl, gegebenenfalls mit Halogen oder C₁₋₄-Alkyl substituiertes Phenyl, gegebenenfalls mit Halogen oder C₁₋₄-Alkyl substituiertes Benzyl oder C₃₋₇-Cycloalkyl,
R^{7'} Wasserstoff, gegebenenfalls mit OH, Phenyl, Cyano, COO₁₋₄-Alkyl oder Carbonyl substituiertes C₁₋₆-Alkyl,
R^{8'} C₁₋₆-Alkyl, das substituiert ist mit C₃₋₇-Cycloalkyl, Indanyl, C₆₋₁₀-Aryl oder 5- oder 6-gliedrigen Heteroaryl mit 1 - 3 Stickstoff-, Sauerstoff- oder Schwefelatomen, wobei der Aryl- und Heteroarylrest mit Halogen, C₁₋₄-Alkyl, C₁₋₄-Alkoxy, CF₃, NO₂, NH2, N(C ₁₋₄-Alkyl)₂, SO₂CH₃, -O-CH₂-O, SO₂NH₂, OH oder COO-C₁₋₄-Alkyl substituiert sein können oder Indanyl oder 1, 2, 3, 4-Tetrahydronaphthyl,
R^{7'} und R^{8'} gemeinsam mit dem Stickstoffatom einen 5 - 7-gliedrigen gesättigten Heterocyclus bilden, der ein weiteres Sauerstoff-, Stickstoff- oder Schwefelatom enthalten und mit C₁₋₄-Alkyl, Phenyl, Benzyl oder Benzoyl substituiert sein kann oder einen ungesättigten 5-gliedrigen Heterocyclus bilden, der 1 - 3 N-Atome enthalten und substituiert sein kann mit Phenyl, C₁₋₄-Alkyl, Halogen oder CH₂-OH,
R⁹, R¹⁰ und R¹⁵, R¹⁶ Wasserstoff oder C₁₋₆-Alkyl,
R¹¹ C₁₋₆-Alkyl, -NH₂, -NH-CH₃,-NH-CN, gegebenenfalls mit Halogen, C₁₋₄-Alkyl oder CF₃ substituiertes C₆₋₁₀-Aryl oder gegebenenfalls mit Halogen, C₁₋₄-Alkyl oder CF₃ substituiertes 5- oder 6-gliedriges Heteroaryl mit 1 bis 4 Stickstoff-, Schwefeloder Sauerstoffatomen,
R¹², R¹³ gemeinsam mit dem Stickstoffatom einen gesättigten 5-, 6- oder 7-gliedrigen Ring bilden, der ein weiteres Stickstoff-, Sauerstoff- oder Schwefelatom enthalten und substituiert sein kann mit C₁₋₄-Alkyl, Phenyl, Benzyl oder Benzoyl und
R¹⁴ Wasserstoff, Phenyl, gegebenenfalls mit CO₂H, CO₂C₁₋₆-Alkyl, Hydroxy, C₁₋₄-Alkoxy, Halogen, NR⁷R⁸, NR¹²R¹³,CONR⁷R⁸ oder Phenyl substituiertes C₁₋₆-Alkyl oder gegebenenfalls mit Phenyl, Cyano, CONR⁷R⁸ oder CO₂C₁₋₄-Alkyl substituiertes C₂₋₆-Alkenyl
bedeuten.

2. Verbindungen nach Anspruch 1 oder 13, worin R^{8'} C₁₋₆-Alkyl ist, das substituiert ist mit C₃₋₇-Cycloalkyl, Indanyl, C₆₋₁₀-Aryl oder 5- oder 6-gliedrigen Heteroaryl mit 1 - 3 Stickstoff-, Sauerstoff- oder Schwefelatomen, wobei der Aryl- und Heteroarylrest mit Halogen, C₁₋₄-Alkyl, C₁₋₄-Alkoxy, CF₃, NO₂, -O-CH₂-O, SO₂NH₂, OH oder COO-C₁₋₄-Alkyl substituiert sein können.

3. Verbindungen der Formel I nach Anspruch 1, deren Tautomere, Stereoisomere, geometrische Isomere und Salze 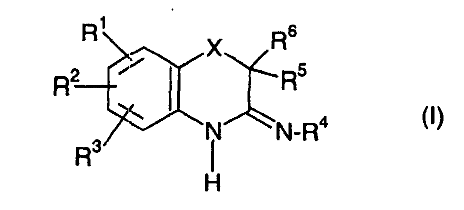 worin
X und R⁴ bis R¹⁶ die Bedeutung gemäß Anspruch 1 haben und
R¹ NO₂, Cyano, CF₃, -OCF₃, -SO₂NR⁷R⁸, -CONR⁷R⁸, -NR⁹-C(=NR¹⁰)-R^{11,}-NH-CS-NR⁷R⁸, -NH-CO-NR⁷R⁸, NR¹²R¹³, -CO-R¹⁴, C₆₋₁₀-Aryl, das gegebenenfalls substituiert ist mit Halogen, Cyano C₁₋₄-Alkyl, -S-R⁹, -O-R⁹,-NR⁷R⁸ oder CONR⁷R⁸,
5- oder 6-gliedriges Heteroaryl mit 1 bis 4 Heteroatomen wie Sauerstoff, Stickstoff oder Schwefel, das gegebenenfalls mit -OR⁹, -SR⁹, Halogen, C₁₋₄-Alkyl, NR⁷R⁸ oder CONR⁷R⁸ substituiert ist,
C₁₋₆-Alkyl, das substituiert ist mit Halogen, -OR⁹, -SR⁹, -NR⁷R⁸, =NR⁷,=NOC₁₋₆-Alkyl, =N-NHAryl, Phenyl, C₃₋₇-Cycloalkyl, 5- oder 6-gliedrigem Heteroaryl,
C₂₋₆-Alkenyl, das gegebenenfalls mit Halogen oder Phenyl substituiert ist,
C₂₋₆-Alkinyl, das gegebenenfalls mit Halogen oder Phenyl substituiert ist,
R² Wasserstoff bedeutet oder
R¹ und R² gemeinsam mit zwei benachbarten Kohlenstoffatomen einen 5-, 6- oder 7-gliedrigen Ring bilden, der monocyclisch oder bicyclisch, gesättigt oder ungesättigt sein kann und bei dem 1 oder 2 CH₂ Gruppen durch Sauerstoff oder Carbonyl oder dessen Derivat, ausgewählt aus der Gruppe umfassend =N-OH, =N-OC₁₋₆-Alkyl, =N-NH₂ und =N-NH-Phenyl, ersetzt sein können und der 1 bis 4-fach mit NR⁷R⁸ oder C₁₋₄-Alkyl substituiert sein kann,
R³ Wasserstoff, Halogen, -S-R⁹ oder -O-R⁹ bedeutet oder unabhängig voneinander eine der Bedeutungen von R¹ ist,
bedeuten.

4. Verbindungen der Formel I nach Anspruch 1, deren Tautomere, Stereoisomere, geometrische Isomere und Salze 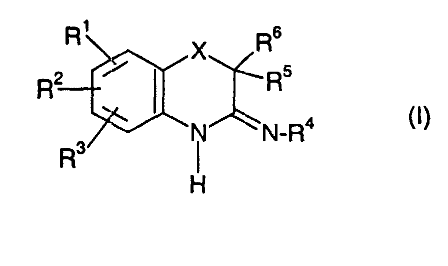 worin
R¹ C₁₋₆-Alkyl, das substituiert ist mit NR^{7'}R^{8'},
R² Wasserstoff oder
R¹ und R² gemeinsam mit zwei benachbarten Kohlenstoffatomen einen 5-, 6-, 7- oder 8-gliedrigen Ring bilden, der monocyclisch oder bicyclisch, gesättigt oder ungesättigt sein kann und bei dem 1 oder 2 CH2-Gruppen durch Sauerstoff oder Carbonyl oder dessen Derivat, ausgewählt aus der Gruppe umfassend =N-OH, =N-OC₁₋₆-Alkyl, =N-NH₂ und =N-NH-Phenyl, ersetzt sein können und der mit NR^{7'}R^{8'} substituiert ist,
R³ Wasserstoff, Halogen, NO₂, Cyano, CF₃, -OCF₃, -SO₂NR⁷R⁸, -CONR⁷R⁸, -NR⁹-C(=NR¹⁰)-R¹¹, -NH-CS-NR⁷R⁸, -NH-CO-NR⁷R⁸, NR¹²R¹³, -CO-R¹⁴, C₆₋ ₁₀-Aryl, das gegebenenfalls substituiert ist mit Halogen, Cyano, C₁₋₄-Alkyl, -S-R⁹, - O-R⁹, -NR⁷R⁸ oder CONR⁷R⁸,
5- oder 6-gliedriges Heteroaryl mit 1 bis 4 Heteroatomen wie Sauerstoff, Stickstoff oder Schwefel, das gegebenenfalls mit -OR⁹, -SR⁹, Halogen, C₁₋₄-Alkyl, NR⁷R⁸ oder CONR⁷R⁸ substituiert ist,
C₁₋₆-Alkyl, das substituiert ist mit Halogen, -OR⁹, -SR⁹, -NR⁷R⁸, =NR⁷,=NOC₁₋₆-Alkyl, =N-NHAryl, Phenyl, C₃₋₇-Cycloalkyl, oder 5- oder 6-gliedrigem Heteroaryl,
C₂₋₆-Alkenyl, das gegebenenfalls mit Halogen oder Phenyl substituiert ist,
C₂₋₆-Alkinyl, das gegebenenfalls mit Halogen oder Phenyl substituiert ist,
R^{7'} Wasserstoff, gegebenenfalls mit OH, Phenyl, Cyano, COO₁₋₄-Alkyl oder Carbonyl substituiertes C₁₋₆-Alkyl,
R^{8'} C₁₋₆-Alkyl, das substituiert ist mit C₃₋₇-Cycloalkyl, Indanyl, C₆₋₁₀-Aryl oder 5- oder 6-gliedrigen Heteroaryl mit 1 - 3 Stickstoff-, Sauerstoff- oder Schwefelatomen, wobei der Aryl- und Heteroarylrest mit Halogen, C₁₋₄-Alkyl, C₁₋₄-Alkoxy, CF₃, NO₂, -O-CH₂-O, SO₂NH₂, OH oder COO-C₁₋₄-Alkyl substituiert sein können oder
R^{7'} und R^{8'} gemeinsam mit dem Stickstoffatom einen 5 - 7-gliedrigen gesättigten Heterocyclus bilden, der ein weiteres Sauerstoff-, Stickstoff- oder Schwefelatom enthalten und mit C₁₋₄-Alkyl, Phenyl, Benzyl oder Benzoyl substituiert sein kann oder einen ungesättigten 5-gliedrigen Heterocyclus bilden, der 1 - 3 N-Atome enthalten und substituiert sein kann mit Phenyl, C₁₋₄-Alkyl, Halogen oder CH₂-OH,
bedeuten und
X und R⁴ bis R¹⁶ die Bedeutung gemäß Anspruch 1 haben.

5. Verbindungen gemäß einem der Ansprüche 1 - 4, worin R ¹ und R² gemeinsam mit 2 benachbarten Kohlenstoffatomen gesättigtes oder ungesättigtes C₃₋₈-Alkylen bedeuten, bei dem eine oder zwei CH₂-Gruppen durch O oder CO ersetzt sein können, wobei der Alkylenrest einen ankondensierten Benzolrest enthalten kann und durch NR⁷R⁸, NR^{7'}R^{8'} oder C₁₋₄-Alkyl substituiert sein kann.

6. Verbindungen gemäß Anspruch 1 oder 3, worin R¹ C₁₋₆-Alkyl bedeutet, das mit NR⁷R⁸ substituiert ist.

7. 6-Phenyl-2-methyl-3-amino-1,4-benzoxazin
4H-Napht[2,3-b]-2-methyl-3-amino-1,4-oxazin
4H-Napht[1,2-b]-2-ethyl-3-amino-1,4-oxazin
6,7-Cyclopenteno-2-methyl-3-amino-1,4-benzoxazin
6,7-Cyclopenteno-2-ethyl-3-amino-1,4-benzoxazin
5,6-Cyclopenteno-2-methyl-3-amino-1,4-benzoxazin
5,6-Cyclopenteno-2-ethyl-3-amino-1,4-benzoxazin
6,7-(Methylendioxy)-2-methyl-3-amino-1,4-benzoxazin
6,7-(Methylendioxy)-2-ethyl-3-amino-1,4-benzoxazin
6-Cyclohexyl-2-methyl-3-amino-1,4-benzoxazin
7-(1-Morpholinyl)-2-methyl-3-amino-1,4-benzoxazin
2-Ethyl-3-amino-6,7,8,9-tetrahydro-napht[2,3-b]-1,4-oxazin
2-Methyl-3-amino-6,7,8,9-tetrahydro-napht[2,3-b]-1,4-oxazin
3-Methyl-2-amino-6,7,8,9-tetrahydro-napht[2,1-b]-1,4-oxazin
3-Ethyl-2-amino-6,7,8,9-tetrahydro-napht[2,1-b]-1,4-oxazin
2-Methyl-3-amino-6,7,8,9-tetrahydro-napht[2,3-b]-1,4-thiazin
2-Ethyl-3-amino-6,7,8,9-tetrahydro-napht[2,3-b]-1,4-thiazin
6,7-Cyclopenteno-2-methyl-3-amino-1,4-benzothiazin
N-[(2-Methyl-1,4-benzoxazin-3-amino-7-yl]-(2-thienyl)carboximidamid
gemäß Anspruch 1 und 2.

8. 6-((Thien-2-yl)-methylaminomethyl)-3-amino-2-methyl-1,4-benzoxazin Dihydrochlorid
6-((4-Methoxybenzyl)aminomethyl)-3-amino-2-methyl-1,4-benzoxazin Dihydrochlorid
6-((3-Chlorbenzyl)aminomethyl)-3-amino-2-methyl-1,4-benzoxazin Dihydrochlorid
6-((4-Chlorbenzyl)aminomethyl)-3-amino-2-methyl-1,4-benzoxazin Dihydrochlorid
6-((4-Pyridyl)-methylaminomethyl)-3-amino-2-methyl-1,4-benzoxazin Dihydrochlorid
6-(Benzylamino)-6,7-cyclopenteno-3-amino-2-methyl-1,4-benzoxazin Dihydrochlorid
6-(Benzylamino)-6,7-cyclohexeno-3-amino-2-methyl-1,4-benzoxazin Dihydrochlorid
6-((Thien-2-yl)-methylamino)-6,7-cyclohexeno-3-amino-2-methyl-1,4-benzoxazin Dihydrochlorid
6-((Thien-2-yl)-methylamino)-6,7-cyclopenteno-3-amino-2-methyl-1,4-benzoxazin Dihydrochlorid
6-(3-Chlorbenzylamino)-6,7-cyclopenteno-3-amino-2-methyl-1,4-benzoxazin Dihydrochlorid
6-(3-Chlorbenzylamino)-6,7-cyclohexeno-3-amino-2-methyl-1,4-benzoxazin Dihydrochlorid
6-(2-(Benzylamino)-prop-1-yl)-3-amino-2-methyl-1,4-benzoxazin Dihydrochlorid
7-(3-Chlorbenzylamino)-6,7-cyclohexeno-3-amino-2-methyl-1,4-benzoxazin Dihydrochlorid
6-((3,4-Dichlorbenzyl)aminomethyl)-3-amino-2-methyl-1,4-benzoxazin Dihydrochlorid
6-((2-Chlorbenzyl)aminomethyl)-3-amino-2-methyl-1,4-benzoxazin Dihydrochlorid
6-((2,4-Dichlorbenzyl)aminomethyl)-3-amino-2-methyl-1,4-benzoxazin Dihydrochlorid
6-((2,3-Dimethylbenzyl)aminomethyl)-3-amino-2-methyl-1,4-benzoxazin Dihydrochlorid
6-((3-Fluorbenzyl)aminomethyl)-3-amino-2-methyl-1,4-benzoxazin Dihydrochlorid
6-((Indan-1-yl) aminomethyl)-3-amino-2-methyl-1,4-benzoxazin Dihydrochlorid
6-((Indan-2-yl) aminomethyl)-3-amino-2-methyl-1,4-benzoxazin Dihydrochlorid
6-((Cyclohexylmethyl)aminomethyl)-3-amino-2-methyl-1,4-benzoxazin Dihydrochlorid
6-((1,2,3,4-Tetrahydronaphth-1-yl) aminomethyl)-3-amino-2-methyl-1,4-benzoxazin Dihydrochlorid
6-((3-Methoxybenzyl)aminomethyl)-3-amino-2-methyl-1,4-benzoxazin Dihydrochlorid
6-((3-Nitrobenzyl)aminomethyl)-3-amino-2-methyl-1,4-benzoxazin Dihydrochlorid
6-((4-Nitrobenzyl)aminomethyl)-3-amino-2-methyl-1,4-benzoxazin Dihydrochlorid
6-((4-Sulfamoylbenzyl)aminomethyl)-3-amino-2-methyl-1,4-benzoxazin Dihydrochlorid
6-((4-Methylsulfonylbenzyl)aminomethyl)-3-amino-2-methyl-1,4-benzoxazin Dihydrochlorid
6-((4-Fluorbenzyl) aminomethyl)-3-amino-2-methyl-1,4-benzoxazin Dihydrochlorid
6-((4-Dimethylaminobenzyl)aminomethyl)-3-amino-2-methyl-1,4-benzoxazin Trihydrochlorid
6-((2-Fluorbenzyl)aminomethyl)-3-amino-2-methyl-1,4-benzoxazin Dihydrochlorid
gemäß Anspruch 1.

9. Arzneimittel enthaltend eine Verbindung gemäß einem der Ansprüche 1 - 8 und einen oder mehrere übliche Träger- oder Hilfsstoffe.

10. Verwendung der Verbindungen gemäß einem der Ansprüche 1 - 8 zur Herstellung eines Arzneimittels.

11. Verfahren zur Herstellung der Verbindungen der Formel I gemäß einem der Ansprüche 1 - 8 oder 13 - 15, dadurch daß man eine Verbindung der Formel II oder deren Salz 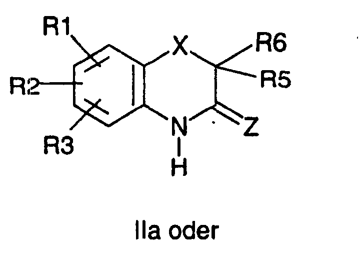 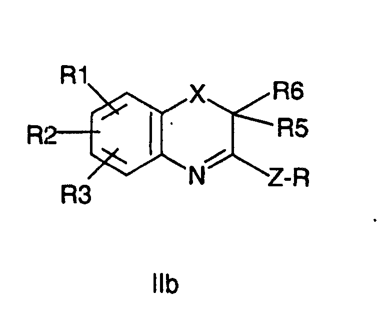 worin
R¹, R², R³, R⁵, R⁶ und X die obige Bedeutung haben, Z Sauerstoff oder Schwefel ist und R C₁₋₆-Alkyl bedeutet, mit Ammoniak oder primären oder sekundären Aminen umsetzt, wobei vorhandene primäre und sekundäre Aminogruppen gegebenenfalls intermediär geschützt sind und gewünschtenfalls anschließend acyliert, die Isomeren trennt oder die Salze bildet.

12. Verbindungen der Formel IIa, 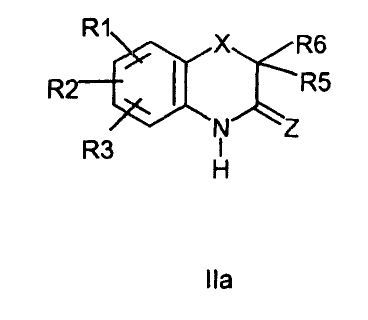 worin R³, R⁵, R⁶, X und Z die obige Bedeutung haben und R¹ und R² gemeinsam mit zwei benachbarten Kohlenstoffatomen einen 5-, 6-, 7- oder 8-gliedrigen Ring bilden, der monocyclisch oder bicyclisch, gesättigt oder ungesättigt und mit Benzol annelliert sein kann und bei dem 1 oder 2 CH₂-Gruppen durch Sauerstoff oder Carbonyl oder dessen Derivat, ausgewählt aus der Gruppe umfassend =N-OH, =N-OC₁₋₆-Alkyl, =N-NH₂ und =N-NH-Phenyl, ersetzt sein können und der mit -NR⁷R⁸, -NR^{7'}R^{8'} oder C₁₋₄-Alkyl substituiert sein kann.

13. Verbindungen gemäß Anspruch 1 oder 4, worin R¹ C₁₋₆-Alkyl bedeutet, das mit NR^{7'}R^{8'} substituiert ist.

14. Verbindungen nach einem der Ansprüche 1 - 6 oder 13, worin R⁶ C₁₋₆-Alkyl und R⁴ Wasserstoff ist.

15. Verbindungen nach einem der Ansprüche 1 - 5, worin R¹ und R² gemeinsam mit zwei benachbarten Kohlenstoffatomen einen 5-, 6-, 7- oder 8-gliedrigen Ring bilden, der mit NR⁷R⁸, NR^{7'}R^{8'} oder C₁₋₄-Alkyl substituiert sein kann.

16. Verwendung der Verbindungen nach einem der Ansprüche 1 - 6 oder 13 - 15 zur Herstellung eines Arzneimittels zur Behandlung einer neurodegenerativen, autoimmunen, inflammatorischen oder Herz-Kreislauf-Erkrankung.

17. Verbindungen nach Anspruch 12, worin R⁶ C₁₋₆-Alkyl ist.

## Claims

1. Compounds of Formula I, their tautomers, stereoisomers, geometric isomers and salts: 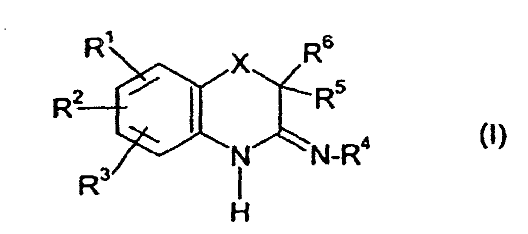 in which
X means O or S,
R¹ means NO₂, cyano, CF₃, -OCF₃, -SO₂NR⁷R⁸, -CONR⁷R⁸, -NR⁹-C(=NR¹⁰)-R¹¹, -NH-CS-NR⁷R⁸, -NH-CO-NR⁷R⁸, NR¹²R¹³, -CO-R¹⁴, C₆₋₁₀ aryl, which optionally is substituted with halogen, cyano C₁₋₄ alkyl, -S-R⁹, -O-R⁹, -NR⁷R⁸ or CONR⁷R⁸,
5- or 6-membered heteroaryl with 1 to 4 heteroatoms such as oxygen, nitrogen or sulphur, which optionally is substituted with -OR⁹, -SR⁹, halogen, C₁₋₄ alkyl, NR⁷R⁸ or CONR⁷R⁸,
C₁₋₆ alkyl, which is substituted with halogen, -OR⁹, -SR⁹, -NR⁷R⁸, -NR^{7'}R^{8'}, =NR⁷, =NOC₁₋₆ alkyl, =N-NHaryl, phenyl, C₃₋₇ cycloalkyl or 5- or 6-membered heteroaryl,
C₂₋₆ alkenyl, which is substituted with halogen, CONH₂, C=N or phenyl,
C₂₋₆ alkynyl, which is substituted with halogen, CONH₂, C=N or phenyl,
C₃₋₇ cycloalkyl,
R² means hydrogen or
R¹ and R² together with two adjacent carbon atoms form a 5-, 6-, 7- or 8-membered ring, which can be monocyclic or bicyclic, saturated or unsaturated and can be anellated with benzene and in which 1 or 2 CH₂ groups can be replaced by oxygen or carbonyl or its derivative, chosen from the group consisting of =N-OH, =N-OC₁₋₆ alkyl, =N-NH₂ and =N-NH-phenyl, and which can be substituted with -NR⁷R⁸, -NR^{7'}R^{8'} or C₁₋₄ alkyl,
R³ means hydrogen, halogen, -S-R⁹ or -O-R⁹ or independently has one of the meanings of R¹,
R⁴ means hydrogen or acyl,
R⁵ means hydrogen,
R⁶ means C₃₋₇ cycloalkyl, C₆₋₁₀ aryl, C₁₋₆ alkyl, C₂₋₆ alkenyl or C₂₋₆ alkynyl radicals, which can be substituted in each case with halogen, OH, O-C₁₋₆ alkyl, SH, S-C₁₋₆ alkyl, NR¹⁵R¹⁶, 5- or 6-membered heteroaryl with 1-3 N, O or S atoms, phenyl or C₃₋₇ cycloalkyl,
R⁷ and R⁸ mean hydrogen, C₁₋₆ alkyl, phenyl optionally substituted with halogen or C₁₋₄ alkyl, benzyl optionally substituted with halogen or C₁₋₄ alkyl or C₃₋₇ cycloalkyl,
R^{7'} means hydrogen, C₁₋₆ alkyl optionally substituted with OH, phenyl, cyano, COO₁₋₄ alkyl or carbonyl,
R^{8'} means C₁₋₆ alkyl, which is substituted with C₃₋₇ cycloalkyl, indanyl, C₆₋₁₀ aryl or 5- or 6-membered heteroaryl with 1-3 nitrogen, oxygen or sulphur atoms, whereby the aryl and heteroaryl radicals can be substituted with halogen, C₁₋₄ alkyl, C₁₋₄ alkoxy, CF₃, NO₂, NH₂, N(C₁₋₄ alkyl)₂, SO₂CH₃, -O-CH₂-O, SO₂NH₂, OH or COO-C₁₋₄ alkyl or indanyl or 1,2,3,4-tetrahydronaphthyl,
R^{7'} and R^{8'} together with the nitrogen atom form a 5- to 7-membered saturated heterocycle, which can contain another oxygen, nitrogen or sulphur atom and can be substituted with C₁₋₄ alkyl, phenyl, benzyl or benzoyl or form an unsaturated 5-membered heterocycle, which can contain 1-3 N atoms and can be substituted with phenyl, C₁₋₄ alkyl, halogen or CH₂-OH,
R⁹, R¹⁰ and R¹⁵, R¹⁶ mean hydrogen or C₁₋₆ alkyl,
R¹¹ means C₁₋₆ alkyl, -NH₂, -NH-CH₃, -NH-CN, C₆₋₁₀ aryl optionally substituted with halogen, C₁₋₄ alkyl or CF₃, or 5- or 6-membered heteroaryl with 1 to 4 nitrogen, sulphur or oxygen atoms that is optionally substituted with halogen, C₁₋₄ alkyl or CF₃,
R¹², R¹³ together with the nitrogen atom form a saturated 5-, 6- or 7-membered ring, which can contain another nitrogen, oxygen or sulphur atom and can be substituted with C₁₋₄ alkyl, phenyl, benzyl or benzoyl and
R¹⁴ means hydrogen, phenyl, C₁₋₆ alkyl optionally substituted with CO₂H, CO₂C₁₋₆ alkyl, hydroxy, C₁₋₄ alkoxy, halogen, NR⁷R^{8,} NR¹²R¹³, CONR⁷R⁸ or phenyl, or C₂₋₆ alkenyl optionally substituted with phenyl, cyano, CONR⁷R⁸ or CO₂C₁₋₄ alkyl.

2. Compounds according to Claim 1, wherein R^{8'} is C₁₋₆ alkyl, which is substituted with C₃₋₇ cycloalkyl, indanyl, C₆₋₁₀ aryl or 5- or 6-membered heteroaryl with 1-3 nitrogen, oxygen or sulphur atoms, whereby the aryl and heteroaryl radicals can be substituted with halogen, C₁₋₄ alkyl, C₁₋₄ alkoxy, CF₃, NO₂, -O-CH₂-O, SO₂NH₂, OH or COO-C₁₋₄ alkyl.

3. Compounds of Formula I according to Claim 1, their tautomers, stereoisomers, geometric isomers and salts 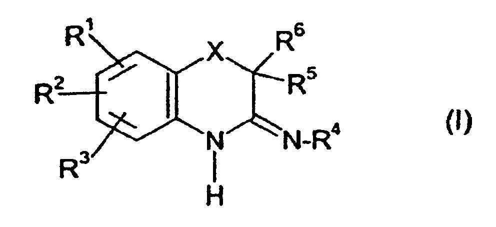 in which
X and R⁴ and R¹⁶ have the meaning according to Claim 1 and
R¹ means NO₂, cyano, CF₃, -OCF₃, -SO₂NR⁷R⁸, -CONR⁷R⁸, -NR⁹-C(=NR¹⁰)-R¹¹, -NH-CS-NR⁷R⁸, -NH-CO-NR⁷R⁸, NR¹²R¹³, -CO-R¹⁴, C₆₋₁₀ aryl, which optionally is substituted with halogen, cyano C₁₋₄ alkyl, -S-R⁹, -O-R⁹, -NR⁷R⁸ or CONR⁷R⁸,
5- or 6-membered heteroaryl with 1 to 4 heteroatoms, such as oxygen, nitrogen or sulphur, which optionally is substituted with -OR⁹, -SR⁹, halogen, C₁₋₄ alkyl, NR⁷R⁸ or CONR⁷R⁸,
C₁₋₆ alkyl, which is substituted with halogen, -OR⁹, -SR⁹, -NR⁷R⁸, =NR⁷, =NOC₁₋₆ alkyl, =N-NHaryl, phenyl, C₃₋₇ cycloalkyl or 5- or 6-membered heteroaryl,
C₂₋₆ alkenyl, which is optionally substituted with halogen or phenyl,
C₂₋₆ alkynyl, which optionally is substituted with halogen or phenyl,
R² means hydrogen or
R¹ and R² together with two adjacent carbon atoms form a 5-, 6- or 7-membered ring, which can be monocyclic or bicyclic, saturated or unsaturated and in which 1 or 2 CH₂ groups can be replaced by oxygen or carbonyl or its derivative, chosen from the group consisting of =N-OH, =N-OC₁₋₆ alkyl, =N-NH₂ and =N-NH-phenyl, and which can be substituted in one to four places with NR⁷R⁸ or C₁₋₄ alkyl,
R³ means hydrogen, halogen, -S-R⁹ or -O-R⁹ or independently has one of the meanings of R¹.

4. Compounds of Formula I according to Claim 1, their tautomers, stereoisomers, geometric isomers and salts 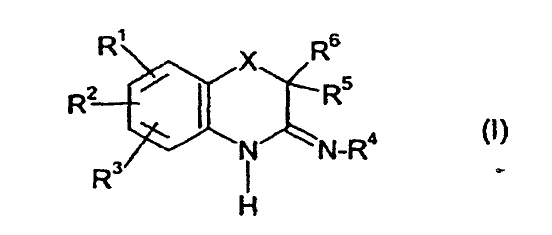 in which
R¹ means C₁₋₆ alkyl, which is substituted with NR^{7'}R^{8'},
R² means hydrogen or
R¹ and R² together with two adjacent carbon atoms form a 5-, 6-, 7- or 8-membered ring, which can be monocyclic or bicyclic, saturated or unsaturated and in which 1 or 2 CH₂ groups can be replaced by oxygen or carbonyl or its derivative, chosen from the group consisting of =N-OH, =N-OC₁₋₆ alkyl, =N-NH₂ and N-NH-phenyl, and which is substituted with -NR^{7'}R^{8'},
R³ means hydrogen, halogen, NO₂, cyano, CF₃, -OCF₃, -SO₂NR⁷R⁸, -CONR⁷R⁸, -NR⁹-C(=NR¹⁰)-R¹¹, -NH-CS-NR⁷R⁸, -NH-CO-NR⁷R⁸, NR¹²R¹³ -CO-R¹⁴, C₆₋₁₀ aryl, which optionally is substituted with halogen, cyano, C₁₋₄ alkyl, -S-R⁹, -O-R⁹, -NR⁷R⁸ or CONR⁷R⁸,
5- or 6-membered heteroaryl with 1 to 4 heteroatoms, such as oxygen, nitrogen or sulphur, which optionally is substituted with -OR⁹, -SR⁹, halogen, C₁₋₄ alkyl, NR⁷R⁸ or CONR⁷R⁸,
C₁₋₆ alkyl, which is substituted with halogen, -OR⁹, -SR⁹, -NR⁷R⁸, =NR⁷, =NOC₁₋₆ alkyl, =N-NHaryl, phenyl, C₃₋₇ cycloalkyl, or 5- or 6-membered heteroaryl,
C₂₋₆ alkenyl, which optionally is substituted with halogen or phenyl,
C₂₋₆ alkynyl, which optionally is substituted with halogen or phenyl,
R^{7'} means hydrogen, C₁₋₆ alkyl optionally substituted with OH, phenyl, cyano, COO₁₋₄ alkyl or carbonyl,
R^{8'} means C₁₋₆ alkyl, which is substituted with C₃₋₇ cycloalkyl, indanyl, C₆₋₁₀ aryl or 5- or 6-membered heteroaryl with 1-3 nitrogen, oxygen or sulphur atoms, whereby the aryl and heteroaryl radicals can be substituted with halogen, C₁₋₄ alkyl, C₁₋₄ alkoxy, CF₃, NO₂, -O-CH₂-O, SO₂NH₂, OH or COO-C₁₋₄ alkyl, or
R^{7'} and R^{8'} together with the nitrogen atom form a 5- to 7-membered saturated heterocycle, which can contain another oxygen, nitrogen or sulphur atom and can be substituted with C₁₋₄ alkyl, phenyl, benzyl or benzoyl or form an unsaturated 5-membered heterocycle, which can contain 1-3 N atoms and can be substituted with phenyl, C₁₋₄ alkyl, halogen or CH₂-OH, and
X and R⁴ to R¹⁶ have the meaning according to Claim 1.

5. Compounds according to one of Claims 1-4, in which R¹ and R² together with 2 adjacent carbon atoms are saturated or unsaturated C₃₋₈ alkylene, in which one or two CH₂ groups may be replaced with O or CO, wherein the alkylene radical optionally contains a slightly condensed benzene radical and is optionally substituted by NR⁷R⁸, NR^{7'}R^{8'} or C₁₋₄ alkyl.

6. Compounds according to Claim 1 or 3, in which R¹ means C₁₋₆ alkyl which is substituted with NR⁷R⁸.

7. 6-phenyl-2-methyl-3-amino-1,4-benzoxazine
4H-naphtho[2,3-b]-2-methyl-3-amino-1,4-oxazine
4H-naphth[1,2-b]-2-ethyl-3-amino-1,4-oxazine
6,7-cyclopenteno-2-methyl-3-amino-1,4-benzoxazine
6,7-cyclopenteno-2-ethyl-3-amino-1,4-benzoxazine
5,6-cyclopenteno-2-methyl-3-amino-1,4-benzoxazine
5,6-cyclopenteno-2-ethyl-3-amino-1,4-benzoxazine
6,7-(methylenedioxy)-2-methyl-3-amino-1,4-benzoxazine
6,7-(methylenedioxy)-2-ethyl-3-amino-1,4-benzoxazine
6-cyclohexyl-2-methyl-3-amino-1,4-benzoxazine
7-(1-morpholinyl)-2-methyl-3-amino-1,4-benzoxazine
2-ethyl-3-amino-6,7,8,9-tetrahydro-naphth[2,3-b]-1,4-oxazine
2-methyl-3-amino-6,7,8,9-tetrahydro-naphth[2,3-b]-1,4-oxazine
3-methyl-2-amino-6,7,8,9-tetrahydro-naphth[2,1-b]-1,4-oxazine
3-ethyl-2-amino-6,7,8,9-tetrahydro-naphth[2,1-b]-1,4-oxazine
2-methyl-3-amino-6,7,8,9-tetrahydro-naphtho[2,3-b]-1,4-thiazine
2-ethyl-3-amino-6,7,8,9-tetrahydro-naphtho[2,3-b]-1,4-thiazine
6,7-cyclopenteno-2-methyl-3-amino-1,4-benzothiazine
N-[(2-methyl-1,4-benzoxazin-3-amino-7-yl]-(2-thienyl)-carboximidamide according to Claim 1 and 2.

8. 6-((thien-2-yl)-methylaminomethyl)-3-amino-2-methyl-1,4-benzoxazine dihydrochloride
6-((4-methoxybenzyl)aminomethyl)-3-amino-2-methyl-1,4-benzoxazine dihydrochloride
6-((3-chlorobenzyl)aminomethyl)-3-amino-2-methyl-1,4-benzoxazine dihydrochloride
6-((4-chlorobenzyl)aminomethyl)-3-amino-2-methyl-1,4-benzoxazine dihydrochloride
6-((4-pyridyl)-methylaminomethyl)-3-amino-2-methyl-1,4-benzoxazine dihydrochloride
6-(benzylamino)-6,7-cyclopenteno-3-amino-2-methyl-1,4-benzoxazine dihydrochloride
6-(benzylamino)-6,7-cyclohexeno-3-amino-2-methyl-1,4-benzoxazine dihydrochloride
6-((thien-2-yl)-methylamino)-6,7-cyclohexeno-3-amino-2-methyl-1,4-benzoxazine dihydrochloride
6-((thien-2-yl)-methylamino)-6,7-cyclopenteno-3-amino-2-methyl-1,4-benzoxazine dihydrochloride
6-(3-chlorobenzylamino)-6,7-cyclopenteno-3-amino-2-methyl-1,4-benzoxazine dihydrochloride
6-(3-chlorobenzylamino)-6,7-cyclohexeno-3-amino-2-methyl-1,4-benzoxazine dihydrochloride
6-(2-(benzylamino)-prop-1-yl)-3-amino-2-methyl-1,4-benzoxazine dihydrochloride
7-(3-chlorobenzylamino)-6,7-cyclohexeno-3-amino-2-methyl-1,4-benzoxazine dihydrochloride
6-((3,4-dichlorobenzyl)aminomethyl)-3-amino-2-methyl-1,4-benzoxazine dihydrochloride
6-((2-chlorobenzyl)aminomethyl)-3-amino-2-methyl-1,4-benzoxazine dihydrochloride
6-((2,4-dichlorobenzyl)aminomethyl)-3-amino-2-methyl-1,4-benzoxazine dihydrochloride
6-((2,3-dimethylbenzyl)aminomethyl)-3-amino-2-methyl-1,4-benzoxazine dihydrochloride
6-((3-fluorobenzyl)aminomethyl)-3-amino-2-methyl-1,4-benzoxazine dihydrochloride
6-((indan-1-yl)-aminomethyl)-3-amino-2-methyl-1,4-benzoxazine dihydrochloride
6-((indan-2-yl)aminomethyl)-3-amino-2-methyl-1,4-benzoxazine dihydrochloride
6-((cyclohexylmethyl)aminomethyl)-3-amino-2-methyl-1,4-benzoxazine dihydrochloride
6-((1,2,3,4-tetrahydronaphth-1-yl)aminomethyl)-3-amino-2-methyl-1,4-benzoxazine dihydrochloride
6-((3-methoxybenzyl)aminomethyl)-3-amino-2-methyl-1,4-benzoxazine dihydrochloride
6-((3-nitrobenzyl)aminomethyl)-3-amino-2-methyl-1,4-benzoxazine dihydrochloride
6-((4-nitrobenzyl)aminomethyl)-3-amino-2-methyl-1,4-benzoxazine dihydrochloride
6-((4-sulphamoylbenzyl)aminomethyl)-3-amino-2-methyl-1,4-benzoxazine dihydrochloride
6-((4-methylsulphonylbenzyl)aminomethyl)-3-amino-2-methyl-1,4-benzoxazine dihydrochloride
6-((4-fluorobenzyl)aminomethyl)-3-amino-2-methyl-1,4-benzoxazine dihydrochloride
6-((4-dimethylaminobenzyl)aminomethyl)-3-amino-2-methyl-1,4-benzoxazine trihydrochloride
6-((2-fluorobenzyl)aminomethyl)-3-amino-2-methyl-1,4-benzoxazine dihydrochloride
according to Claim 1.

9. Pharmaceutical composition which comprises a compound according to one of Claims 1-8 and one or more customary vehicles or adjuvants.

10. Use of the compounds according to one of Claims 1-8 for producing a pharmaceutical composition.

11. Process for the production of the compounds of Formula I according to one of Claims 1-8 or 13-15, which comprises reacting a compound of Formula II or its salt 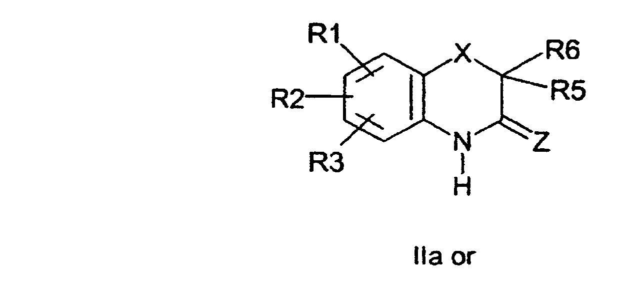 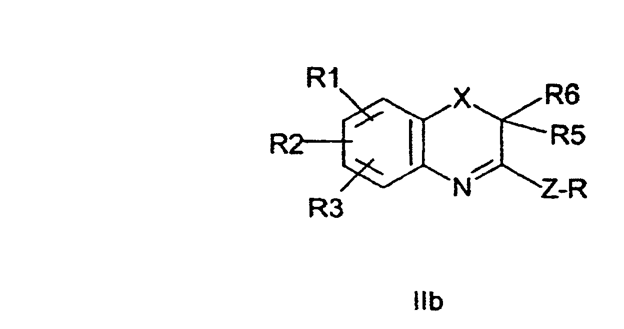 in which
R¹, R², R³, R⁵, R⁶ and X have the abovementioned meaning, Z is oxygen or sulphur and R means C₁₋₆ alkyl, with ammonia or primary or secondary amines, whereby existing primary and secondary amino groups are optionally intermediately protected and optionally then acylated, the isomers are separated or the salts are formed.

12. Compounds of Formula IIa 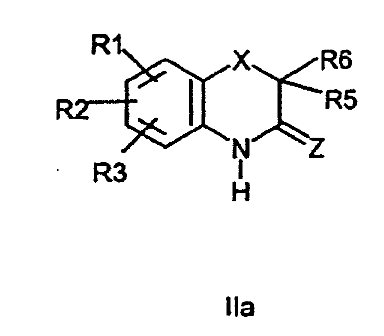 wherein R³,R⁵,R⁶, X and Z have the abovementioned meaning and R¹ and R² together with two adjacent carbon atoms form a 5-,6-,7- or 8-membered ring, which can be monocyclic or bicyclic, saturated or unsaturated and anellated with benzene and in which 1 or 2 CH₂ groups can be replacd by oxygen or carbonyl or its derivative, chosen from the group consisting of =N-OH, =N-OC₁₋₆alkyl, =N-NH₂ and =N-NH-phenyl, and which can be substituted with -NR⁷R⁸, -NR^{7'}R^{8'} or C₁₋₄ alkyl.

13. Compounds according to Claim 1 or 4, wherein R¹ means C₁₋₆ alkyl, which is substituted with NR^{7'}R^{8'}.

14. Compounds according to one of Claims 1-6 or 13, wherein R⁶ is C₁₋₆ alkyl and R⁴ is hydrogen.

15. Compounds according to one of Claims 1-5, wherein R¹ and R² together with two adjacent carbon atoms form a 5-,6-,7- or 8-membered ring, which can be substituted with NR⁷R⁸, NR^{7'}R^{8'} or C₁₋₄ alkyl.

16. Use of the compounds according to one of Claims 1-6 or 13-15 for producing a pharmaceutical preparation for treating neurodegenerative, auto-immune inflammatory, or cardiovascular disease.

17. Compounds according to Claim 12, wherein R⁶ is C₁₋₆ alkyl.

## Revendications

1. Composés de formule I, leurs tautomères, stéréoisomères, isomères géométriques et sels 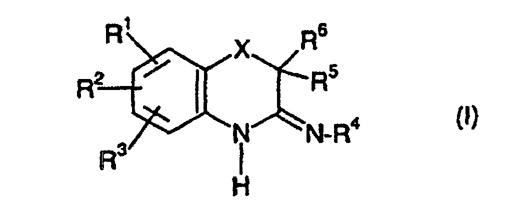 formule dans laquelle
X représente O ou S,
R¹ représente un groupe NO₂, cyano, CF₃, -OCF₃, -SO₂NR⁷R⁸, -CONR⁷R⁸, -NR⁹-C(=NR¹⁰)-R¹¹, -NH-CS-NR⁷R⁸, - NH-CO-NR⁷R⁸, NR¹²R¹³, -CO-R¹⁴, aryle en C₆₋₁₀ qui est éventuellement substitué par un atome d'halogène ou par un groupe cyano-alkyle(C₁₋₄), -S-R⁹, -O-R⁹, -NR⁷R⁸ ou CONR⁷R⁸,
un groupe hétéroaryle à 5 ou 6 chaînons ayant de 1 à 4 hétéroatomes, tels que des atomes d'oxygène, d'azote ou de soufre, qui est éventuellement substitué par un atome d'halogène ou par un groupe -OR⁹, -SR⁹, alkyle en C₁₋₄, NR⁷R⁸ ou CONR⁷R⁸,
un groupe alkyle en C₁₋₆ qui est substitué par un atome d'halogène ou par un groupe -OR⁹, -SR⁹, -NR⁷R⁸, -NR^{7'}R^{8'}, =NR⁷, =NO-alkyle(C₁₋₆), =N-NH-aryle, phényle, cycloalkyle en C₃₋₇ ou hétéroaryle à 5 ou 6 chaînons,
un groupe alcényle en C₂₋₆ qui est substitué par un atome d'halogène ou par un groupe CONH₂, C≡N ou phényle,
un groupe alcynyle en C₂₋₆ qui est substitué par un atome d'halogène ou par un groupe CONH₂, C≡N ou phényle,
un groupe cycloalkyle en C₃₋₇,
R² représente un atome d'hydrogène ou
R¹ et R² forment ensemble, avec deux atomes de carbone voisins, un cycle à 5, 6, 7 ou 8 chaînons, qui peut être monocyclique ou bicyclique, saturé ou insaturé et soudé à un noyau benzénique, et dans lequel 1 ou 2 groupes CH₂ peuvent être remplacés par un atome d'oxygène ou par un groupe carbonyle ou un dérivé de celui-ci, choisi dans l'ensemble comprenant les groupes =N-OH, =N-O-alkyle(C₁₋₆), =N-NH₂ et =N-NH-phényle et qui peut être substitué par un groupe -NR⁷R⁸, -NR^{7'}R^{8'} ou alkyle en C₁₋₄,
R³ représente un atome d'hydrogène ou d'halogène, ou un groupe -SR⁹ ou -O-R⁹ ou a, indépendamment l'un de l'autre, l'une des significations de R¹,
R⁴ représente un atome d'hydrogène ou un groupe acyle,
R⁵ représente un atome d'hydrogène,
R⁶ représente un groupe cycloalkyle en C₃₋₇, aryle en C₆₋₁₀, alkyle en C₁₋₆, alcényle en C₂₋₆ ou alcynyle en C₂₋₆, qui peuvent être substitués chacun par un atome d'halogène ou par un groupe OH, O-alkyle(C₁₋₆), SH, S-alkyle (C₁₋₆), NR¹⁵R¹⁶, hétéroaryle à 5 ou 6 chaînons, comportant 1-3 atomes de N, O ou S, phényle ou cycloalkyle en C₃₋₇,
R⁷ et R⁸ représentent un atome d'hydrogène ou un groupe alkyle en C₁₋₆, un groupe phényle éventuellement substitué par un atome d'halogène ou par un groupe alkyle en C₁₋₄, un groupe benzyle éventuellement substitué par un atome d'halogène ou par un groupe alkyle en C₁₋₄, ou un groupe cycloalkyle en C₃₋₇,
R^{7'} représente un atome d'hydrogène, un groupe alkyle en C₁₋₆ éventuellement substitué par un groupe OH, phényle, cyano, COO-alkyle(C₁₋₄) ou carbonyle,
R^{8'} représente un groupe alkyle en C₁₋₆ qui est substitué par un radical cycloalkyle en C₃₋₇, indanyle, aryle en C₆₋₁₀ ou hétéroaryle à 5 ou 6 chaînons, comportant 1-3 atomes d'azote, d'oxygène ou de soufre, le radical aryle et le radical hétéroaryle pouvant être substitués par des atomes d'halogène ou des groupes alkyle en C₁₋₄, alcoxy en C₁₋₄, CF₃, NO₂, NH₂, N-alkyle(C₁₋₄)₂, SO₂CH₃, -O-CH₂-O, SO₂NH₂, OH ou COO-alkyle(C₁₋₄), ou un groupe indanyle ou 1,2,3,4-tétrahydronaphtyle,
R^{7'} et R^{8'} forment ensemble avec l'atome d'azote un hétérocycle saturé à 5-7 chaînons, qui peut contenir un autre atome d'oxygène, d'azote ou de soufre et être substitué par un groupe alkyle en C₁₋₄, phényle, benzyle ou benzoyle, ou forment un hétérocycle insaturé à 5 chaînons, qui peut contenir 1-3 atomes de N et peut être substitué par un atome d'halogène ou par un groupe phényle, alkyle en C₁₋₄ ou CH₂OH,
R⁹, R¹⁰ et R¹⁵, R¹⁶ représentent un atome d'hydrogène ou un groupe alkyle en C₁₋₆,
R¹¹ représente un groupe alkyle en C₁₋₆, -NH₂, -NH-CH₃, - NH-CN, un groupe aryle en C₆₋₁₀ éventuellement substitué par un atome d'halogène ou par un groupe alkyle en C₁₋₄ ou CF₃, ou un groupe hétéroaryle à 5 ou 6 chaînons, comportant 1 à 4 atomes d'azote, de soufre ou d'oxygène et éventuellement substitué par un atome d'halogène ou par un groupe alkyle en C₁₋₄ ou CF₃,
R¹², R¹³ forment ensemble avec l'atome d'azote un cycle saturé à 5, 6 ou 7 chaînons, qui peut contenir un autre atome d'oxygène, d'azote ou de soufre et être substitué par un groupe alkyle en C₁₋₄, phényle, benzyle ou benzoyle et
R¹⁴ représente un atome d'hydrogène, un groupe phényle, un groupe alkyle en C₁₋₆ éventuellement substitué par un atome d'halogène ou par un groupe CO₂H, CO₂-alkyle(C₁₋₆), hydroxy, alcoxy en C₁₋₄, NR⁷R⁸, NR¹²R¹³, CONR⁷R⁸ ou phényle, ou un groupe alcényle en C₂₋₆ éventuellement substitué par un groupe phényle, cyano, CONR⁷R⁸ ou CO₂-alkyle(C₁₋₄).

2. Composés selon la revendication 1 ou 13, dans lesquels R^{8'} est un groupe alkyle en C₁₋₆ qui est substitué par un radical cycloalkyle en C₃₋₇, indanyle, aryle en C₆₋₁₀ ou hétéroaryle à 5 ou 6 chaînons, comportant 1-3 atomes d'azote, d'oxygène ou de soufre, le radical aryle et le radical hétéroaryle pouvant être substitués par des atomes d'halogène ou des groupes alkyle en C₁₋₄, alcoxy en C₁₋₄, CF₃, NO₂, -O-CH₂-O, SO₂NH₂, OH ou COO-alkyle(C₁₋₄).

3. Composés de formule I selon la revendication 1, leurs tautomères, stéréoisomères, isomères géométriques et sels 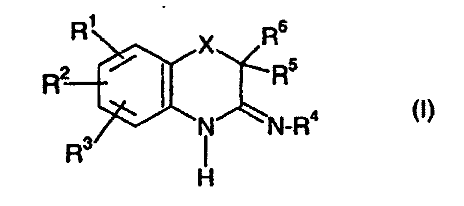 formule dans laquelle
X et R⁴ à R¹⁶ ont les significations selon la revendication 1 et
R¹ représente un groupe NO₂, cyano, CF₃, -OCF₃, -SO₂NR⁷R⁸, -CONR⁷R⁸, -NR⁹-C(=NR¹⁰)-R¹¹, -NH-CS-NR⁷R⁸, - NH-CO-NR⁷R⁸, NR¹²R¹³, -CO-R¹⁴, aryle en C₆₋₁₀ qui est éventuellement substitué par un atome d'halogène ou par un groupe cyano-alkyle(C₁₋₄), -S-R⁹, -O-R⁹, -NR⁷R⁸ ou CONR⁷R⁸,
un groupe hétéroaryle à 5 ou 6 chaînons ayant de 1 à 4 hétéroatomes, tels que des atomes d'oxygène, d'azote ou de soufre, qui est éventuellement substitué par un atome d'halogène ou par un groupe -OR⁹, -SR⁹, alkyle en C₁₋₄, NR⁷R⁸ ou CONR⁷R⁸,
un groupe alkyle en C₁₋₆ qui est substitué par un atome d' halogène ou par un groupe -OR⁹, -SR⁹, -NR⁷R⁸, =NR⁷, =NO-alkyle(C₁₋₆), =N-NH-aryle, phényle, cycloalkyle en C₃₋₇ ou hétéroaryle à 5 ou 6 chaînons,
un groupe alcényle en C₂₋₆ qui est substitué par un atome d'halogène ou par un groupe phényle,
un groupe alcynyle en C₂₋₆ qui est substitué par un atome d'halogène ou par un groupe phényle,
R² représente un atome d'hydrogène ou
R¹ et R² forment ensemble, avec deux atomes de carbone voisins, un cycle à 5, 6 ou 7 chaînons, qui peut être monocyclique ou bicyclique, saturé ou insaturé et dans lequel 1 ou 2 groupes CH₂ peuvent être remplacés par un atome d'oxygène ou par un groupe carbonyle ou un dérivé de celui-ci, choisi dans l'ensemble comprenant les groupes =N-OH, =N-O-alkyle(C₁₋₆), =N-NH₂ et =N-NH-phényle et qui peut être une à quatre fois substitué par un ou des groupes -NR⁷R⁸ ou alkyle en C₁₋₄,
R³ représente un atome d'hydrogène ou d'halogène, ou un groupe -S-R⁹ ou -O-R⁹ ou a, indépendamment les unes des autres, l'une des significations de R¹.

4. Composés de formule I selon la revendication 1, leurs tautomères, stéréoisomères, isomères géométriques et sels 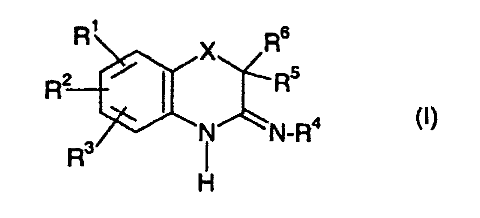 formule dans laquelle
R¹ représente un groupe alkyle en C₁₋₆ qui est substitué par -NR^{7'}R^{8'},
R² représente un atome d'hydrogène ou
R¹ et R² forment ensemble, avec deux atomes de carbone voisins, un cycle à 5, 6, 7 ou 8 chaînons, qui peut être monocyclique ou bicyclique, saturé ou insaturé et dans lequel 1 ou 2 groupes CH₂ peuvent être remplacés par un atome d'oxygène ou par un groupe carbonyle ou un dérivé de celui-ci, choisi dans l'ensemble comprenant les groupes =N-OH, =N-O-alkyle(C₁₋₆), =N-NH₂ et =N-NH-phényle et qui est substitué par un groupe -NR^{7'}R^{8'},
R³ représente un atome d'hydrogène ou d'halogène, ou un groupe NO₂, cyano, CF₃, -OCF₃, -SO₂NR⁷R⁸, -CONR⁷R⁸, - NR⁹-C(=NR¹⁰)-R¹¹, -NH-CS-NR⁷R⁸, -NH-CO-NR⁷R⁸, NR¹²R¹³, -CO-R¹⁴, aryle en C₆₋₁₀ qui est éventuellement substitué par un atome d'halogène ou par un groupe cyano, alkyle en C₁₋₄, -S-R⁹, -O-R⁹, -NR⁷R⁸ ou CONR⁷R⁸,
un groupe hétéroaryle à 5 ou 6 chaînons ayant de 1 à 4 hétéroatomes, tels que des atomes d'oxygène, d'azote ou de soufre, qui est éventuellement substitué par un atome d'halogène ou par un groupe -OR⁹, -SR⁹, alkyle en C₁₋₄, NR⁷R⁸ ou CONR⁷R⁸,
un groupe alkyle en C₁₋₆ qui est substitué par un atome d'halogène ou par un groupe -OR⁹, -SR⁹, -NR⁷R⁸, =NR⁷, =NO-alkyle(C₁₋₆), =N-NH-aryle, phényle, cycloalkyle en C₃₋₇ ou hétéroaryle à 5 ou 6 chaînons,
un groupe alcényle en C₂₋₆ qui est éventuellement substitué par un atome d'halogène ou par un groupe phényle,
un groupe alcynyle en C₂₋₆ qui est éventuellement substitué par un atome d'halogène ou par un groupe phényle,
R^{7'} représente un atome d'hydrogène, un groupe alkyle en C₁₋₆ éventuellement substitué par un groupe OH, phényle, cyano, COO-alkyle(C₁₋₄) ou carbonyle,
R^{8'} représente un groupe alkyle en C₁₋₆ qui est substitué par un radical cycloalkyle en C₃₋₇, indanyle, aryle en C₆₋₁₀ ou hétéroaryle à 5 ou 6 chaînons, comportant 1-3 atomes d'azote, d'oxygène ou de soufre, le radical aryle et le radical hétéroaryle pouvant être substitués par des atomes d'halogène ou des groupes alkyle en C₁₋₄, alcoxy en C₁₋₄, CF₃, NO₂, -O-CH₂-O, SO₂NH₂, OH ou COO-alkyle(C₁₋₄),
R^{7'} et R^{8'} forment ensemble avec l'atome d'azote un hétérocycle saturé à 5-7 chaînons, qui peut contenir un autre atome d'oxygène, d'azote ou de soufre et être substitué par un groupe alkyle en C₁₋₄, phényle, benzyle ou benzoyle, ou forment un hétérocycle insaturé à 5 chaînons, qui peut contenir 1-3 atomes de N et peut être substitué par un atome d'halogène ou par un groupe phényle, alkyle en C₁₋₄ ou CH₂OH,
et
X et R⁴ à R¹⁶ ont les significations selon la revendication 1.

5. Composés selon l'une quelconque des revendications 1 à 4, dans lesquels R¹ et R² forment ensemble, avec deux atomes de carbone voisins, un cycle alkylène en C₃₋₈ saturé ou insaturé, dans lequel un ou deux groupes CH₂ peuvent être remplacés par O ou CO, le radical alkylène pouvant contenir un radical benzénique soudé et pouvant être substitué par un groupe -NR⁷R⁸, -NR^{7'}R^{8'} ou alkyle en C₁₋₄.

6. Composés selon la revendication 1 ou 3, dans lesquels R¹ représente un groupe alkyle en C₁₋₆ qui est substitué par NR⁷R⁸.

7. 6-phényl-2-méthyl-3-amino-1,4-benzoxazine
4H-napht[2,3-b]-2-méthyl-3-amino-1,4-oxazine
4H-napht[1,2-b]-2-éthyl-3-amino-1,4-oxazine
6,7-cyclopenténo-2-méthyl-3-amino-1,4-benzoxazine
6,7-cyclopenténo-2-éthyl-3-amino-1,4-benzoxazine
5,6-cyclopenténo-2-méthyl-3-amino-1,4-benzoxazine
5,6-cyclopenténo-2-éthyl-3-amino-1,4-benzoxazine
6,7-(méthylènedioxy)-2-méthyl-3-amino-1,4-benzoxazine
6,7-(méthylènedioxy)-2-éthyl-3-amino-1,4-benzoxazine
6-cyclohexyl-2-méthyl-3-amino-1,4-benzoxazine
7-(1-morpholinyl)-2-méthyl-3-amino-1,4-benzoxazine
2-éthyl-3-amino-6,7,8,9-tétrahydro-napht[2,3-b]-1,4-oxazine
2-méthyl-3-amino-6,7,8,9-tétrahydro-napht[2,3-b]-1,4-oxazine
3-méthyl-2-amino-6,7,8,9-tétrahydro-napht[2,1-b]-1,4-oxazine
3-éthyl-2-amino-6,7,8,9-tétrahydro-napht[2,1-b]-1,4-oxazine
2-méthyl-3-amino-6,7,8,9-tétrahydro-napht[2,3-b]-1,4-thiazine
2-éthyl-3-amino-6,7,8,9-tétrahydro-napht[2,3-b]-1,4-thiazine
6,7-cyclopenténo-2-méthyl-3-amino-1,4-benzothiazine
N-[(2-méthyl-1,4-benzoxazine-3-amino-7-yl]-(2-thiényl)carboximidamide
selon les revendications 1 et 2.

8. Dichlorhydrate de 6-((thién-2-yl)-méthylaminométhyl)-3-amino-2-méthyl-1,4-benzoxazine
dichlorhydrate de 6-((4-méthoxybenzyl)aminométhyl)-3-amino-2-méthyl-1,4-benzoxazine
dichlorhydrate de 6-((3-chlorobenzyl)aminométhyl)-3-amino-2-méthyl-1,4-benzoxazine
dichlorhydrate de 6-((4-chlorobenzyl)aminométhyl)-3-amino-2-méthyl-1,4-benzoxazine
dichlorhydrate de 6-((4-pyridyl)-méthylaminométhyl)-3-amino-2-méthyl-1,4-benzoxazine
dichlorhydrate de 6-(benzylamino)-6,7-cyclopenténo-3-amino-2-méthyl-1,4-benzoxazine
dichlorhydrate de 6-(benzylamino)-6,7-cyclohexéno-3-amino-2-méthyl-1,4-benzoxazine
dichlorhydrate de 6-((thién-2-yl)-méthylamino)-6,7-cyclohexéno-3-amino-2-méthyl-1,4-benzoxazine
dichlorhydrate de 6-((thién-2-yl)-méthylamino)-6,7-cyclopenténo-3-amino-2-méthyl-1,4-benzoxazine
dichlorhydrate de 6-(3-chlorobenzylamino)-6,7-cyclopenténo-3-amino-2-méthyl-1,4-benzoxazine
dichlorhydrate de 6-(3-chlorobenzylamino)-6,7-cyclohexéno-3-amino-2-méthyl-1,4-benzoxazine
dichlorhydrate de 6-(2-(benzylamino)-prop-1-yl)-3-amino-2-méthyl-1,4-benzoxazine
dichlorhydrate de 7-(3-chlorobenzylamino)-6,7-cyclohexéno-3-amino-2-méthyl-1,4-benzoxazine
dichlorhydrate de 6-((3,4-dichlorobenzyl)aminométhyl)-3-amino-2-méthyl-1,4-benzoxazine
dichlorhydrate 6-((2-chlorobenzyl)aminométhyl)-3-amino-2-méthyl-1,4-benzoxazine
dichlorhydrate de 6-((2,4-dichlorobenzyl)aminométhyl)-3-amino-2-méthyl-1,4-benzoxazine
dichlorhydrate de 6-((2,3-diméthylbenzyl)aminométhyl)-3-amino-2-méthyl-1,4-benzoxazine
dichlorhydrate de 6-((3-fluorobenzyl)aminométhyl)-3-amino-2-méthyl-1,4-benzoxazine
dichlorhydrate de 6-((indan-1-yl)aminométhyl)-3-amino-2-méthyl-1,4-benzoxazine
dichlorhydrate de 6-((indan-2-yl)aminométhyl)-3-amino-2-méthyl-1,4-benzoxazine
dichlorhydrate de 6-((cyclohexylméthyl)aminométhyl)-3-amino-2-méthyl-1,4-benzoxazine
dichlorhydrate de 6-((1,2,3,4-tétrahydronapht-1-yl)-aminométhyl)-3-amino-2-méthyl-1,4-benzoxazine
dichlorhydrate de 6-((3-méthoxybenzyl)aminométhyl)-3-amino-2-méthyl-1,4-benzoxazine
dichlorhydrate de 6-((3-nitrobenzyl)aminométhyl)-3-amino-2-méthyl-1,4-benzoxazine
dichlorhydrate de 6-((4-nitrobenzyl)aminométhyl)-3-amino-2-méthyl-1,4-benzoxazine
dichlorhydrate de 6-((4-sulfamoylbenzyl)aminométhyl)-3-amino-2-méthyl-1,4-benzoxazine
dichlorhydrate de 6-((4-méthylsulfonylbenzyl)-aminométhyl)-3-amino-2-méthyl-1,4-benzoxazine
dichlorhydrate de 6-((4-fluorobenzyl)aminométhyl)-3-amino-2-méthyl-1,4-benzoxazine
trichlorhydrate de 6-((4-diméthylaminobenzyl)-aminométhyl)-3-amino-2-méthyl-1,4-benzoxazine
dichlorhydrate de 6-((2-fluorobenzyl)aminométhyl)-3-amino-2-méthyl-1,4-benzoxazine
selon la revendication 1.

9. Médicament contenant un composé selon l'une quelconque des revendications 1 à 8 et un ou plusieurs véhicules et adjuvants usuels.

10. Utilisation des composés selon l'une quelconque des revendications 1 à 8, pour la fabrication d'un médicament.

11. Procédé pour la préparation des composés de formule I selon l'une quelconque des revendications 1-8 ou 13-15, **caractérisé en ce qu'**on fait réagir un composé de formule II ou un sel d'un tel composé 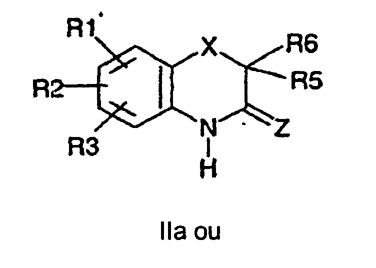 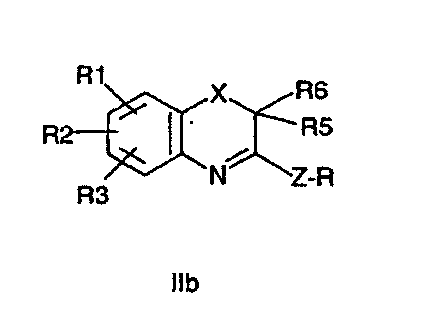 dans laquelle
R¹, R², R³, R⁵, R⁶ et X ont les significations données plus haut, Z est un atome d'oxygène ou de soufre et R représente un groupe alkyle en C₁₋₆, avec de l'ammoniac ou des amines primaires ou secondaires, des groupes amino primaires et secondaires présents étant éventuellement protégés de façon intermédiaire et éventuellement on effectue ensuite une acylation, on sépare les isomères ou on forme les sels.

12. Composés de formule IIa 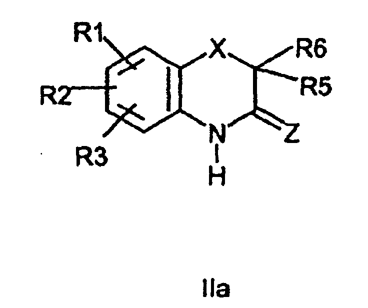 dans laquelle R³, R⁵, R⁶, X et Z ont les significations données plus haut et R¹ et R² forment ensemble, avec deux atomes de carbone voisins, un cycle à 5, 6, 7 ou 8 chaînons, qui peut être monocyclique ou bicyclique, saturé ou insaturé et soudé à un noyau benzénique, et dans lequel 1 ou 2 groupes CH₂ peuvent être remplacés par un atome d'oxygène ou par un groupe carbonyle ou un dérivé de celui-ci, choisi dans l'ensemble comprenant les groupes =N-OH, =N-O-alkyle(C₁₋₆), =N-NH₂ et =N-NH-phényle et qui peut être substitué par un groupe -NR⁷R⁸, -NR^{7'}R^{8'} ou alkyle en C₁₋₄,

13. Composés selon la revendication 1 ou 4, dans lesquels R¹ représente un groupe alkyle en C₁₋₆ qui est substitué par NR^{7'}R^{8'}.

14. Composés selon l'une quelconque des revendications 1-6 ou 13, dans lesquels R⁶ est un groupe alkyle en C₁₋₆ et R⁴ est un atome d'hydrogène.

15. Composés selon l'une quelconque des revendications 1-5, dans lesquels R¹ et R² forment ensemble, avec deux atomes de carbone voisins, un cycle à 5, 6, 7 ou 8 chaînons, qui peut être substitué par un groupe -NR⁷R⁸, -NR^{7'}R^{8'} ou alkyle en C₁₋₄,

16. Utilisation des composés selon l'une quelconque des revendications 1-6 ou 13-15, pour la fabrication d'un médicament destiné au traitement d'une maladie neurodégénérative, auto-immune, inflammatoire ou cardiovasculaire.

17. Composés selon la revendication 12, dans lesquels R⁶ est un groupe alkyle en C₁₋₆.
